# EUROPEAN PATENT SPECIFICATION

(11) **EP 1 986 665 B1**
(45) Date of publication and mention of the grant of the patent: **02.05.2018**
(21) Application number: 07718342.4
(22) Date of filing: 19.01.2007
(51) Int. Cl.: A61K 8/73, C08L 5/00, C12P 19/04, A61K 36/04, A61Q 19/08, A61P 39/06, C08B 37/00, A61K 31/715

(54) **MICROALGAE-DERIVED COMPOSITIONS FOR IMPROVING THE HEALTH AND APPEARANCE OF SKIN**
AUS MIKROALGEN STAMMENDE ZUSAMMENSETZUNGEN ZUR VERBESSERUNG DER GESUNDHEIT UND DES ERSCHEINUNGSBILDS DER HAUT
COMPOSITIONS DÉRIVÉES DE MICROALGUES DESTINÉES À AMÉLIORER LA SANTÉ ET L'ASPECT DE LA PEAU

(30) Priority: 19.01.2006 US 337171; 19.01.2006 US 337103; 19.01.2006 US 336656; 19.01.2006 US 336428; 19.01.2006 US 336426; 19.01.2006 US 336431; 19.01.2006 US 336430; 28.06.2006 US 816967 P; 20.07.2006 US 832091 P; 17.08.2006 US 838452 P; 30.11.2006 US 872072 P
(43) Date of publication of application: 05.11.2008
(73) Proprietor: TerraVia Holdings, Inc., South San Francisco, CA 94080 (US)
(72) Inventor: DILLON, Harrison, F., South San Francisco, CA 94080 (US); SOMANCHI, Aravind, South San Francisco, CA 94080 (US); ZAMAN, Anwar, South San Francisco, CA 94080 (US); RAO, Kamalesh, South San Francisco, CA 94080 (US); WOLFSON, Jonathan, South San Francisco, CA 94080 (US); DAY, Anthony, G., South San Francisco, CA 94080 (US); CORAGLIOTTI, Anna, South San Francisco, California 94080 (US)
(74) Representative: Marshall, Cameron John
(86) International application number: PCT/US2007/001653
(87) International publication number: WO 2007/084769

(56) References cited:
- WO-A1-00/75282
- WO-A1-97/00689
- WO-A1-2007/066340
- WO-A2-01/81603
- WO-A2-03/041679
- US-A- 4 417 415
- US-A- 4 906 746
- US-A- 5 089 481
- US-A- 5 916 577
- US-B2- 6 680 062
- MATSUI MARY S ET AL: "SULFATED POLYSACCHARIDES FROM RED MICROALGAE HAVE ANTIINFLAMMATORY PROPERTIES IN VITRO AND IN VIVO", APPLIED BIOCHEMISTRY AND BIOTECHNOLOGY, HUMANA PRESS, INC, UNITED STATES, vol. 104, no. 1, 1 January 2003 (2003-01-01), pages 13-22, XP008075424, ISSN: 0273-2289, DOI: 10.1385/ABAB:104:1:13
- TANNIN-SPITZ TEHILA ET AL: "Antioxidant activity of the polysaccharide of the red microalga Porphyridium sp.", JOURNAL OF APPLIED PHYCOLOGY, vol. 17, no. 3, May 2005 (2005-05), pages 215-222, XP002685902, ISSN: 0921-8971
- DATABASE WPI Week 200256 Thomson Scientific, London, GB; AN 2002-522564 XP002685903, & JP 2002 069443 A (MICRO ALGE CORP KK) 8 March 2002 (2002-03-08)
- GERESH S ET AL: "Characterization of the extracellular polysaccharide of Porphyridium sp.: molecular weight determination and rheological properties", CARBOHYDRATE POLYMERS, APPLIED SCIENCE PUBLISHERS, LTD. BARKING, GB, vol. 50, no. 2, 1 November 2002 (2002-11-01), pages 183-189, XP004367043, ISSN: 0144-8617, DOI: 10.1016/S0144-8617(02)00019-X

## Description

### BACKGROUND OF THE INVENTION

Carbohydrates have the general molecular formula CH₂O, and thus were once thought to represent "hydrated carbon". However, the arrangement of atoms in carbohydrates has little to do with water molecules. Starch and cellulose are two common carbohydrates. Both are macromolecules with molecular weights in the hundreds of thousands. Both are polymers; that is, each is built from repeating units, monomers, much as a chain is built from its links.

Three common sugars share the same molecular formula: C₆H₁₂O₆. Because of their six carbon atoms, each is a hexose. Glucose is the immediate source of energy for cellular respiration. Galactose is a sugar in milk. Fructose is a sugar found in honey. Although all three share the same molecular formula (C₆H₁₂O₆), the arrangement of atoms differs in each case. Substances such as these three, which have identical molecular formulas but different structural formulas, are known as structural isomers. Glucose, galactose, and fructose are "single" sugars or monosaccharides.

Two monosaccharides can be linked together to form a "double" sugar or disaccharide. Three common disaccharides are sucrose, common table sugar (glucose + fructose); lactose, the major sugar in milk (glucose + galactose); and maltose, the product of starch digestion (glucose + glucose). Although the process of linking the two monomers is complex, the end result in each case is the loss of a hydrogen atom (H) from one of the monosaccharides and a hydroxyl group (OH) from the other. The resulting linkage between the sugars is called a glycosidic bond. The molecular formula of each of these disaccharides is C₁₂H₂₂O₁₁ = 2 C₆H₁₂O₆ - H2O. All sugars are very soluble in water because of their many hydroxyl groups. Although not as concentrated a fuel as fats, sugars are the most important source of energy for many cells.

### BRIEF SUMMARY OF THE INVENTION

The present invention relates to polysaccharides and biomass produced from microalgae. Representative polysaccharides include those present in the cell wall of microalgae as well as secreted polysaccharides, or exopolysaccharides. In addition to the polysaccharides themselves, such as in an isolated, purified, or semi-purified form, the invention includes a variety of compositions containing one or more microalgal polysaccharides as disclosed herein. The compositions include nutraceutical, cosmeceutical, industrial and pharmaceutical compositions which may be used for a variety of indications and uses as described herein. Other compositions include those containing one or more microalgal polysaccharides and a suitable carrier or excipient for topical or oral administration.

The present invention relates to a composition comprising a microalgal polysaccharide, the composition produced by:
a) isolating a polysaccharide from microalgae;
b) drying the isolated polysaccharide to form a film that has been made completely or partially insoluble in water by heating; and
c) homogenizing the film to generate particles, wherein the particles increase in volume on contact with water compared to their anhydrous or partially hydrated volume,
wherein step b) is carried out by (i) heating in two parts wherein the second heating is at a temperature of 148°C to 160°C, or (ii) heating at a temperature of 135°C to 152°C.

The present invention also relates to a method of manufacturing a composition, comprising:
a) isolating a polysaccharide from microalgae;
b) drying the isolated polysaccharide to a solid film that has been made completely or partially insoluble in water by heating;
c) homogenizing the film into particles, wherein the particles increase in volume on contact with water compared to their anhydrous or partially hydrated volume; and
d) formulating the particles into a non-aqueous material,
wherein step b) is carried out by (i) heating in two parts wherein the second heating is at a temperature of 148°C to 160°C, or (ii) heating at a temperature of 135°C to 152°C.Decolorized microalgae for formulation in skin care products as a composition are disclosed. Highly desirable compositions of microalgal cells that do not stain human skin with red or green pigments but still provide delivery or high value cosmeceutical ingredients such as carotenoids, polyunsaturated fatty acids, moisturizing polysaccharides, superoxide dismutase, and other components.

The insight that combinations of high light irradiance and
limiting levels of nitrogen-containing compounds in the culture media allow production of
biomass high in cosmeceutical/nutraceutical value but do not contain substantial amounts of pigments that stain human skin when applied as part of a skin care formulation is disclosed. In addition, antioxidant, moisturizing polysaccharides are produced at higher levels in microalgae cells such as those of the genus Porphyridium under high light/low nitrogen conditions. Compositions of Porphyridium biomass that are substantially free of red coloration and contain higher amounts of exopolysaccharide than cells containing significant amounts of red coloration that are grown under nitrogen-replete conditions are disclosed.

So in one aspect, the disclosed includes a composition comprising cells of the genus *Porphyridium,* wherein an aqueous extract of the composition contains a reduced
level of red pigmentation, or a reduced absorbance at 545 nm, relative to the same cells

grown under different conditions. In some embodiments, the extract contains no more than about 75% to no more than about 5% of the absorbance per gram at 545nm compared to an extract of cells of the same species grown in a photobioreactor in ATCC 1495 artificial seawater (ASW) media in the presence of 50 microeinsteins of light per second per square meter. In other embodiments, the composition comprises a carrier and/or a preservative suitable for topical administration. In additional embodiments, the carrier is suitable for human topical administration.

Methods of producing or preparing microalgal polysaccharides are disclosed. In some disclosed methods, exogenous sugars are incorporated into the
polysaccharides to produce polysaccharides distinct from those present in microalgae that do not incorporate exogenous sugars. Methods of trophic conversion and recombinant gene expression in microalgae are also disclosed. In some methods, recombinant microalgae are prepared to express heterologous gene products, such as mammalian proteins
as a non-limiting example, while in other embodiments, the microalgae are modified to produce more of a small molecule already made by microalgae in the absence of genetic modification.

Methods of growing, producing and preparing microalgal biomass are also disclosed. In some disclosed methods, excess light is provided as one method of
removing pigmentation. In other methods, reducing the amount of nitrogen provided to microalgae cells in culture is provided as one method of removing pigmentation. In other methods increased light irradiance combined with culture media containing limiting amounts of nitrogen are used to reduce and/or eliminate red or green pigmentation. Additionally, the disclosure provides decolorized strains produced through chemical mutagenesis or gene
insertion/deletion methods that are used to generate biomass for skin care products.

In another aspect, the disclosure relates to compositions for topical application, such as a composition for application to human skin comprising a polysaccharide isolated from cells of the genus *Porphyridium.* In some embodiments, the composition comprises a polysaccharide that is part of a microalgal cell, or a homogenate thereof. In other embodiments, the polysaccharide is contained within microalgal cells, or a homogenate thereof, which is essentially free, or completely free, of red coloration. Thus a composition disclosed herein may also be essentially free, or completely free, of red coloration. Non-limiting examples include compositions comprising less than about 15 milligrams, less than about 1 milligram, or less than about 0.1 milligrams of phycoerythrin per dry gram of cells in the composition.

In additional embodiments, the composition is that of a cosmetic or other skin care product. Such products may contain one or more microalgal polysaccharides, or a microalgal cell homogenate, a topical carrier, and/or a preservative. In some embodiments, the carrier may be any carrier suitable for topical application, such as, but not limited to, use on human
skin or human mucosal tissue. In other embodiments, the composition may contain a purified microalgal polysaccharide, such as an exopolysaccharide, and a topical carrier. Exemplary carriers include liposome formulation, biodegradable microcapsule, lotion, spray, aerosol, dusting powder, biodegradable polymer, mineral oil, liquid petroleum, white petroleum, propylene glycol, polyoxyethylene polyoxypropylene compound, emulsifying wax, sorbitan
monostearate, polysorbate 60, cetyl ester wax, cetearyl alcohol, 2-octyldodecanol, benzyl alcohol and water. Exemplary preservatives include diiodomethyl-p-tolylsulfone, 2-Bromo-2-nitropropane-1,3-diol, cis isomer 1-(3-chloroallyl)-3,5,7-triaza-1-azoniaadamantane chloride, glutaraldehyde, 4,4-dimethyl oxazolidine, 7-Ethylbicyclooxazolidine, methyl paraben, sorbic acid, Germaben 11, and disodium EDTA.

As a cosmeceutical, the composition may contain a microalgal polysaccharide or homogenate and other component material found in cosmetics. In some embodiments, the component material may be that of a fragrance, a colorant (e.g. black or red iron oxide, titanium dioxide and/or zinc oxide, etc.), a sunblock (e.g. titanium, zinc, etc.), and a mineral or metallic additive.

In other aspects, the disclosure includes methods of preparing or producing a microalgal polysaccharide. In some aspects relating to an exopolysaccharide, the disclosure includes methods that separate the exopolysaccharide from other molecules present in the medium used to culture exopolysaccharide producing microalgae. In some embodiments, separation includes removal of the microalgae from the culture medium containing the exopolysaccharide, after the microalgae has been cultured for a period of time. Of course the methods may be practiced with microalgal polysaccharides other than exopolysaccharides. In other embodiments, the methods include those where the microalgae was cultured in a bioreactor, optionally where a gas is infused into the bioreactor.

In one embodiment, the disclosure includes a method of producing an
exopolysaccharide, wherein the method comprises culturing microalgae in a bioreactor, wherein gas is infused into the bioreactor; separating the microalgae from culture media, wherein the culture media contains the exopolysaccharide; and separating the exopolysaccharide from other molecules present in the culture media.

The microalgae of the invention or disclosure may be that of any species, including those listed in Table 1 herein. In some embodiments, the microalgae is a red algae, such as the red algae *Porphyridium,* which has two known species *(Porphyridium sp.* and *Porphyridium cruentum)* that have been observed to secrete large amounts of polysaccharide into their surrounding growth media. In other embodiments, the microalgae is of a genus selected from *Rhodella,*

*Chlorella,* and *Achnanthes.* Non-limiting examples of species within a microalgal genus of the invention include *Porphyridium sp., Porphyridium cruentum, Porphyridium purpuretun, Porphyridium aerugineunz, Rhodella nzaculata, Rhodella reticulata, Chlorella autotrophica, Chlorella stigmatophora, Chlorella capsulata, Achnanthes brevipes* and *Achnanthes longipes.*

In some embodiments, a polysaccharide preparation method is practiced with culture media containing over 26.7, or over 27, mM sulfate (or total S0₄²"). Non-limiting examples include media with more than about 28, more than about 30, more than about 35, more than about 40, more than about 45, more than about 50, more than about 55, more than about 60, more than about 65, more than about 70, more than about 75, more than about 80, more than about 85, more than about 90, more than about 95, more than about 100 mM sulfate, and in some instances more than 250mM, more than 400mM, more than 550mM, and more than 750mM, more than 900mM, more than 1M, and more than 2mM sulfate. Sulfate in the media may be provided in one or more of the following forms: Na₂SO₄ 10 H₂O, MgSO₄.7H₂O,
MnSO₄, and CuSO₄.

Other embodiments of the method include the separation of an exopolysaccharide from other molecules present in the culture media by tangential flow filtration. Alternatively, the methods may be practiced by separating an exopolysaccharide from other molecules present in the culture media by alcohol precipitation. Non-limiting examples of alcohols to use include ethanol, isopropanol, and methanol.

In other embodiments, a method may further comprise treating a polysaccharide or exopolysaccharide with a protease to degrade polypeptide (or proteinaceous) material attached to, or found with, the polysaccharide or exopolysaccharide. The methods may optionally comprise separating the polysaccharide or exopolysaccharide from proteins, peptides, and amino acids after protease treatment.

In other embodiments, a method of formulating a cosmeceutical composition is disclosed. As one non-limiting example, the composition may be prepared by adding separated polysaccharides, or exopolysaccharides, to homogenized microalgal cells before, during, or after homogenization. Both the polysaccharides and the microalgal cells may be from a culture of microalgae cells in suspension and under conditions allowing or permitting cell division. The culture medium containing the polysaccharides is then separated from the microalgal cells followed by 1) separation of the polysaccharides from other molecules in the medium and 2) homogenization of the cells.

Other compositions of the disclosure may be formulated by subjecting a culture of microalgal cells and soluble exopolysaccharide to tangential flow filtration until the composition is substantially free of salts. Alternatively, a polysaccharide is prepared after proteolysis of polypeptides present with the polysaccharide. The polysaccharide and any contaminating polypeptides may be that of a culture medium separated from microalgal cells in a culture thereof. In some embodiments, the cells are of the genus *Porphyriditun.*

In a further aspect, the disclosure includes a composition comprising particulate polysaccharides. The polysaccharides may be from any microalgal source, and with any level of sulfation, as described herein. The composition may be sterile or substantially free of endotoxins and/or proteins in some embodiments. In other
embodiments, the composition further comprises hyaluronic acid or another agent suitable or desirable for treatment of skin. The particles in some embodiments are generated by first purifying the polysaccharide away from biomass, then drying the purified polysaccharide into a film, and then homogenizing and/or grinding the film into smaller particles. Compositions as defined in claim 1 form part of the invention.

In some embodiments, the polysaccharides are in the form of a purified material that was dried to be completely or partially insoluble in water. Preferably the purified material
has been separated from cell biomass, for example as described in Example 2. In such purified form the polysaccharide is at least 50% polysaccharide by weight, and more preferably above 75% polysaccharide by weight. In some embodiments the polysaccharide is associated with one or more species of protein that can be endogenous to the microalgae
source, and alternatively can be a fusion protein that is partially endogenous to the
microalgae source as described herein. In some embodiments, the dried polysaccharide particles are in mixture with a non-aqueous solvent or material. In other embodiments, the dried polysaccharide particles are partially soluble such that they are from less than about 70% to less than about 1% soluble in water.

In additional embodiments, the polysaccharide particles increase in volume, or swell, on contact with water or water vapor. Thus the volume of the polysaccharide particles increases compared to its anhydrous or partially hydrated volume before exposure to the water or water vapor. In some embodiments, the particles increase in volume by an amount selected from at least about 5% to at least about 5000%.

The disclosed invention further includes methods for the preparation or manufacture of the dried polysaccharide particles. In some embodiments, the method comprises formulating particles of polysaccharide material into a non-aqueous material. The particles may be formed from a film of dried polysaccharide material, wherein at least a portion (or some proportion) of the film has been made completely or partially insoluble in water.
Optionally, the particles are formed by homogenization of the film into particulate form. Methods as defined in claim 12 form part of the invention.

In some cases, the film is formed by heating a suspension of polysaccharide material until all or part of the film is insoluble. The heating may be of an aqueous suspension of the material to remove water from the suspension. Of course the polysaccharide in the suspension may be from any microalgal source as described herein. Optionally, the
polysaccharide in the suspension has been isolated from microalgal biomass. Optionally, the polysaccharide in the suspension has been isolated from supernatant of a culture of microalgae.

The disclosed invention thus includes a method of preparing or manufacturing a composition for topical application, such as for improving the appearance of skin. The method may comprise 1) drying an aqueous suspension of a polysaccharide isolated from microalgae to a solid film, wherein at least some proportion of the film has been made completely or partially insoluble in water; 2) homogenizing the film into particles; and optionally 3) formulating the particles into a non-aqueous material. In some embodiments, the homogenizing is via a method selected from jet milling, ball milling, Retsch® milling, pin milling and milling in a Quadro® device. Optionally, the formulating of the particles is into the non-aqueous phase of an oil-in-water emulsion, such as an emulsion suitable for topical application. The non-aqueous phase may comprise an oil suitable for topical application, such as hexadecanoic acid as a non-limiting example. In other cases, the formulating of the particles is into a carrier suitable for topical administration as described herein. In some embodiments, the particles may be relatively uniform in size or may range in size, but in many embodiments, the particles have an average size between about 400 and 0.1 microns.

The formation of a solid film may be by heating performed between about 40 and about 180 degrees Celsius. In other embodiments, the heating is performed in two parts. The first part may comprise heating a suspension, optionally aqueous, of polysaccharide material to no more than about 60 to about 100°C for a time period sufficient to form or produce a solid film. This may be followed by a second heating of the solid film for a (second) time period sufficient to reach no more than about 148 to about 160°C. In one embodiment the first heating is in the presence of air, which may be optionally combined with the second heating (of the solid film) being in at least a partial vacuum or in a high vacuum. Of course the second heating under reduced pressure may be used independent of the first heating in the presence of air. In other embodiments the heating is done in a single step, either in the presence of air or in the presence of a partial or full vacuum.

In some alternative embodiments, a method to render the polysaccharide material insoluble is selected from chemical cross-linking, chemical dehydration through displacement of bound water by an alcohol, precipitation from solution using an alcohol or a ketone or pH, and coating of particles by microencapsulation.

In an additional aspect, the disclosed invention includes a method of topically applying a composition comprising polysaccharides in particulate form. In some embodiments, the application is to skin, such as to mammalian or human skin. Alternatively, the application is to lips or wrinkles on human skin, or by injection into skin or a skin tissue. In many embodiments, the application is to improve the appearance of skin.

In additional embodiments of the disclosure, a polysaccharide containing composition,(optionally with polysaccharides in particulate form) may be used in a method of cosmetic enhancement. In one embodiment, a method may include injecting a polysaccharide produced by microalgae into mammalian skin. Preferably the polysaccharide is sterile and free of protein.

In further embodiments, a method to treat skin, such as mammalian or human skin, is disclosed. In some embodiments, the method is for the treatment of human facial skin or a
tissue thereof. Such methods include a method to stimulating collagen synthesis, stimulating elastin synthesis, or inhibiting collagenase activity in such skin by applying a disclosed composition of the invention to the skin. Additional methods include a method to reduce the signs of aging or reduce the appearance of aging in human skin by applying a composition of the disclosed invention to the skin. Non-limiting examples of a sign of aging or an
appearance of aging include wrinkles, such as those on the forehead or around the eyes and/or lips, and liver spots (yellowish-brown flat spots that appear as large freckles). In some embodiments, a sign or appearance of aging is associated with reactive oxygen species (ROS) formation and/or activity in the skin. The use of a composition may thus be based in part on the insight that the disclosed polysaccharides possess anti-oxidant activity, and that further the high sulfated polysaccharides wherein the percent of sulfur by weight is above 4.75%.

Additional embodiments include the use of a polysaccharide containing composition in a method of reducing the effects of ultraviolet (UV) light or radiation, such as that present
in sunlight, on skin or a skin tissue. One non-limiting example is a method of shielding mammalian skin from UV light. The method may comprise applying a composition of the disclosed invention to skin or a skin tissue in an effective or sufficient amount to shield, at
least in part, the skin from UV radiation. In an alternative embodiment, a composition of the invention may be applied in an effective or sufficient amount to treat skin that has been damaged by UV radiation. An additional non-limiting example is a method of for treating skin to reduce the risk of skin cancer induced by sunlight or UV radiation. The method may
comprise applying a composition of the invention in an effective or sufficient amount to reduce the risk of UV or sunlight induced skin cancer.

An additional non-limiting example is a method of for treating skin to reduce the risk of skin cancer induced by sunlight or UV radiation that causes erythema. Erythema is redness of the skin caused by increased blood flow to the capillaries. A subject can assess the effective amount of microalgal materials sufficient to treat erythema using methods known in the art. See for example J. Invest. Dermatol., 117 (5); 1318-1321 (2001).

In addition to the above, application of a composition of the disclosure to human skin may be used in a method of reducing reactive oxygen species (ROS) in the skin or a skin
tissue. This is based in part on the insight that the disclosed polysaccharides possess anti-oxidant activity. In some embodiments, the method is used to prevent or treat a disease or unwanted condition associated with ROS or oxidative stress. Non-limiting examples of such a disease or unwanted condition include reducing inflammation or irritation of the skin. In some embodiments, the polysaccharide composition comprises one or more other agents or compounds with anti-oxidant activity. In further embodiments, the method may be used to
lower the level of ROS, or reduce or decrease the amount of damage caused by ROS in skin or a skin tissue. The amount of the composition may be any that is effective or sufficient to produce a desired improvement or therapeutic benefit.

The details of additional embodiments of the invention are set forth in the accompanying drawings and the description below. Other features and advantages of the invention will be apparent from the drawings and detailed description, and from the claims.

### BRIEF DESCRIPTION OF THE DRAWINGS

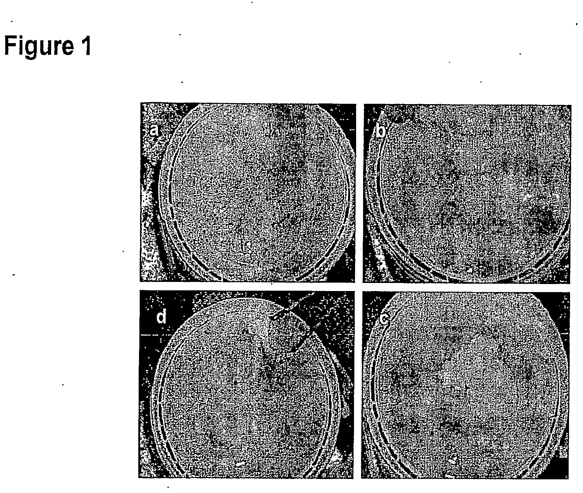

Figure 1 shows precipitation of 4 liters of *Porphyridium cruentum* exopolysaccharide using 38.5% isopropanol. (a) supernatant; (b) addition of 38.5% isopropanol; (c) precipitated polysaccharide; (d) separating step.
Figure 2 shows growth of Porphyridium sp. and Porphyridium cruentum cells grown in light in the presence of various concentrations of glycerol.
Figure 3 shows *Porphyridium sp.* cells grown in the dark in the presence of various concentrations of glycerol.
Figure 4 shows levels of solvent-accessible polysaccharide in *Porphyridium sp.* homogenates subjected to various amounts of physical disruption from Sonication Experiment 1.
Figure 5 shows levels of solvent-accessible polysaccharide in *Porphyridium sp.* homogenates subjected to various amounts of physical disruption from Sonication Experiment 2.
Figure 6 shows protein concentration measurements of autoclaved, protease-treated, and diafiltered exopolysaccharide.
Figure 7 shows various amounts and ranges of amounts of compounds found per gram of cells in cells of the genus Porphyridium.
Figure 8 shows *Porphyridium sp.* cultured on agar plates containing various concentrations of zeocin.

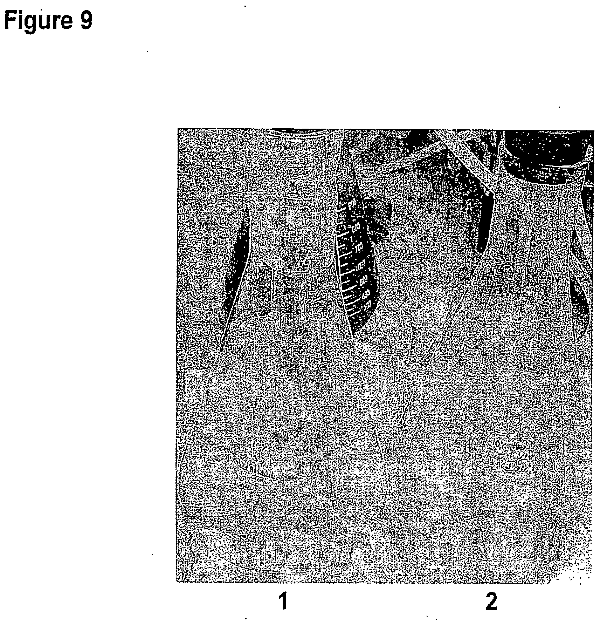

Figure 9 shows cultures of nitrogen-replete (flask 1) and nitrogen-starved (flask 2) *Porphyridium cruentum.* The culture media in flask 1 is as described in Example 1. The culture media in flask 2 is as described in Example 1 except that the culture media contained no Tris and 0.125g/L potassium nitrate, pH 7.6. Flasks 1 and 2 were inoculated with identical amounts of deep red colored cells taken from a culture grown in ATCC1495 ASW media. The cells in flask 2 are substantially free of red coloration.

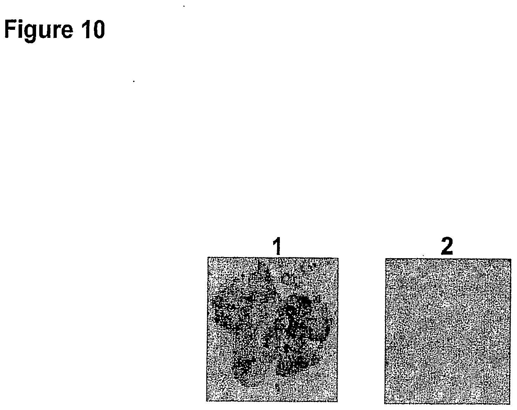

Figure 10 shows lyophilized biomass from nitrogen-replete (panel 1) and nitrogen-starved (panel 2) *Porphyridium cruentum* cells. The culture media used to grow the cells shown in panel 1 was as described in Example 1. The culture media used to grow the cells shown in panel 2 was as described in Example 1 except that the culture media contained no Tris and 0.125g/L potassium nitrate, pH 7.6. The lyophilized biomass in panel 2 is substantially free of red coloration.
Figure 11(a) shows UVB-induced TT dimer formation in the presence and absence of microalgae-derived materials. Figure 11(b) shows secretion of procollagen by human fibroblasts in the presence and absence of microalgae-derived materials.
Figure 12(a) shows secretion of elastin by human fibroblasts in the presence and absence of microalgae-derived materials. Figure 12(b) shows inhibition of PMN migration in the presence and absence of microalgae-derived materials.
Figure 13(a) shows secretion of IL1-α in the presence and absence of microalgae-derived materials. Figure 13(b) shows secretion of gamma interferon in the presence and absence of microalgae-derived materials.
Figure 14(a) shows PCR genotyping of two Porphyridium transformants for the ble antibiotic resistance transgene. Figure 14(b) shows PCR genotyping of two Porphyridium transformants for the endogenous glycoprotein gene promoter. Figure 14(c) shows PCR genotyping of one Porphyridium transformants for an exogenous gene encoding a recoded human GLUT1 transporter.
Figure 15 shows a Southern blot indicating chromosomal integration of an exogenous gene encoding a recoded human GLUT1 transporter.
Figure 16 shows insoluble and soluble polysaccharide bead preparations.
Figure 17(a) shows swelling of polysaccharide beads over time. Figure 17(b) shows percentages of polysaccharide in the insoluble gel phase over time.
Figure 18 shows PBMC proliferation in the presence and absence of microalgae-derived materials.

### DETAILED DESCRIPTION OF THE INVENTION

Definitions: The following definitions are intended to convey the intended meaning of terms used throughout the specification and claims, however they are not limiting in the
sense that minor or trivial differences fall within their scope.

"Active in microalgae" means a nucleic acid that is functional in microalgae. For example, a promoter that has been used to drive an antibiotic resistance gene to impart antibiotic resistance to a transgenic microalgae is active in microalgae. Non-limiting
examples of promoters active in microalgae are promoters endogenous to certain algae
species and promoters found in plant viruses.

"ARA" means Arachidonic acid.

"Associates with" means, within the context of a polysaccharide binding fusion protein, one molecule binding to another molecule. Affinity and selectivity of binding can vary when a polysaccharide and a polysaccharide binding protein are in association with each other.

"Axenic" means a culture of an organism that is free from contamination by other living organisms.

"Bioreactor" means an enclosure or partial enclosure in which cells are cultured in suspension.

"Carrier suitable for topical administration" means a compound that may be administered, together with one or more compounds of the present disclosure, and which does not destroy the activity thereof and is nontoxic when administered in concentrations and amounts sufficient to deliver the compound to the skin or a mucosal tissue.
"Combination Product" means a product that comprises at least two distinct
compositions intended for human administration through distinct routes, such as a topical route and an oral route. In some embodiments the same active agent is contained in both the topical and oral components of the combination product.

"Conditions favorable to cell division" means conditions in which cells divide at least once every 72 hours.

"DHA" means Docosahexaenoic acid.

"Endopolysaccharide" means a polysaccharide that is retained intracellularly.

"EPA" means eicosapentaenoic acid.

Cells or biomass that are "essentially free of red coloration" contain either no red color visible to the naked eye or a small amount of red color such that red is a minor color of the overall composition compared to at least one other color such as yellow.

Cells or biomass that are "completely free of red coloration" contain no red color visible to the naked eye.

"Exogenous gene" means a gene transformed into a wild-type organism. The gene can be heterologous from a different species, or homologous from the same species, in which case the gene occupies a different location in the genome of the organism than the endogenous gene.

"Exogenously provided" describes a molecule provided to the culture media of a cell culture.

"Exopolysaccharide" means a polysaccharide that is secreted from a cell into the extracellular environment.

"Filtrate" means the portion of a tangential flow filtration sample that has passed through the filter.

"Fixed carbon source" means molecule(s) containing carbon that are present at ambient temperature and pressure in solid or liquid form.

"Glycopolymer" means a biologically produced molecule comprising at least two monosaccharides. Examples of glycopolymers include glycosylated proteins, polysaccharides, oligosaccharides, and disaccharides.

"Homogenate" means cell biomass that has been disrupted. A homogenate is not necessarily homogeneous.

A compound that can be "metabolized by cells" means a compound whose elemental components are incorporated into products endogenously produced by the cells. For example, a compound containing nitrogen that can be metabolized by cells is a compound containing at least one nitrogen atom per molecule that can be incorporated into a nitrogen-containing, endogenously produced metabolite such as an amino acid.

"Microalgae" means a single-celled organism that is capable of performing photosynthesis. Microalgae include obligate photoautotrophs, which cannot metabolize a fixed carbon source as energy, as well as heterotrophs, which can live solely off of light, solely off of a fixed carbon source, or a combination of the two.

"Naturally produced" describes a compound that is produced by a wild-type organism.

"Photobioreactor" means a waterproof container, at least part of which is at least partially transparent, allowing light to pass through, in which one or more microalgae cells are cultured. Photobioreactors may be sealed, as in the instance of a polyethylene bag, or may be open to the environment, as in the instance of a pond.

"Polysaccharide material" is a composition that contains more than one species of polysaccharide, and optionally contaminants such as proteins, lipids, and nucleic acids, such as, for example, a microalgal cell homogenate.

"Polysaccharide" means a compound or preparation containing one or more molecules that contain at least two saccharide molecules covalently linked. A "polysaccharide", "endopolysaccharide" or "exopolysaccharide" can be a preparation of polymer molecules that have similar or identical repeating units but different molecular weights within the population.

"Port", in the context of a photobioreactor, means an opening in the photobioreactor that allows influx or efflux of materials such as gases, liquids, and cells. Ports are usually connected to tubing leading to and/or from the photobioreactor.

"Red microalgae" means unicellular algae that is of the list of classes comprising Bangiophyceae, Florideophyceae, Goniotrichales, or is otherwise a member of the Rhodophyta.

"Retentate" means the portion of a tangential flow filtration sample that has not passed through the filter.

"Small molecule" means a molecule having a molecular weight of less than 2000 daltons, in some instances less than 1000 daltons, and in still other instances less than 500 daltons or less. Such molecules include, for example, heterocyclic compounds, carbocyclic compounds, sterols, amino acids, lipids, carotenoids and polyunsaturated fatty acids.

A molecule is "solvent available" when the molecule is isolated to the point at which it can be dissolved in a solvent, or sufficiently dispersed in suspension in the solvent such that it can be detected in the solution or suspension. For example, a polysaccharide is "solvent available" when it is sufficiently isolated from other materials, such as those with which it is naturally associated, such that the polysaccharide can be dissolved or suspended in an aqueous buffer and detected in solution using a dimcthylmethylene blue (DMMB) or phenol:sulfuric acid assay. In the case of a high molecular weight polysaccharide containing hundreds or thousands of monosaccharides, part of the polysaccharide can be "solvent available" when it is on the outermost layer of a cell wall while other parts of the same polysaccharide molecule are not "solvent available" because they are buried within the cell wall. For example, in a culture of microalgae in which polysaccharide is present in the cell wall, there is little "solvent available" polysaccharide since most of the cell wall polysaccharide is sequestered within the cell wall and not available to solvent. However, when the cells are disrupted, e.g., by sonication, the amount of "solvent available" polysaccharide increases. The amount of "solvent accessible" polysaccharide before and after homogenization can be compared by taking two aliquots of equal volume of cells from the same culture, homogenizing one aliquot, and comparing the level of polysaccharide in solvent from the two aliquots using a DMMB assay. The amount of solvent accessible polysaccharide in a homogenate of cells can also be compared with that present in a quantity of cells of the same type in a different culture needed to generate the same amount of homogenate.

"Substantially free of protein" means compositions that are preferably of high purity and are substantially free of potentially harmful contaminants, including proteins (e.g., at least National Food (NF) grade, generally at least analytical grade, and more typically at least pharmaceutical grade). Compositions are at least 80, at least 90, at least 99 or at least 99.9% w/w pure of undesired contaminants such as proteins are substantially free of protein. To the extent that a given compound must be synthesized prior to use, the resulting product is typically substantially free of any potentially toxic agents, particularly any endotoxins, which may be present during the synthesis or purification process. Compositions are usually made under GMP conditions. Compositions for parenteral administration are usually sterile and substantially isotonic.

### I General

Polysaccharides form a heterogeneous group of polymers of different length and composition. They are constructed from monosaccharide residues that are linked by glycosidic bonds. Glycosidic linkages may be located between the C₁ (or C₂) of one sugar residue and the C₂, C₃, C₄, C₅ or C₆ of the second residue. A branched sugar results if more than two types of linkage are present in single monosaccharide molecule.

Monosaccharides are simple sugars with multiple hydroxyl groups. Based on the number of carbons (e.g., 3, 4, 5, or 6) a monosaccharide is a triose, tetrose, pentose, or hexose. Pentoses and hexoses can cyclize, as the aldehyde or keto group reacts with a hydroxyl on one of the distal carbons. Examples of monosaccharides are galactose, glucose, and rhamnose.

Polysaccharides are molecules comprising a plurality of monosaccharides covalently linked to each other through glycosidic bonds. Polysaccharides consisting of a relatively small number of monosaccharide units, such as 10 or less, are sometimes referred to as oligosaccharides. The end of the polysaccharide with an anomeric carbon (C₁) that is not involved in a glycosidic bond is called the reducing end. A polysaccharide may consist of one monosaccharide type, known as a homopolymer, or two or more types of monosaccharides, known as a heteropolymer. Examples of homopolysaccharides are cellulose, amylose, inulin, chitin, chitosan, amylopectin, glycogen, and pectin. Amylose is a glucose polymer with α(1→4) glycosidic linkages. Amylopectin is a glucose polymer with α(1→4) linkages and branches formed by α(1→6) linkages. Examples of heteropolysaccharides are glucomannan, galactoglucomannan, xyloglucan, 4-O-methylglucuronoxylan, arabinoxylan, and 4-O-Methylglucuronoarabinoxylan.

Polysaccharides can be structurally modified both enzymatically and chemically. Examples of modifications include sulfation, phosphorylation, methylation, O-acetylation, fatty acylation, amino N-acetylation, N-sulfation, branching, and carboxyl lactonization.

Glycosaminoglycans are polysaccharides of repeating disaccharides. Within the disaccharides, the sugars tend to be modified, with acidic groups, amino groups, sulfated hydroxyl and amino groups. Glycosaminoglycans tend to be negatively charged, because of the prevalence of acidic groups. Examples of glycosaminoglycans are heparin, chondroitin, and hyaluronic acid.

Polysaccharides are produced in eukaryotes mainly in the endoplasmic reticulum (ER) and Golgi apparatus. Polysaccharide biosynthesis enzymes are usually retained in the ER, and amino acid motifs imparting ER retention have been identified (Gene. 2000 Dec 31;261(2):321-7). Polysaccharides are also produced by some prokaryotes, such as lactic acid bacteria.

Polysaccharides that are secreted from cells are known as exopolysaccharides. Many types of cell walls, in plants, algae, and bacteria, are composed of polysaccharides. The cell walls are formed through secretion of polysaccharides. Some species, including algae and bacteria, secrete polysaccharides that are released from the cells. In other words, these molecules are not held in association with the cells as are cell wall polysaccharides. Instead, these molecules are released from the cells. For example, cultures of some species of microalgae secrete exopolysaccharides that are suspended in the culture media.

### II Methods of Producing Polysaccharides

### A. Cell Culture Methods: Microalgae

Polysaccharides can be produced by culturing microalgae. Examples of microalgae that can be cultured to produce polysaccharides are shown in Table 1. Also listed are references that enable the skilled artisan to culture the microalgae species under conditions sufficient for polysaccharide production. Also listed are strain numbers from various publicly available algae collections, as well as strains published in journals that require public dissemination of reagents as a prerequisite for publication.

**Table 1**

| **Species** | **Strain Number / Source** | **Culture and polysaccharide purification method reference** | **Reported Monosaccharide Composition** | **Culture conditions** |
|---|---|---|---|---|
| **Porphyridium cruentum** | UTEX¹ 161 | M.A. Guzman-Murillo and F. Ascencio., Letters in Applied Microbiology 2000, 30, 473-478 | Xylose, Glucose, Galactose, Glucoronic acid | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Porphyridium cruentum** | UTEX 161 | Fabregas et al., Antiviral Research 44(1999)-67-73 | Xylose, Glucose, Galactose and Glucoronic acid | Cultured in 80 ml glass tubes with aeration of 100ml/min and 10% CO₂, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984 modified in 4 mmol Nitrogen/L) |
| **Porphyridium sp.** | UTEX 637 | Dvir, Brit. J.of Nutrition (2000), 84 469-476. [Review: S. Geresh Biosource Technology 38 (1991) 195-201]-Huleihel, 2003, Applied Spectoscopy, v57, No. 4 2003 | Xylose, Glucose and Galactose, Methyl hexoses, Mannose, Rhamnose | Outdoor cultivation for 21 days in artficial sea water in polyethylene sleeves. See Jones (1963) and Cohen & Malis Arad, 1989) |
| **Porphyridium aerugineum** | SAG² 111.79 | Talyshinsky, Marina Cancer Cell Int'l 2002, 2; Review: S. Geresh Biosource Technology 38 (1991) 195-201]l See Ramus_1972 | xylose, glucose, galactose, methyl hexoses | see Dubinsky ct al. Plant Physio. And Biochem. (192) 30:409-414. Pursuant to Ramus_1972--> Axenic culutrcs are grown in MCYII liquid medium at 25°C and illuminated with Cool While fluorescent tubes on a 16:8 hr light dark cycle. Cells kept in suspension by agitation on a gyrorotary shaker or by a stream of filtered air. |
| **Porphyridium purpurpeum** | strain 1380-1a | Schmitt D., Water Research Volume 35, Issue 3 ,March 2001, Pages 779-785, Bioprocess Biosyst Eng. 2002 Apr;25(1):35-42. Epub 2002 Mar 6 | unknown | See cited reference |
| **Chaetoceros sp.** | USCE³ | M.A. Guzman-Murillo and F. Ascencio., Letters in Applied Microbiology 2000, 30, 473-478 | unknown | See cited reference |
| **Chlorella autotropica** | USCE | M.A. Guzman-Murillo and F. Ascencio., Letters in Applied Microbiology 2000, 30, 473-478 | unknown | See cited reference |
| **Chlorella autotropica** | UTEX 580 | Fabregas et al., Antiviral Research 44(1999)-67-73 | unknown | Cultured in 80 ml glass tubes with aeration of 100ml/min and 10% CO2, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984) |
| **Chlorella capsulata** | UTEX LB2074 | M.A. Guzman-Murillo and F. Ascencio., Letters in Applied Microbiology 2000, 30, 473-478 (species is a.k.a. Schizochlamydella capsulata) | Unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Chlorella stigmatophora** | GGMCC⁴ | S. Guzman, Phytotherapy Rscrh (2003) 17: 665-670 | glucose, glucuronic acid, xylose, ribose/fucose | Grown in 10 L of membrane filtered (0.24 um) seawater and sterilized at 120° for 30 min and enriched with Erd Schreiber medium. Cultures maintained at 18 +/- 1°C under constant 1% CO₂ bubbling. |
| **Dunalliela tertiolecta** | DCCBC⁵ | Fabregas et al., Antiviral Research 44(1999)-67-73 | unknown | Cultured in 80 ml glass tubes with aeration of 100ml/min and 10% CO2, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984) |
| **Dunalliela bardawil** | DCCBC | Fabregas et al., Antiviral Research 44(1999)-67-73 | unknown | Cultured in 80 ml glass tubes with aeration of 100ml/min and 10% CO2, for 10s every ten minutes to maintain pH > 7.6. Maintained at 22° in 12:12 Light/dark periodicity. Light at 152.3 umol/m²/s. Salinity 3.5% (nutrient enriched as Fabregas, 1984) |
| **Isochrysis galbana var. tahitiana** | HCTMS⁶ | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um millipore filler or distilled water depending on microalgae salt tolerance. Incubated at 25°C in flasks and illuminated with white fluorescent lamps. |
| **Isochrysis galbana var. Tiso** | UTEX LB 987 | Fabregas et al., Antiviral Research 44(1999)-67-73 | unknown | Cultured in 80 ml glass tubes with aeration of 100ml/min and 10% CO2, for 10s every ten 22° minutes to maintain pH > 7.6. Maintained at 22° in 12:12 Light/dark periodicity. Light at 152.3 umol/m²/s. Salinity 3.5% (nutrient enriched as Fabregas, 1984) |
| **Isochrysis sp.** | CCMP⁷ | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Phaeodactylum tricornutum** | UTEX 642, 646, 2089 | M.A M.A. Guzman-Murillo and F. Ascencio., Letters in Applied Microbiology 2000, 30. 473-478 | unknown | Cultures obtained from various sources and were cultured in F/2 broth prepared with seawater filtered through a 0.45um Millipore filter or distilled water depending on microalgae sail tolerance. Incubated at 25°C in flasks and illuminated with white fluorescent lamps. |
| **Phacodactylum tricornutum** | GGMCC | S. Guzman, Phytotherapy Rscrh (2003) 17: 665-670 | glucose, glucuronic acid, and mannose | Grown in 10 L of membrane filtered (0.24 um) seawater and sterilized at 120° for 30 min and enriched with Erd Schreiber medium. Cultures maintained at 18 +/- 1° C under constant 1% CO2 bubbling. |
| **Tetraselmis sp.** | CCMP 1634-1640; UTEX 2767 | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Botrycoccus braunii** | UTEX 572 and 2441 | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Cholorococcu m** | UTEX 105 | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Hormotilopsis gelatinosa** | UTEX 104 | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Neochloris oleoabundans** | UTEX 1185 | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Ochromonas Danica** | UTEX L1298 | M.A. Guzman-Murillo and F. Ascencio., Letters in Applied Microbiology 2000, 30, 473-478 | unknown | Cultures obtained from various sources and were cultured in F/2 broth prepared with seawater filtered through a 0.45um Millipore filter or distilled water depending on microalgae salt tolerance. Incubated at 25°C in flasks and illuminated with white fluorescent lamps. |
| **Gyrodinium impudicum** | KG03; KG09; KGJOI | Yim, Joung Han et. Al., J. of Microbiol 2004, 305-14; Yim, J.H. (2000) Ph.D. Dissertations, University of Kyung Hee, Seoul | Dec. Homopolysacchari de of galactose w/ 2.96% uronic acid | Isolated from seawater collected from red-tide bloom in Korean coastal water. Maintained in 172 medium at 22° under circadian light at 100uE/m2/sec: dark cycle of 14h: 10h for 19 days. Selected with neomycin and/or cephalosporin 20ug/ml |
| **Ellipsoidon sp.** | See cited references | Fabregas et al., Antiviral Research 44(1999)-67-73; Lewin, R.A. Cheng, L., 1989, Phycologya 28, 96-108 | unknown | Cultured in 80 ml glass tubes with aeration of 100ml/min and 10% CO2, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984) |
| **Rhodella reticulata** | UTEX 2320 | Talyshinsky, Marina Cancer Cell Int'l 2002, 2 | unknown | See Dubinsky O. et al. Composition of Cell wall polysaccharide produced by unicellular red algae Rhodella reticulata. 1992 Plant Physiology and biochemistry 30:409-414 |
| **Rhodella maculata** | UTEX LB 2506 | Evans, LV., et al. J. Cell Sci 16, 1-21(1974); EVANS, L. V. (1970). (1970). Br. phycol.J. 5, 1-13. | Galactose, xylose, glucuronic acid | Grown in either SWM3 medium or ASP12, MgCl2 supplement. 100 mls in 250 mls volumetric Erlenmeyer flask with gentle shaking and 4000lx Northern Light fluorescent light for 16 hours. |
| **Gymnodinium sp.** | Oku-1 | Sogawa, K., et al., Life Sciences, Vol. 66, No. 16, pp. PL 227-231 (2000) AND Umermura, Ken: Biochemical Pharmacology 66 (2003) 481-487 | unknown | See cited reference |
| **Spirilina platensis** | UTEX LB 1926 | Kaji, T et. Al., Life Sci 2002 Mar 8;70(16):1841-8 Schaeffer and Krylov (2000) Review-Ectoxicology and Environmental Safety. 45, 208-227. | Na-Sp contains two disaccharide repeats: Aldobiuronic acid and Acofriose + other minor saccharides and sodium ion | See cited reference |
| **Cochlodinuium polykrikoides** | Oku-2 | Hasui., et. Al., Int. J. Bio. Macromol. Volume 17 No.5 1995. | mannose, galactose, glucose and uronic acid | Precultures grown in 500 ml conicals containing 300 mls ESM (?) at 21.5°C for 14 days in continuous light (3500 lux) in growth cabinet) and then transferred to 5 liter conical flask containing 3 liters of ESM. Grown 50 days and then filtered thru wortmann GFF filter. |
| **Nostoc muscorum** | PCC⁸74)3, 7936, 8113 | Sangar, VK Applied Micro. (1972) & A.M. Burja et al Tetrahydron 57 (2001) 937-9377; Otero A., J Biotechnol. 2003 Apr 24;102(2):143 - 52 | unknown | Growth in nitrogen fixing conditions in BG-11 medium in aerated cultures maintained in log phase for several months. 250 mL culture media that were disposed in a temperature controlled incubator and continuously illuminated with 70umol photon m-2 s-1 at 30°C. |
| **Cyanospira capsulata** | See cited references | A.M. Burja et al. Tetrahydron 57 937-9377 & Garozzo, D., Carbohydrate Res. 1998 307 113-124. Ascensio, F., Folia Microbiol (Praha). 2004;49(1):64-70., Cesaro, A., et al., Int J Biol Macromol. 1990 Apr; 12(2):79-84 | unknown | See cited reference |
| **Cyanothece sp.** | ATCC 51142 | Ascensio F., Folia Microbiol (Praha). 2004;49(1):64-70. | unknown | Maintained at 27°C in ASN III medium with light/dark cycle of 16/8 h under fluorescent light of 3,000 lux light intensity. In Phillips each of 15 strains were grown photoautotrophically in enriched seawater medium. When required the amount of NaNO3 was reduced from 1.5 to 0.35 g/L. Strains axenically grown in an atmosphere of 95% air and 5% CO2 for 8 days under continuous illumination, with mean photon flux of 30umol photon/m2/s for the first 3 days of growth and 80 umol photon/m/s |
| **Chlorella pyrenoidosa** | UTEX 343; UTEX 1806 | Cheng_2004 Journal of Medicinal Food 7(2) 146-152 | unknown | See cited reference |
| **Phaeodactylum tricornutum** | CCAP 1052/1A | Fabregas et al., Antiviral Research 44(1999)-67-73 | unknown | Cultured in 80 ml glass tubes with aeration of 100m)/min and 10% CO2, for 10s every ten minutes to maintain pH > 7.6. Maintained at 22 in 12:12 Light/dark periodicity. Light at 152.3 umol/m2/s. Salinity 3.5% (nutrient enriched as Fabregas, 1984) |
| **Chlorella autotropica** | USCE | M.A. Guzman-Murillo and F. Ascencio., Letters in Applied Microbiology 2000, 30, 473-478 | unknown | See cited reference |
| **Chlorella sp.** | CCM | M.A. Guzman-Murillo and F. Ascencio., Letters in Applied Microbiology 2000, 30, 473-478 | unknown | See cited reference |
| **Dunalliela tertiolecta** | USCE | M.A. Guzman-Murillo and F. Ascencio., Letters in Applied Microbiology 2000, 30, 473-478 | unknown | See cited reference |
| **Isochrysis galabana** | UTEX LB 987 | Fabregas et al., Antiviral Research 44(1999)-67-73 | unknown | Cultured in 80 ml glass tubes with aeration of 100ml/min and 10% CO₂, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984) |
| **Tetraselmis tetrathele** | CCAP 66/1A-D | Fabregas et al., Antiviral Research 44(1999)-67-73 | unknown | Cultured in 80 ml glass tubes with aeration of 100ml/min and 10% CO₂, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984) |
| **Tetraselmis suecica** | UTEX LB 2286 | M.A. Guzman-Murillo and F. Ascencio., Letters in Applied Microbiology 2000, 30, 473-478 | unknown | See cited reference |
| **Tetraselmis suecica** | CCAP 66/4 | Fabregas et al., Antiviral Research 44(1999)-67-73 and Otero and Fabregas-Aquaculture 159 (1997) 111-123. | unknown | Cultured in 80 ml glass tubes with aeration of 100ml/min and 10% CO₂, for 10s every ten minutes to maintain pH > 7.6. Maintained at 22° in 12:12 Light/dark periodicity. Light at 152.3 umol/m2/s. Salinity 3.5% (nutrient enriched as Fabregas, 1984) |
| **Botrycoccus sudeticus** | UTEX 2629 | M.A. Guzman-Murillo and F. Ascencio., Letters in Applied Microbiology 2000, 30, 473-478 | unknown | See cited reference |
| **Chlamydomon as mexicana** | UTEX 729 | Moore and Tisher Science. 1964 Aug 7;145:586-7. | unknown | See cited reference |
| **Dysmorphococ cus globosus** | UTEX LB 65 | M.A. Guzman-Murillo and F. Ascencio., Letters in Applied Microbiology 2000, 30, 473-478 | unknown | See cited reference |
| **Rhodella reticulata** | UTEX LB 2320 | S. Geresh et al., J Biochem. Biophys. Methods 50 (2002) 179-187 [Review: S. Geresh Biosource Technology 38(1991) 195-201] | unknown | See cited reference |
| **Anabena cylindrica** | ATCC 29414 | Sangar, VK Appl Microbiol. 1972 Nov;24(5):732-4 | In Vegative wall where only 18% is carbohydrateGlucose [35%], mannose [50%], galactose, xylose, and fucose. In heterocyst wall where 73% is carbohydrateGlucose 73% and Mannose is 21% with some galactose and xylose | See cited reference |
| **Anabena flosaquae** | A37; JM Kingsbury Laboratory, Cornell University | Moore, BG[1965] Can J. Microbiol. Dec;11(6):877-85 | Glucose and mannose | See cited reference and APPLIED ENVIRONMENTAL MICROBIOLOGY, Apr. 1978, 718-723) |
| **Palmella mucosa** | See cited references | Sangar, VK Appl Microbiol. 1972 Nov;24(5):732-4 ; Lewin RA., (1956) Can J Microbiol. 2 :665-672 ; Arch Mikrobiol. 1964 Aug 17 ;49 :158-66 | unknown | See cited reference |
| **Anacystis nidulans** | PCC 6301 | Sangar, VK Appl Microbiol. 1972 Nov;24(5):732-4 | Glucose, galactose, mannose | See cited reference |
| **Phormidium 94a** | See cited reference | Vicente-Garcia V. et al., Biotechnol Bioeng. 2004 Feb 5;85(3):306-10 | Galactose, Mannose, Galacturonic acid, Arabinose, and Ribose | Cultivated in 2 L BG-II medium at 28° C. Acetone was added to precipitate exopolysaccharide. |
| **Anabaenaopsis circularis** | 1402/1⁹ | David KA, Fay P. Appl Environ Microbiol. 1977 Dec;34(6):640-6 | unknown | See cited reference |
| **Aphanocapsa halophtia** | MN-11 | Sudo H., et al., Current Micrcobiology Vol. 30 (1995), pp.219-222 | Rhamnose; mannose;fucose; galactose; xylose; glucose In ratio of :15:53:3:3:25 | Cultured aerobically for 20 days in seawater-based medium, with 8% NaCl, and 40 mg/L NaHPO4. Nitrate changed the Exopolysaccharide content. Highest cell density was obtained from culture supplemented with 100 mg/l NaNO₃. Phosphorous (40 mg/L) could be added to control the biomass and exopolysaccharide concentration. |
| **Aphanocapsa sp** | See reference | De Philippis R et al., Sci Total Environ. 2005 Nov 2; | unknown | Incubated at 20 and 28°C with artificial light at u photon flux of 5-20 umol m⁻² s⁻¹. |
| **Cylindrotheca sp** | See reference | De Philippis R et al., Sci Total Environ. 2005 Nov 2; | Glucuronic acid, Galacturonic acid, Glucose, Mannose Arabinose, Fructose and Rhamnose | Stock enriched cultures incubated at 20 and 28°C with artificial light at a photon flux of 5-20 umol m-2 s-1. Exopolysaccharide production done in glass tubes containing 100 mL culture at 28°C with continuous illumination at photon density of 5-10 uE m-2 s-1. |
| **Navicula sp** | See reference | De Philippis R et al., Sci Total Environ. 2005 Nov 2; | Glucuronic acid, Galacturonic acid, Glucose, Mannose, Arabinose, Fructose and Rhamnose | Incubated at 20 and 28°C with artificial light at a photon flux of 5-20 umol m-2 s-1. EPS production done in glass tubes containing 100 mL culture at 28°C with continuous illumination at photon density of 5-10 uE m-2 s-1. |
| **Gloeocapsa sp** | See reference | De Philippis R et al., Sci Total Environ. 2005 Nov 2; | unknown | Incubated at 20 and 28°C with artifical light at a photon flux of 5-20 umol m-2 s-1. |
| **Gloeocapsa alpicola** | See cited references | J Appl Microbiol. 2005;98(1):114-20; Photochem Photobiol. 1982 Mar;35(3):359-64; J Gen Microbiol. 1977 Jan;98( 1):277-80; Arch Microbiol. 1976 Feb; 107(1):93-7; FEMS Microbiol Lett. 2002 Sep 10;214(2):229-33 | unknown | See cited references |
| **Phaeocystis pouchetii** | See cited references | Toxicology. 2004 Jul 1;1 99(2-3):207-17; Toxicon. 2003 Jun;41(7):803-12; Protist. 2002 Sep;153(3):275-82; J Virol. 2005 Jul;79(14):9236-43; J Bacteriol. 1961 Jul;82(1):72-9 | unknown | See cited references |
| **Leptolyngbya sp** | See reference | De Philippis R et al., Sci Total Environ. 2005 Nov 2; | unknown | Incubated at 20 and 28°C with artificial light at a photon flux of 5-20 umol m-2 s-1. |
| **Symploca sp.** | See reference | De Philippis R et al., Sci Total Environ. 2005 Nov 2; | unknown | Incubated at 20 and 28°C with artificial light at a photon flux of 5-20 umol m-2 s-1. |
| **Synechocystis** | PCC 6714/6803 | Jurgens UJ, Weckesser J. J Bacteriol. 1986 Nov; 168(2):568-73 | Glucoseamine, mannosamine, galactosamine, mannose and glucose | Photoautotrophically grown in BG-II medium, pH 7.5 at 25°C. Mass cultures prepared in a 12 liter fermentor and gassed by air and carbon dioxide at flow rates of 250 an d2.5 liters/h, with illumination from white fluorescent lamps at a constant light intensity of 5,000 lux. |
| **Stauroncis decipiens** | See reference | Lind, JL (1997) Planta 203:213-221 | unknown | See cited reference |
| **Achnanthes brevipes** | Indiana University Culture Collection | Holdsworth, RH., Cell Biol. 1968 Jun;37(3):831-7; Acta Cient Venez. 2002;53(1):7-14.; J. Phycol 36 pp. 882-890 (2000) | unknown | See cited reference |
| **Achnanthes longipes** | Strain 330 from National Institute for Environmental Studies | Wang, Y., et al., Plant Physiol. 1997 Apr;113(4):1071-1080. | unknown | See cited reference |

Microalgae are preferably cultured in liquid media for polysaccharide production. Culture condition parameters can be manipulated to optimize total polysaccharide production as well as to alter the structure of polysaccharides produced by microalgae.

Microalgal culture media usually contains components such as a fixed nitrogen source, trace elements, a buffer for pH maintenance, and phosphate. Other components can include a fixed carbon source such as acetate or glucose, and salts such as sodium chloride, particularly for seawater microalgae. Examples of trace elements include zinc, boron, cobalt, copper, manganese, and molybdenum in, for example, the respective forms of ZnCl₂, H₃BO₃, COCl₂·6H₂O, CuCl₂·2H₂O, MnCl₂·4H₂O and (NH₄)₆Mo₇O₂₄·4H₂O.

Some microalgae species can grow by utilizing a fixed carbon source such as glucose or acetate. Such microalgae can be cultured in bioreactors that do not allow light to enter. Alternatively, such microalgae can also be cultured in photobioreactors that contain the fixed carbon source and allow light to strike the cells. Such growth is known as heterotrophic growth. Any strain of microalgae, including those listed in Table 1, can be cultured in the presence of any one or more fixed carbon source including those listed in Tables 2 and 3.

**Table 2**

| |
|---|
| 2,3-Butanediol |
| 2-Aminoethanol |
| 2'-Deoxy Adenosine |
| 3-Methyl Glucose |
| Acetic Acid |
| Adenosine |
| Adenosine-5'-Monophosphate |
| Adonitol |
| Amygdalin |
| Arbutin |
| Bromosuccinic Acid |
| Cis-Aconitic Acid |
| Citric Acid |
| D,L-Carnitine |
| D,L-Lactic Acid |
| D,L-α-Glycerol Phosphate |
| D-Alanine |
| D-Arabitol |
| D-Cellobiose |
| Dextrin |
| D-Fructose |
| D-Fructose-6-Phosphate |
| D-Galactonic Acid Lactone |
| D-Galactose |
| D-Galacturonic Acid |
| D-Gluconic Acid |
| D-Glucosaminic Acid |
| D-Glucose |
| D-Glucose-6-Phosphate |
| D-Glucuronic Acid |
| D-Lactic Acid Methyl Ester |
| D-L-α-Glycerol Phosphate |
| D-Malic Acid |
| D-Mannitol |
| D-Mannose |
| D-Melezitose |
| D-Melibiose |
| D-Psicose |
| D-Raffinose |
| D-Ribose |
| D-Saccharic Acid |
| D-Serine |
| D-Sorbitol |
| D-Tagatose |
| D-Trehalose |
| D-Xylose |
| Formic Acid |
| Gentiobiose |
| Glucuronamide |
| Glycerol |
| Glycogen |
| Glycyl-LAspartic Acid |
| Glycyl-LGlutamic Acid |
| Hydroxy-LProline |
| i-Erythritol |
| Inosine |
| Inulin |
| Itaconic Acid |
| Lactamide |
| Lactulose |
| L-Alaninamide |
| L-Alanine |
| L-Alanylglycine |
| L-Alanyl-Glycine |
| L-Arabinose |
| L-Asparagine |
| L-Aspartic Acid |
| L-Fucose |
| L-Glutamic Acid |
| L-Histidine |
| L-Lactic Acid |
| L-Leucine |
| L-Malic Acid |
| L-Ornithine |
| LPhenylalanine |
| L-Proline |
| L-Pyroglutamic Acid |
| L-Rhamnose |
| L-Serine |
| L-Threonine |
| Malonic Acid |
| Maltose |
| Maltotriose |
| Mannan |
| m-Inositol |
| N-Acetyl-DGalactosamine |
| N-Acetyl-DGlucosamine |
| N-Acetyl-LGlutamic Acid |
| N-Acetyl-β-DMannosamine |
| Palatinose |
| Phenyethylamine |
| p-Hydroxy-Phenylacetic Acid |
| Propionic Acid |
| Putrescine |
| Pyruvic Acid |
| Pyruvic Acid Methyl Ester |
| Quinic Acid |
| Salicin |
| Sebacic Acid |
| Sedoheptulosan |
| Stachyose |
| Succinamic Acid |
| Succinic Acid |
| Succinic Acid |
| Succinic Acid Mono-Methyl-Ester |
| Sucrose |
| Thymidine |
| Thymidine-5'-Monophosphate |
| Turanose |
| Tween 40 |
| Tween 80 |
| Uridine |
| Uridine-5'-Monophosphate |
| Urocanic Acid |
| Water |
| Xylitol |
| α-Cyclodextrin |
| α-D-Glucose |
| α-D-Glucose-1-Phosphate |
| α-D-Lactose |
| α-Hydroxybutyric Acid |
| α-Keto Butyric Acid |
| α-Keto Glutaric Acid |
| α-Keto Valeric Acid |
| α-Ketoglutaric Acid |
| α-Ketovaleric Acid |
| α-Methyl-DGalactoside |
| α-Methyl-DGlucoside |
| α-Methyl-DMannoside |
| β-Cyclodextrinβ-Hydroxybutyric Acid |
| β-Methyl-DGalactoside |
| β-Methyl-D-Glucoside |
| γ-Amino Butyric Acid |
| γ-Hydroxybutyric Acid |

**Table 3**

| |
|---|
| (2-amino-3,4-dihydroxy-5-hydroxymethy)-1-cyclohexyl)glucopyranoside |
| (3,4-disinapoyl)fructofuranosyl-(6-sinapoyl)glucopyranoside |
| (3-sinapoyl)fructofuranosyl-(6-sinapoyl)glucopyranoside |
| 1 reference |
| 1,10-di-O-(2-acetamido-2-deoxyglucopyranosyl)-2-azi-1,10-decanediol |
| 1,3-mannosylmannose |
| 1,6-anhydrolactose |
| 1,6-anhydrolactose hexaacetate |
| 1,6-dichlorosucrose |
| 1-chlorosucrose |
| 1-desoxy-1-glycinomaltose |
| 1-O-alpha-2-acetamido-2-deoxygalactopyranosyl-inositol |
| 1-O-methyl-di-N-trifluoroacetyl-beta-chitobioside |
| 1-propyl-4-O-beta galactopyranosyl-alpha galactopyranoside |
| 2-(acetylamino)-4-O-(2-(acetylamino)-2-deoxy-4-O-sulfogalactopyranosyl)-2-deoxyglucose |
| 2-(trimethylsilyl)ethyl lactoside |
| 2,1',3',4',6'-penta-O-acetylsucrose |
| 2,2'-O-(2,2'-diacetamido-2,3,2',3'-tetradeoxy-6,6'-di-O-(2-tetradecylhexadecanoyl)-alpha,alpha'-trehalose-3,3'-diyl)bis(N-lactoyl-alanyl-isoglutamine) |
| 2,3,6,2',3',4',6'-hepta-O-acetylcellobiose 2,3'-anhydrosucrose |
| 2,3-di-O-phytanyl-1-O-(mannopyranosyl-(2-sulfate)-(1-2)-glucopyranosyl)-sn-glycerol |
| 2,3-epoxypropyl O-galactopyranosyl(1-6)galactopyranoside 2,3-isoprolylideneerthrofuranosyl 2,3-O-isopropylideneerythrofuranoside |
| 2,3-epoxypropyl O-galactopyranosyl(1-6)galactopyranoside 2,3-isoprolylideneerthrofuranosyl 2,3-O-isopropylideneerythrofuranoside |
| 2',4'-dinitrophenyl 2-deoxy-2-fluoro-beta-xylobioside |
| 2,5-anhydromannitol iduronate |
| 2,6-sialyllactose |
| 2-acetamido-2,4-dideoxy-4-fluoro-3-O-galactopyranosylglucopyranose |
| 2-acetamido-2-deoxy-3-O-(gluco-4-enepyranosyluronic acid)glucose |
| 2-acetamido-2-deoxy-3-0-rhamnopyranosylglucose |
| 2-acetamido-2-deoxy-6-O-beta galactopyranosylgalactopyranose |
| 2-acetamido-2-deoxyglucosylgalactitol |
| 2-acetamido-3-O-(3-acetamido-3,6-dideoxy-beta-glucopyranosyl)-2-deoxy-galactopyranose |
| 2-amino-6-O-(2-amino-2-deoxy-glucopyranosyl)-2-deoxyglucose |
| 2-azido-2-deoxymannopyranosyl-(1,4)-rhamnopyranose |
| 2-deoxy-6-O-(2,3-dideoxy-4,6-O-isopropylidene-2,3-(N-tosylepimino)mannopyranosyl)-4,5-O-isopropylidene-1,3-di-N-tosylstreptamine |
| 2-deoxymaltose |
| 2-iodobenzyl-1-thiocellobioside |
| 2-N-(4-benzoyl)benzoyl-1,3-bis(mannos-4-yloxy)-2-propylamine |
| 2-nitrophenyl-2-acetamido-2-deoxy-6-O-beta galactopyranosyl-alpha galactopyranoside |
| 2-O-(glucopyranosyluronic acid)xylose |
| 2-O-glucopyranosylribitol-1-phosphate |
| 2-O-glucopyranosylribitol-4'-phosphate |
| 2-O-rhamnopyranosyl-rhamnopyranosyl-3-hydroxyldecanoyl-3-hydroxydecanoate |
| 2-O-talopyranosylmannopyranoside |
| 2-thiokojibiose |
| 2-thiosophorose |
| 3,3'-neotrehalosadiamine |
| 3,6,3',6'-dianhydro(galactopyranosylgalactopyranoside) |
| 3,6-di-O-methyl-beta-glucopyranosyl-(1-4)-2,3-di-O-methyl-alpha-rhamnopyranose |
| 3-amino-3-deoxyaltropyranosyl-3-amino-3-deoxyaltropyranoside |
| 3-deoxy-3-fluorosucrose |
| 3-deoxy-5-O-rhamnopyranosyl-2-octulopyranosonate |
| 3-deoxyoctulosonic acid-(alpha-2-4)-3-deoxyoctulosonic acid |
| 3-deoxysucrose |
| 3-ketolactose |
| 3-ketosucrose |
| 3-ketotrehalose |
| 3-methyllactose |
| 3-O-(2-acetamido-6-O-(N-acetyineuraminyl)-2-deoxygalactosyl)serine |
| 3-O-(glucopyranosyluronic acid)galactopyranose |
| 3-O-beta-glucuronosylgalactose |
| 3-O-fucopyranosyl-2-acetamido-2-deoxyglucopyranose |
| 3'-O-galactopyranosyl-1-4-O-galactopyranosylcytarabine |
| 3-O-galactosylarabinose 3-O-talopyranosylmannopyranoside |
| 3-trehalosamine |
| 4-(trifluoroacetamido)phenyl-2-acetamido-2-deoxy-4-O-beta-mannopyranosyl-beta-glucopyranoside |
| 4,4',6,6'-tetrachloro-4,4',6,6'-tetradeoxygalactotrehalose |
| 4,6,4',6'-dianhydro(galactopyranosylgalactopyranoside) |
| 4,6-dideoxysucrosc |
| 4,6-O-(1-ethoxy-2-propenylidene)sucrose hexaacetate |
| 4-chloro-4-deoxy-alpha-galactopyranosyl 3,4-anhydro-1,6-dichloro-1,6-dideoxy-beta-lyxo-hexulofuranoside |
| 4-glucopyranosylmannose |
| 4-methylumbelliferylcellobioside |
| 4-nitrophenyl 2-fucopyranosyl-fucopyranoside |
| 4-nitrophenyl 2-O-alpha-D-galactopyranosyl-alpha-D-mannopyranoside |
| 4-nitrophenyl 2-O-alpha-D-glucopyranosyl-alpha-D-mannopyranoside |
| 4-nitrophenyl 2-O-alpha-D-mannopyranosyl-alpha-D-mannopyranoside |
| 4-nitrophenyl 6-O-alpha-D-mannopyranosyl-alpha-D-mannopyranoside |
| 4-nitrophenyl-2-acetamido-2-deoxy-6-O-beta-D-galactopyranosyl-beta-D-glucopyranoside |
| 4-O-(2-acetamido-2-deoxy-beta-glucopyranosyl)ribitol |
| 4-O-(2-amino-2-deoxy-alpha-glucopyranosyl)-3-deoxy-manno-2-octulosonic acid |
| 4-O-(glucopyranosyluronic acid)xylose 4-O-acetyl-alpha-N-acetylneuraminyl-(2-3)-lactose |
| 4-O-alpha-D-galactopyranosyl-D-galactose |
| 4-O-galactopyranosyl-3,6-anhydrogalactose dimethylacetal |
| 4-O-galactopyranosylxylose |
| 4-O-mannopyranosyl-2-acetamido-2-deoxyglucose |
| 4-thioxylobiose 4-trehalosamine |
| 4-trifluoroacetamidophenyl 2-acetamido-4-O-(2-acetamido-2-deoxyglucopyranosyl)-2-deoxymannopyranosiduronic acid |
| 5-bromoindoxyl-beta-cellobioside |
| 5'-O-(fructofuranosyl-2-1-fructofuranosyl)pyridoxine |
| 5-O-beta-galactofuranosyl-galactofuranose |
| 6 beta-galactinol |
| 6(2)-thiopanose |
| 6,6'-di-O-corynomycoloyl-alpha-mannopyranosyl-alpha-mannopyranoside |
| 6,6-di-O-maltosyl-beta-cyclodextrin |
| 6,6'-di-O-mycoloyl-alpha-mannopyranosyl-alpha-marmopyranoside |
| 6-chloro-6-deoxysucrose |
| 6-deoxy-6-fluorosucrose |
| 6-deoxy-alpha-gluco-pyranosiduronic acid |
| 6-deoxy-gluco-heptopyranosyl 6-deoxy-gluco-heptopyranoside |
| 6-deoxysucrose |
| 6-O-decanoyl-3,4-di-O-isobutyrylsucrose |
| 6-O-galactopyranosyl-2-acetamido-2-deoxygalactose |
| 6-O-galactopyranosylgalactose |
| 6-O-heptopyranosylglucopyranose |
| 6-thiosucrose |
| 7-O-(2-amino-2-deoxyglucopyranosyl)heptose |
| 8-methoxycarbonyloctyl-3-O-glucopyranosyl-mannopyranoside |
| 8-O-(4-amino-4-deoxyarabinopyranosyl)-3-deoxyoctulosonic acid |
| allolactose |
| allosucrose |
| allyl 6-O-(3-deoxyoct-2-ulopyranosylonic acid)-(1-6)-2-deoxy-2-(3-hydroxytetradecanamido)glucopyranoside 4-phosphate |
| alpha-(2-9)-disialic acid |
| alpha,alpha-trehalose 6,6'-diphosphate |
| alpha-glucopyranosyl alpha-xylopyranoside |
| alpha-maltosyl fluoride |
| aprosulate |
| benzyl 2-acetamido-2-deoxy-3-O-(2-O-methyl-beta-galactosyl)-beta-glucopyranoside |
| benzyl 2-acetamido-2-deoxy-3-O-betafucopyranosyl-alpha-galactopyranoside |
| benzyl 2-acetamido-6-O-(2-acetamido-2,4-dideoxy-4-fluoroglucopyranosyl)-2-deoxygalactopyranoside |
| benzyl gentiobioside |
| beta-D-galactosyl(1-3)-4-nitrophenyl-N-acetyl-alpha-D-galactosamine |
| beta-methylmelibiose |
| calcium sucrose phosphate |
| camiglibose |
| cellobial |
| cellobionic acid |
| cellobionolactone |
| Cellobiose |
| cellobiose octaacetate |
| cellobiosyl bromide heptaacetate |
| Celsior |
| chitobiose |
| chondrosine |
| Cristolax |
| deuterated methyl beta-mannobioside |
| dextrin maltose |
| D-glucopyranose, O-D-glucopyranosyl |
| Dietary Sucrose |
| difructose anhydride I |
| difructose anhydride III |
| difructose anhydride IV |
| digalacturonic acid |
| DT 5461 |
| ediol |
| epilactose |
| epsilon-N-1-(1-deoxylactulosyl)lysine |
| feruloyl arabinobiose |
| floridoside |
| fructofuranosyl-(2-6)-glucopyranoside |
| FZ 560 |
| galactosyl-(1-3)galactose |
| garamine |
| gentiobiose |
| geranyl 6-O-alpha-L-arabinopyranosyl-beta-D-glucopyranoside |
| geranyl 6-O-xylopyranosyl-glucopyranoside |
| glucosaminyl-1,6-inositol-1,2-cyclic monophosphate |
| glucosyl (1-4) N-acetylglucosamine |
| glucuronosyl(1-4)-rhamnose |
| heptosyl-2-keto-3-deoxyoctonate |
| inulobiose |
| Isomaltose |
| isomaltulose |
| isoprimeverose |
| kojibiose |
| lactobionic acid |
| lacto-N-biose II |
| Lactose |
| lactosylurea |
| Lactulose |
| laminaribiose |
| lepidimoide |
| leucrose |
| levanbiose |
| lucidin 3-O-beta-primveroside |
| LW 10121 |
| LW 10125 |
| LW 10244 |
| maltal |
| maltitol |
| Maltose |
| maltose hexastearate |
| maltose-maleimide |
| maltosylnitromethane heptaacetate |
| maltosyltriethoxycholesterol |
| maltotetraose |
| Malun 25 |
| mannosucrose |
| mannosyl-(1-4)-N-acetylglucosaminyl-(1-N)-urea |
| mannosyl(2)-N-acetyl(2)-glucose |
| melibionic acid |
| Melibiose melibiouronic acid |
| methyl 2-acetamido-2-deoxy-3-O-(alpha-idopyranosyluronic acid)-4-O-sulfo-beta-galactopyranoside |
| methyl 2-acetamido-2-deoxy-3-O-(beta-glucopyranosyluronic acid)-4-O-sulfo-beta-galactopyranoside |
| methyl 2-acetamido-2-deoxy-3-O-glucopyranosyluronoylglucopyranoside |
| methyl 2-O-alpha-rhamnopyranosyl-beta-galactopyranoside |
| methyl 2-O-beta-rhamnopyranosyl-beta-galactopyranoside |
| methyl 2-O-fucopyranosylfucopyranoside 3 sulfate |
| methyl 2-O-mannopyranosylmannopyranoside |
| methyl 2-O-mannopyranosyl-rhamnopyranoside |
| methyl 3-O-(2-acetamido-2-deoxy-6-thioglucopyranosyl)galactopyranoside |
| methyl 3-O-galactopyranosylmannopyranoside |
| methyl 3-O-mannopyranosylmannopyranoside |
| methyl 3-O-mannopyranosyltalopyranoside |
| methyl 3-O-talopyranosyltalopyranoside |
| methyl 4-O-(6-deoxy-manno-heptopyranosyl)galactopyranoside |
| methyl 6-O-acetyl-3-O-benzoyl-4-O-(2,3,4,6-tetra-O-benzoylgalactopyranosyl)-2-deoxy-2-phthalimidoglucopyranoside |
| methyl 6-O-mannopyranosylmannopyranoside |
| methyl beta-xylobioside |
| methyl fucopyranosyl(1-4)-2-acetamido-2-deoxyglucopyranoside |
| methyl laminarabioside |
| methyl O-(3-deoxy-3-fluorogalactopyranosyl)(1-6)galactopyranoside |
| methyl-2-acetamido-2-deoxyglucopyranosyl-1-4-glucopyranosiduronic acid |
| methyl-2-O-fucopyranosylfucopyranoside 4-sulfate |
| MK 458 |
| N(1)-2-carboxy-4,6-dinitrophenyl-N(6)-lactobionoyl-1,6-hexanediamine |
| N-(2,4-dinitro-5-fluorophenyl)-1,2-bis(mannos-4'-yloxy)propyl-2-amine |
| N-(2'-mercaptoethyl)lactamine |
| N-(2-nitro-4-azophenyl)-1,3-bis(mannos-4'-yloxy)propyl-2-amine |
| N-(4-azidosalicylamide)-1,2-bis(mannos-4'-yloxy)propyl-2-amine |
| N,N-diacetylchitobiose |
| N-acetylchondrosine |
| N-acetyldermosine |
| N-acetylgalactosaminyl-(1-4)-galactose |
| N-acetylgalactosaminyl-(1-4)-glucose |
| N-acetylgalactosaminyl-1-4-N-acetylglucosamine |
| N-acetylgalactosaminyl-1-4-N-acetylglucosamine |
| N-acetylgalactosaminyl-alpha(1-3)galactose |
| N-acetylglucosamine-N-acetylmuramyl-alanyl-isoglutaminyl-alanyl-glycerol dipalmitoyl |
| N-acetylglucosaminyl beta(1-6)N-acetylgalactosamine |
| N-acetylglucosaminyl-1-2-mannopyranose |
| N-acetylhyalobiuronic acid |
| N-acetylneuraminoyllactose |
| N-acetylneuraminoyllactose sulfate ester |
| N-acetylneuraminyl-(2-3)-galactose |
| N-acetylneuraminyl-(2-6)-galactose |
| N-benzyl-4-O-(beta-galactopyranosyl)glucamine-N-carbodithioate |
| neoagarobiose |
| N-formylkansosaminyl-(1-3)-2-O-methylrhamnopyranose |
| O-((Nalpha)-acetylglucosamine 6-sulfate)-(1-3)-idonic acid |
| O-(4-O-feruloyl-alpha-xylopyranosy1)-(1-6)-glucopyranose |
| O-(alpha-idopyranosyluronic acid)-(1-3)-2,5-anhydroalditol-4-sulfate |
| O-(glucuronic acid 2-sulfate)-(1--3)-O-(2,5)-andydrotalitol 6-sulfate |
| O-(glucuronic acid 2-sulfate)-(1--4)-O-(2,5)-anhydromannitol 6-sulfate |
| O-alpha-glucopyranosyluronate-(1-2)-galactose |
| O-beta-galactopyranosyl-(1-4)-O-beta-xylopyranosyl-(1-0)-serine |
| octyl maltopyranoside |
| O-demethylpaulomycin A |
| O-demethylpaulomycin B |
| O-methyl-di-N-acetyl beta-chitobioside |
| Palatinit |
| paldimycin |
| paulomenol A |
| paulomenol B |
| paulomycin A |
| paulomycin A2 |
| paulomycin B |
| paulomycin C |
| paulomycin D |
| paulomycin E |
| paulomycin F |
| phenyl 2-acetamido-2-deoxy-3-O-beta-D-galactopyranosyl-alpha-D-galactopyranoside |
| phenyl O-(2,3,4,6-tetra-O-acetylgalactopyranosyl)-(1-3)-4,6-di-O-acetyl-2-deoxy-2-phthalimido-1-thioglucopyranoside |
| poly-N-4-vinylbenzyllactonamide |
| pseudo-cellobiose |
| pseudo-maltose |
| rhamnopyranosyl-(1-2)-rhamnopyranoside-(1-methyl ether) |
| rhoifolin |
| ruberythric acid |
| S-3105 |
| senfolomycin A |
| senfolomycin B |
| solabiose |
| SS 554 |
| streptobiosamine |
| Sucralfate |
| Sucrose |
| sucrose acetate isobutyrate |
| sucrose caproate |
| sucrose distearate |
| sucrose monolaurate |
| sucrose monopalmitate |
| sucrose monostearate |
| sucrose myristate |
| sucrose octaacetate |
| sucrose octabenzoic acid |
| sucrose octaisobutyrate |
| sucrose octasulfate |
| sucrose polyester |
| sucrose sulfate |
| swertiamacroside |
| T-1266 |
| tangshenoside I |
| tetrahydro-2-((tetrahydro-2-furanyl)oxy)-2H-pyran |
| thionigerose |
| Trehalose |
| trehalose 2-sulfate |
| trehalose 6,6'-dipalmitate |
| trehalose-6-phosphate |
| trehalulose |
| trehazolin |
| trichlorosucrose |
| tunicamine |
| turanose |
| U 77802 |
| U 77803 |
| xylobiose |
| xylose-glucose |
| xylosucrose |

Microalgae contain photosynthetic machinery capable of metabolizing photons, and transferring energy harvested from photons into fixed chemical energy sources such as monosaccharide. Glucose is a common monosaccharide produced by microalgae by metabolizing light energy and fixing carbon from carbon dioxide. Some microalgae can also grow in the absence of light on a fixed carbon source that is exogenously provided (for example see Plant Physiol. 2005 Feb;137(2):460-74). In addition to being a source of chemical energy, monosaccharides such as glucose are also substrate for production of polysaccharides (see Example 14). The disclosure provides methods of producing
polysaccharides with novel monosaccharide compositions. For example, microalgae is cultured in the presence of culture media that contains exogenously provided monosaccharide, such as glucose. The monosaccharide is taken up by the cell by either active or passive transport and incorporated into polysaccharide molecules produced by the cell. This novel method of polysaccharide composition manipulation can be performed with,
for example, any microalgae listed in Table 1 using any monosaccharide or disaccharide listed in Tables 2 or 3.

In one disclosure, the fixed carbon source is one or more selected from glucose, galactose, xylose, mannose, rhamnose, N-acetylglucosamine, glycerol, floridoside, and glucuronic acid. The methods may be practiced cell growth in the presence of at least about
5.0 µM, at least about 10 µM at least about 15.0 µM, at least about 20.0 µM, at least about
25.0 µM, at least about 30.0 µM, at least about 35.0 µM, at least about 40.0 µM, at least about
45.0 µM, at least about 50.0 µM, at least about 55.0 µM, at least about 60.0 µM, at least about
75.0 µM, at least about 80.0 µM, at least about 85.0 µM, at least about 90.0 µM, at least about
95.0 µM, at least about 100.0 µM, or at least about 150.0 µM, of one or more
exogenously provided fixed carbon sources selected from Tables 2 and 3.

In some embodiments using cells of the genus Porphyridium, the methods include the use of approximately 0.5-0.75% glycerol as a fixed carbon source when the cells are cultured in the presence of light. Alternatively, a range of glycerol, from approximately 4.0% to approximately 9.0% may be used when the Porphyridium cells are cultured in the dark, more preferably from 5.0% to 8.0%, and more preferably 7.0%.

After culturing the microalgae in the presence of the exogenously provided carbon source, the monosaccharide composition of the polysaccharide can be analyzed as described in Example 5.

Microalgae culture media can contain a fixed nitrogen source such as KNO₃. Alternatively, microalgae are placed in culture conditions that do not include a fixed nitrogen source. For example, *Porphyridium sp.* cells are cultured for a first period of time in the presence of a fixed nitrogen source, and then the cells are cultured in the absence of a fixed nitrogen source (see for example Adda M., Biomass 10:131-140. (1986); Sudo H., et al., Current Microbiology Vol. 30 (1995), pp. 219-222; Marinho-Soriano E., Bioresour Technol. 2005 Feb;96(3):379-82; Bioresour. Technol. 42:141-147 (1992)). While the invention is not limited by theory, it is well accepted by those skilled in the art that the red color of *Porphyridium* is due to the red pigmented light harvesting protein phycoerythrin (for example see Fujimori and Pecci, Distinct subunits of phycoerythrin from Porphyridium cruentum and their spectral characteristics, Arch. Biochem. Biophys. 118, 448-55 1967). Culture of *Porphyridium* in the presence of reduced levels of nitrogen causes cells to degrade phycoerythrin, resulting in a significant decrease in the amount of red pigmentation. Because phycoerythrin constitutes over 2% of the dry weight of *Porphyridium* cells under nitrogen-replete conditions (see for example M.M. Rebolloso Fuentes 2000, Food Chemistry, 70;345-353), this catabolic process allows a significant amount of fixed nitrogen to be recycled. Again while the invention is not limited by theory, providing excess light also causes *Porphyridium* cells to degrade phycoerythrin to reduce the amount of light harvesting per cell. This process reduces oxidative stress caused by excess photon flux in the thylakoid membrane. *Porphyridium* biomass grown in nitrogen-limited conditions, particularly when grown under high light, lose red coloration and turn yellow with occasional shades of light brown, referred to as "decolorized biomass". The invention provides novel methods of production of compositions for topical application including culturing cells of the genus *Porphyridium* under reduced levels of nitrogen (such as, for example, no Tris and less than 20% of the KNO₃ per liter of ATCC 1495 ASW media) and optionally also under relatively light conditions such as for example 130 µE m⁻² s⁻¹. In other embodiments, the culture media contains no more than 300 mg/L of one or more nitrate-containing compounds that can be metabolized by the cells (such as, for example, but not limited to KNO₃) at inoculation, no more than 250 mg/L at inoculation, no more than 200 mg/L at inoculation, no more than 175 mg/L at inoculation, no more than 150 mg/L at inoculation, no more than 135 mg/L at inoculation, no more than 125 mg/L at inoculation, no more than 50 mg/L at inoculation, no more than 25 mg/L at inoculation, and no more than 12.5 mg/L at inoculation. In some methods the nitrate-containing compounds are provided in the culture media at inoculation at approximately 180mg/L, approximately 160 mg/L, approximately 140 mg/L, approximately 130 mg/L, approximately 125 mg/L, approximately 110 mg/L, approximately 100 mg/L, and approximately 90 mg/L. In other embodiments, the culture media contains no more than 300 millimolar of one or more nitrate-containing compounds that can be metabolized by the cells (such as, for example, but not limited to KNO₃) at inoculation, no more than 250 millimolar at inoculation, no more than 200 millimolar at inoculation, no more than 175 millimolar at inoculation, no more than millimolar at inoculation, no more than millimolar at inoculation, no more than millimolar at inoculation, no more than 50 millimolar at inoculation, no more than 25 millimolar at inoculation, and no more than 12.5 millimolar at inoculation. In some methods the nitrate-containing compounds are provided in the culture media at inoculation at approximately 180 millimolar, approximately 160 millimolar, approximately 140 millimolar, approximately 130 millimolar, approximately 125 millimolar, approximately 110 millimolar, approximately 100 millimolar, and approximately 90 millimolar. Inoculation can mean when seed cells are infused into a bioreactor, and can also mean when cells grown in nitrogen-replete media that have been pelleted, optionally washed, and are resuspended in culture media that has limiting amounts of nitrogen-containing compounds or no nitrogen-containing compounds. Cells inoculated into media containing limiting amounts of nitrogen-containing compounds, such as 125 mg/L, will typically undergo cell division until nitrogen is used up (assuming other nutrients are not limiting), and then begin the "bleaching" process in which phycoerythrin is degraded. This process is accelerated as light intensity is increased. Cells grown in nitrogen-replete media can also be harvested and washed and resuspended in culture media that contains no nitrogen or a limited amount of nitrogen such as the amounts listed above. In addition, nitrogen-replete media can be exchanged with nitrogen-limited media through tangential flow filtration using a filter that has a pore size smaller than the diameter of the cells. The diameter of *Porphyridium* cells is approximately 4-8 microns. This method avoids centrifugation and reduces the possibility of contamination.

Other methods include removing red coloration from different species of microalgae through mutagenesis. For example, species of the genus Porphyridium are subjected to chemical mutagenesis, followed by screening for colonies lacking red coloration. See for example Sivan and Arad, Phycologia 32(1), pp.68-72 (1993). Such genetically decolorized strains are used to generate non-red biomass for formulation as skin care products. In a preferred embodiment the genetically decolorized biomass is homogenized. In another preferred embodiment the polysaccharide contains more than 4.75% sulfur by weight. Production of phycoerythrin is reduced in some mutagenized strains due to mutations in various regions of the genome including promoters, coding regions, and other functional elements. Both bleaching through nutrient limitation and excess light, as well as through mutagenesis, can be performed on any microalgae species, including those listed in Table 1.

Some methods further comprise formulating decolorized *Porphyridium* biomass, generated through any or all of the methods nutrient limitation, excess light, and mutagenesis, with a carrier suitable for topical administration. The methods also optionally include formulating decolorized Porphyridium biomass with one or more preservatives, such as for example diiodomethyl-p-tolylsulfone, 2-Bromo-2-nitropropane-1,3-diol, cis isomer 1-(3-ehloroallyl)-3,5,7-triaza-1-azoniaadamantane chloride (a.k.a. Dowicil 200), glutaraldehyde, 4,4-dimethyl oxazolidine, 7-Ethylbicyclooxazolidine, methyl paraben, sorbic acid, methyl paraben, Germaben II, and disodium EDTA.

Other culture parameters can also be manipulated, such as the pH of the culture media, the identity and concentration of trace elements such as those listed in Table 3, and other media constituents. One non-limiting example is the inclusion of at least one source of sulfate in the culture media to increase the level of sulfation in the polysaccharides produced.

In some embodiments, a polysaccharide preparation method is practiced with culture media containing more than about 100, more than about 150, more than about 200, more than about 250, more than about 300, more than about 350, more than about 400, more than about 450, more than about 500, more than about 550, more than about 600, more than about 650, more
than about 700, or more than about 750, more than about 800, more than about 850, more
than about 900, more than about 950, or more than about 1 or 2 M sulfate (or total S0₄²⁻). Increasing the sulfation has been demonstrated to increase the antioxidant capacity of the polysaccharide (see example 23 and Spitz et al. (J. Appl. Phycology (2005) 17:215-222). Without being bound by theory, and offered to improved the understanding of certain aspects
of the disclosure, it is possible that an increased level of sulfation may increase the anti-cholesterol characteristics of the homogenized cell material or polysaccharide preparation disclosed herein. The correlation between higher amounts of sulfation and antioxidant activity demonstrated herein was unexpected given the weak antioxidant activity of carrageenan, which contains as much as 40% sulfate.

It is believed that microalgae of the genus Porphyridium have not been grown or propagated under conditions with sulfate concentrations of 100 mM to 2 M. The surprising discovery that microalgae are capable of growth under such conditions is disclosed. Additionally, the disclosure is based in part on the surprising discovery that growth under higher sulfate concentrations can produce polysaccharides with higher levels of
sulfation. This allows for the production of cells (and so biomass) containing highly sulfated polysaccharides that may be used in the form of purified polysaccharides, a homogenate of cells (biomass), intact cells (biomass)per *se*, or a combination thereof.

The discovery that Porphyridium can survive above 100mM sulfate was surprising for a number of reasons. First, it is known that sulfate can alter the rate of uptake of toxic metal ions in algae, such as chromium, and that increasing metal accumulation can lead to toxicity (see for example Kaszycki et al, Plant, Cell & Environment, 28(2): p.260, 2005). Second, it is also known that sulfate can inhibit the uptake of metal ions required for nitrogen fixation such as molybdenum, and that increasing sulfate concentrations negatively affects algae such
as cyanobacteria even at sulfate concentrations in estuarine (>8-10 mM) and seawater (28mM) levels of sulfate (see for example Marino et al, Hydrobiologia 500: pp. 277-293, 2004). Third, sulfate at high levels can often be taken up and reduced and sulfide, which is toxic to photosynthesis because it attacks photosystem II (see for example Khanal et al., J. Envir. Engrg., 129(12); pp. 1104-1111, 2003). Fourth, high sulfate levels alter the osmotic pressure of the growth media, and many organisms cannot survive at such an elevated osmoticum. For example, it is well established that photosynthesis of algae is inhibited by hyperosmotic and salt stresses. See for example "Suppression of Quantum Yield of Photosystem II by Hyperosmotic Stress in Chlamydomonas reinhardtii" Plant Cell Physiol. 36: pp.1253-1258 (1995); and "The Effect of Osmotic and Ionic Stress on the Primary Processes of Photosynthesis in Dunaliella tertiolecta ", J. Exp. Bot. 1984 35(1): 18-27 (1984).

By use of methods described above, the disclosure includes a preparation of cells, cell biomass, or cell homogenate containing a reduced level of green pigmentation, or a reduced absorbance at 545 nm, relative to the same cells grown under different conditions.
The cells may be those of any microalgae as described herein, including those of the genus *Porphyridium.* The cells, cell biomass, or cell homogenate may be formulated into a composition of the disclosure such that an aqueous extract of the composition would contain the same reduced level of green pigmentation.

In some embodiments, an aqueous extract of the composition contains no more than about 75%, no more than about 70%, no more than about 65%, no more than about 60%, no more than about 55%, no more than about 50%, no more than about 45%, no more than about 40%, no more than about 35%, no more than about 30%, no more than about 25%, no more than about 20%, no more than about 15%, no more than about 10%, or no more than about 5% of the absorbance per gram at 545nm compared to an extract of cells of the same species
grown in a photobioreactor in ATCC 1495 ASW media (as described in Example 1) in the presence of 50 microeinsteins of light per second per square meter. One non-limiting means for detection of absorbance is by use of a spectrophotometer.

Microalgae can be grown in the presence of light. The number of photons striking a culture of microalgae cells can be manipulated, as well as other parameters such as the wavelength spectrum and ratio of dark:light hours per day. Microalgae can also be cultured in natural light, as well as simultaneous and/or alternating combinations of natural light and artificial light. For example, microalgae of the genus *Chlorella* be cultured under natural light during daylight hours and under artificial light during night hours.

The gas content of a photobioreactor can be manipulated. Part of the volume of a photobioreactor can contain gas rather than liquid. Gas inlets can be used to pump gases into the photobioreactor. Any gas can be pumped into a photobioreactor, including air, air/CO₂ mixtures, noble gases such as argon and others. The rate of entry of gas into a photobioreactor can also be manipulated. Increasing gas flow into a photobioreactor increases the turbidity of a culture of microalgae. Placement of ports conveying gases into a photobioreactor can also affect the turbidity of a culture at a given gas flow rate. Air/CO₂ mixtures can be modulated to generate different polysaccharide compositions by manipulating carbon flux. For example, air:CO₂ mixtures of about 99.75% air:0.25% CO₂, about 99.5% air:0.5% CO₂, about 99.0% air: 1.00% CO₂, about 98.0% air:2.0% CO₂, about 97.0% air:3.0% CO₂, about 96.0% air:4.0% CO₂, and about 95.00% air:5.0% CO₂ can be infused into a bioreactor or photobioreactor.

Microalgae cultures can also be subjected to mixing using devices such as spinning blades and propellers, rocking of a culture, stir bars, and other instruments.

### B. Cell Culture Methods: Photobioreactors

Microalgae can be grown and maintained in closed photobioreactors made of different types of transparent or semitransparent material. Such material can include Plexiglas® enclosures, glass enclosures, bags bade from substances such as polyethylene, transparent or semitransparent pipes, and other materials. Microalgae can also be grown in open ponds.

Photobioreactors can have ports allowing entry of gases, solids, semisolids and liquids into the chamber containing the microalgae. Ports are usually attached to tubing or other means of conveying substances. Gas ports, for example, convey gases into the culture. Pumping gases into a photobioreactor can serve to both feed cells CO₂ and other gases and to aerate the culture and therefore generate turbidity. The amount of turbidity of a culture varies as the number and position of gas ports is altered. For example, gas ports can be placed along the bottom of a cylindrical polyethylene bag. Microalgae grow faster when CO₂ is added to air and bubbled into a photobioreactor. For example, a 5% CO₂:95% air mixture is infused into a photobioreactor containing cells of the genus Porphyridium (see for example Biotechnol Bioeng. 1998 Sep 20;59(6):705-13; textbook from office; US Patents 5,643,585 and 5,534,417; Lebeau, T., et. al. Appl. Microbiol Biotechnol (2003) 60:612-623; Muller-Fuega, A., Journal of Biotechnology 103 (2003 153-163; Muller-Fuega, A., Biotechnology and Bioengineering, Vol. 84, No. 5, December 5, 2003; Garcia-Sanchez, J.L., Biotechnology and Bioengineering, Vol. 84, No. 5, December 5, 2003; Garcia-Gonzales, M., Journal of Biotechnology, 115 (2005) 81-90. Molina Grima, E., Biotechnology Advances 20 (2003) 491-515).

Photobioreactors can be exposed to one or more light sources to provide microalgae with light as an energy source via light directed to a surface of the photobioreactor. Preferably the light source provides an intensity that is sufficient for the cells to grow, but not so intense as to cause oxidative damage or cause a photoinhibitive response. In some instances a light source has a wavelength range that mimics or approximately mimics the range of the sun. In other instances a different wavelength range is used. Photobioreactors can be placed outdoors or in a greenhouse or other facility that allows sunlight to strike the surface. Photon intensities are typically measured in microeinsteins of light per square meter per second (uE m⁻² s⁻¹) although other measurements such as lux and footcandles are sometimes used. Preferred photon intensities for culturing species of the genus *Porphyridium* are between 50 and 300 uE m⁻² s⁻¹ (see for example Photosynth Res. 2005 Jun;84(1-3):21-7), although in cases of inducing Porphyridium cells to degrade phycoerythrin preferred light intensities can be higher, such as for example 400-700 uE m⁻² s⁻¹.

Photobioreactors preferably have one or more parts that allow media entry. It is not necessary that only one substance enter or leave a port. For example, a port can be used to flow culture media into the photobioreactor and then later can be used for sampling, gas entry, gas exit, or other purposes. In some instances a photobioreactor is filled with culture media at the beginning of a culture and no more growth media is infused after the culture is inoculated. In other words, the microalgal biomass is cultured in an aqueous medium for a period of time during which the microalgae reproduce and increase in number; however quantities of aqueous culture medium are not flowed through the photobioreactor throughout the time period. Thus in some embodiments, aqueous culture medium is not flowed through the photobioreactor after inoculation.

In other instances culture media can be flowed though the photobioreactor throughout the time period during which the microalgae reproduce and increase in number. In some instances media is infused into the photobioreactor after inoculation but before the cells reach a desired density. In other words, a turbulent flow regime of gas entry and media entry is not maintained for reproduction of microalgae until a desired increase in number of said microalgae has been achieved, but instead a parameter such as gas entry or media entry is altered before the cells reach a desired density.

Photobioreactors preferably have one or more ports that allow gas entry. Gas can serve to both provide nutrients such as CO₂ as well as to provide turbulence in the culture media. Turbulence can be achieved by placing a gas entry port below the level of the aqueous culture media so that gas entering the photobioreactor bubbles to the surface of the culture. One or more gas exit ports allow gas to escape, thereby preventing pressure buildup in the photobioreactor. Preferably a gas exit port leads to a "one-way" valve that prevents contaminating microorganisms to enter the photobioreactor. In some instances cells are cultured in a photobioreactor for a period of time during which the microalgae reproduce and increase in number, however a turbulent flow regime with turbulent eddies predominantly throughout the culture media caused by gas entry is not maintained for all of the period of time. In other instances a turbulent flow regime with turbulent eddies predominantly throughout the culture media caused by gas entry can be maintained for all of the period of time during which the microalgae reproduce and increase in number. In some instances a predetermined range of ratios between the scale of the photobioreactor and the scale of eddies is not maintained for the period of time during which the microalgae reproduce and increase in number. In other instances such a range can be maintained.

Photobioreactors preferably have at least one port that can be used for sampling the culture. Preferably a sampling port can be used repeatedly without altering compromising the axenic nature of the culture. A sampling port can be configured with a valve or other device that allows the flow of sample to be stopped and started. Alternatively a sampling port can allow continuous sampling. Photobioreactors preferably have at least one port that allows inoculation of a culture. Such a port can also be used for other purposes such as media or gas entry.

Microalgae that produce polysaccharides can be cultured in photobioreactors. Microalgae that produce polysaccharide that is not attached to cells can be cultured for a period of time and then separated from the culture media and secreted polysaccharide by methods such as centrifugation and tangential flow filtration. Preferred organisms for culturing in photobioreactors to produce polysaccharides include *Porphyridium sp., Porphyridium cruentum*, *Porphyridium purpureum*, *Porphyridium aerugineum, Rhodella maculata, Rhodella reticidata, Chlorella autotrophica, Chlorella stigmatophora, Chlorella capsulata, Achnanthes brevipes, Achnanthes longipes, Gloeocapsa alpicola and Phaeocysstis pouchettii.*

### C. Non-Microalgal Polysaccharide Production

Organisms besides microalgae can be used to produce polysaccharides, such as lactic acid bacteria (see for example Stinglee, F., Molecular Microbiology (1999) 32(6), 1287-1295; Ruas_Madiedo, P., J. Dairy Sci. 88:843-856 (2005); Laws, A., Biotechnology Advances 19 (2001) 597-625; Xanthan gum bacteria: Pollock, TJ., J. Ind. Microbiol Biotechnol., 1997 Aug;19(2):92-7.; Becker, A., Appl. Micrbiol. Bioltechnol. 1998 Aug;50(2):92-7; Garcia-Ochoa, F., Biotechnology Advances 18 (2000) 549-579., seaweed: Talarico, LB., et al., Antiviral Research 66 (2005) 103-110; Dussealt, J., et al., J Biomed Mater Res A., (2005) Nov1; Melo, F.R., J Biol Chem 279:20824-35 (2004)).

### D. Ex Vivo Methods

Microalgae and other organisms can be manipulated to produce polysaccharide molecules that are not naturally produced by methods such as feeding cells with monosaccharides that are not produced by the cells (Nature. 2004 Aug 19;430(7002):873-7). For example, species listed in Table 1 are grown according to the referenced growth protocols, with the additional step of adding to the culture media a fixed carbon source that is not in the culture media as published and referenced in Table 1 and is not produced by the cells in a detectable amount.

### E. In vitro methods

Polysaccharides can be altered by enzymatic and chemical modification. For example, carbohydrate modifying enzymes can be added to a preparation of polysaccharide and allowed to catalyze reactions that alter the structure of the polysaccharide. Chemical methods can be used to, for example, modify the sulfation pattern of a polysaccharide (see for example Carbohydr. Polym. 63:75-80 (2000); Pomin VH., Glycobiology. 2005 Dec;15(12):1376-85; Naggi A., Semin Thromb Hemost. 2001 Oct;27(5):437-43 Review; Habuchi, O., Glycobiology. 1996 Jan;6(1);51-7; Chen, J., J. Biol. Chem. In press; Geresh., S et al., J. Biochem. Biophys. Methods 50 (2002) 179-187.).

### F. Polysaccharide Purification Methods

Exopolysaccharides can be purified from microalgal cultures by various methods, including those disclosed herein.

### Precipitation

For example, polysaccharides can be precipitated by adding compounds such as cetylpyridinium chloride, isopropanol, ethanol, or methanol to an aqueous solution containing a polysaccharide in solution. Pellets of precipitated polysaccharide can be washed and resuspended in water, buffers such as phosphate buffered saline or Tris, or other aqueous solutions (see for example Farias, W.R.L., et al., J. Biol. Chem. (2000) 275;(38)29299-29307; U.S. Patent No. 6,342,367; U.S. Patent No. 6,969,705).

### Dialysis

Polysaccharides can also be dialyzed to remove excess salt and other small molecules (see for example Gloaguen, V., et al., Carbohydr Res. 2004 Jan 2;339(1):97-103; Microbiol Immunol. 2000;44(5):395-400.).

### Tangential Flow Filtration

Filtration can be used to concentrate polysaccharide and remove salts. For example, tangential flow filtration (TFF), also known as cross-flow filtration, can be used)). For a preferred filtration method see Geresh, Carb. Polym. 50; 183-189 (2002), which discusses use of a MaxCell A/G technologies 0.45uM hollow fiber filter. Also see for example Millipore Pellicon® devices, used with 100kD, 300kD, 1000 kD (catalog number P2C01MC01), 0.1 uM (catalog number P2VVPPV01), 0.22uM (catalog number P2GVPPV01), and 0.45uM membranes (catalog number P2HVMPV01). It is preferred that the polysaccharides do not pass through the filter at a significant level. It is also preferred that polysaccharides do not adhere to the filter material. TFF can also be performed using hollow fiber filtration systems.

Non-limiting examples of tangential flow filtration include use of a filter with a pore size of at least about 0.1 micrometer, at least about 0.12 micrometer, at least about 0.14 micrometer, at least about 0.16 micrometer, at least about 0.18 micrometer, at least about 0.2 micrometer, at least about 0.22 micrometer, or at least about 0.45 micrometer. Preferred pore sizes of TFF allow contaminants to pass through but not polysaccharide molecules.

### Ion Exchange Chromatography

Anionic polysaccharides can be purified by anion exchange chromatography. (Jacobsson, I., Biochem J. 1979 Apr 1; 179(1):77-89; Karamanos, NK., Eur J Biochem. 1992 Mar. 1;204(2):553-60).

### Protease Treatment

Polysaccharides can be treated with proteases to degrade contaminating proteins. In some instances the contaminating proteins are attached, either covalently or noncovalently to polysaccharides. In other instances the polysaccharide molecules are in a preparation that also contains proteins. Proteases can be added to polysaccharide preparations containing proteins to degrade proteins (for example, the protease from *Streptomyces griseus* can be used (SigmaAldrich catalog number P5147). After digestion, the polysaccharide is preferably purified from residual proteins, peptide fragments, and amino acids. This purification can be accomplished, for example, by methods listed above such as dialysis,
filtration, and precipitation.

Heat treatment can also be used to eliminate proteins in polysaccharide preparations (see for example Biotechnol Lett. 2005 Jan;27(1):13-8; FEMS Immunol Med Microbiol. 2004 Oct 1;42(2): 155-66; Carbohydr Res. 2000 Sep 8;328(2): 199-207; J Biomed Mater Res. 1999;48(2):111-6.; Carbohydr Res. 1990 Oct 15;207(1):101-20;).
Production of an exopolysaccharide comprising
separating the exopolysaccharide from contaminants after proteins attached to the exopolysaccharide have been degraded or destroyed is disclosed. The proteins may be those attached to the exopolysaccharide during culture of a microalgal cell in media, which is first separated from the cells prior to proteolysis or protease treatment. The cells may be those of the genus
*Porphyridium* as a non-limiting example.

In one non-limiting example, a method of producing an exopolysaccharide is provided wherein the method comprises culturing cells of the genus *Porphyridium;* separating cells from culture media; destroying protein attached to the exopolysaccharide present in the culture media; and separating the exopolysaccharide from contaminants. In some methods, the contaminant(s) are selected from amino acids, peptides, proteases, protein fragments, and salts. In other methods, the contaminant is selected from NaCl, MgSO₄, MgCl₂, CaCl2, KNO3, KH2PO4, NaHCO₃, Tris, ZnCl₂, H3B03, CoCl₂, CuCl₂, MnCl₂, (NH₄) _{&}Mo₇O2₄, FeC13 and EDTA.

### Drying Methods

After purification of methods such as those above, polysaccharides can be dried using methods such as lyophilization and heat drying (see for example Shastry, S., Brazilian Journal of Microbiology (2005) 36:57-62; Matthews KH.,Int J Pharm. 2005 Jan 31;289(1-2):51-62. Epub 2004 Dec 30; Gloaguen, V., et al., Carbohydr Res. 2004 Jan 2;339(1):97-103).

Tray dryers accept moist solid on trays. Hot air (or nitrogen) can be circulated to dry. Shelf dryers can also employ reduced (below atmospheric at sea level, such as at about 25 in Hg or less) pressure or vacuum to dry at room temperature when products are temperature sensitive and are similar to a freeze-drier but less costly to use and can be easily scaled-up. In some embodiments drying in oven tray dryers is performed under vacuum.

Spray dryers are relatively simple in operation, which accept feed in fluid state and convert it into a dried particulate form by spraying the fluid into a hot drying medium.

Rotary dryers operate by continuously feeding wet material, which is dried by contact with heated air, while being transported along the interior of a rotating cylinder, with the rotating shell acting as the conveying device and stirrer.

Spin flash dryers are used for drying of wet cake, slurry, or paste which is normally difficult to dry in other dryers. The material is fed by a screw feeder through a variable speed drive into the vertical drying chamber where it is heated by air and at the same time disintegrated by a specially designed disintegrator. The heating of air may be direct or indirect depending upon the application. The dry powder is collected through a cyclone separator/bag filter or with a combination of both.

### Whole Cell Extraction

Intracellular polysaccharides and cell wall polysaccharides can be purified from whole cell mass (see form example U.S. Patent 4,992,540; U.S. Patent 4,810,646; J Sietsma JH., et al., Gen Microbiol. 1981 Jul;125(1):209-12; Fleet GH, Manners DJ., J Gen Microbiol. 1976 May;94(1):180-92).

### G. Microalgae Homogenization Methods

A pressure disrupter pumps of a slurry through a restricted orifice valve. High pressure (up to 1500 bar) is applied, followed by an instant expansion through an exiting nozzle. Cell disruption is accomplished by three different mechanisms: impingement on the valve, high liquid shear in the orifice, and sudden pressure drop upon discharge, causing an explosion of the cell. The method is applied mainly for the release of intracellular molecules. According to Hetherington et al., cell disruption (and consequently the rate of protein release) is a first-order process, described by the relation: log[Rm/(Rm-R)] = K N P72.9. R is the amount of soluble protein; Rm is the maximum amount of soluble protein K is the temperature dependent rate constant; N is the number of passes through the homogenizer (which represents the residence time). P is the operating pressure.

In a ball mill, cells are agitated in suspension with small abrasive particles. Cells break because of shear forces, grinding between beads, and collisions with beads. The beads disrupt the cells to release biomolecules. The kinetics of biomolecule release by this method is also a first-order process.

Another widely applied method is the cell lysis with high frequency sound that is produced electronically and transported through a metallic tip to an appropriately concentrated cellular suspension, ie: sonication. The concept of ultrasonic disruption is based on the creation of cavities in cell suspension. Homogenization can also be performed with a Microfluidizer® device (such as the M-110Y Microfluidizer® model, Microfluidics Inc., Newton, MA).

Blending (high speed or Waring), the french press, or even centrifugation in the case of weak cell walls, also disrupt the cells by using the same concepts.

Cells can also be ground after drying in devices such as a colloid mill.

Because the percentage of polysaccharide as a function of the dry weight of a microalgae cell can frequently be in excess of 50%, microalgae cell homogenates can be considered partially pure polysaccharide compositions. Cell disruption aids in increasing the amount of solvent-accessible polysaccharide by breaking apart cell walls that are largely composed of polysaccharide.

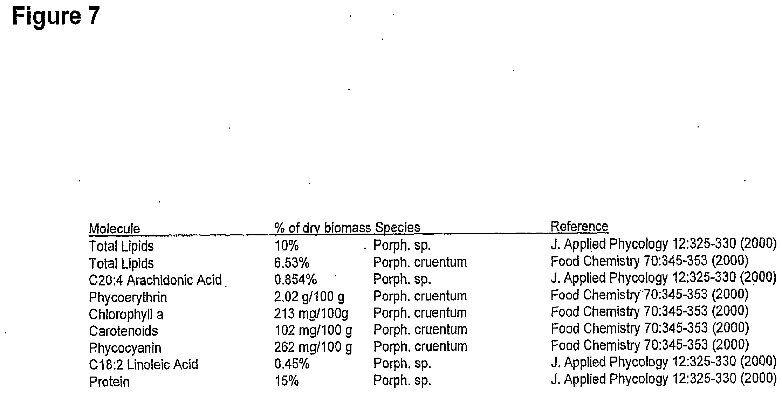

Homogenization as described herein can increase the amount of solvent-available polysaccharide significantly. For example, homogenization can increase the amount of solvent-available polysaccharide by at least a factor of 0.25, at least a factor of 0.5, at least a factor of 1, at least a factor of 2, at least a factor of 3, at least a factor of 4, at least a factor of 5, at least a factor of 8, at least a factor of 10, at least a factor of 15, at least a factor of 20, at least a factor of 25, and at least a factor of 30 or more compared to the amount of solvent-available polysaccharide in an identical or similar quantity of non-homogenized cells of the same type. One way of determining a quantity of cells sufficient to generate a given quantity of homogenate is to measure the amount of a compound in the homogenate and calculate the gram quantity of cells required to generate this amount of the compound using known data for the amount of the compound per gram mass of cells. The quantity of many such compounds per gram of particular microalgae cells are know. For examples, see Figure 7. Given a certain quantity of a compound in a composition, the skilled artisan can determine the number of grams of intact cells necessary to generate the observed amount of the compound. The number of grams of microalgae cells present in the composition can then be used to determine if the composition contains at least a certain amount of solvent-available polysaccharide sufficient to indicate whether or not the composition contains homogenized cells, such as for example five times the amount of solvent-available polysaccharide present in a similar or identical quantity of unhomogenized cells.

### H. Analysis Methods

Assays for detecting polysaccharides can be used to quantitate starting polysaccharide concentration, measure yield during purification, calculate density of secreted polysaccharide in a photobioreactor, measure polysaccharide concentration in a finished product, and other purposes.

The phenol: sulfuric acid assay detects carbohydrates (see Hellebust, Handbook of Phycological Methods, Cambridge University Press, 1978; and Cuesta G., et al., J Microbiol Methods. 2003 Jan;52(1):69-73). The 1,6 dimethylmethylene blue assay detects anionic polysaccharides. (see for example Braz J Med Biol Res. 1999 May;32(5):545-50; Clin Chem. 1986 Nov;32(11):2073-6).

Polysaccharides can also be analyzed through methods such as HPLC, size exclusion chromatography, and anion exchange chromatography (see for example Prosky L, Asp N, Schweizer TF, DeVries JW & Furda I (1988) Determination of insoluble, soluble and total dietary fiber in food and food products: Interlaboratory study. Journal of the Association of Official Analytical Chemists 71, 1017±1023; Int J Biol Macromol. 2003 Nov;33(1-3):9-18)

Polysaccharides can also be detected using gel electrophoresis (see for example Anal Biochem. 2003 Oct 15;321(2):174-82; Anal Biochem. 2002 Jan 1;300(1):53-68).

Monosaccharide analysis of polysaccharides can be performed by combined gas chromatography/mass spectrometry (GC/MS) of the per-O-trimethylsilyl (TMS) derivatives of the monosaccharide methyl glycosides produced from the sample by acidic methanolysis (see Merkle and Poppe (1994) Methods Enzymol. 230: 1-15; York, et al. (1985) Methods Enzymol. 118:3-40).

The determination of protein concentration may be by use of any known procedure, such as the Lowry assay, the Biuret assay, the Bradford assay, or the bicinchoninic acid (BCA) assay. As a non-limiting example, the BCA assay is based on the formation of a Cu²⁺-protein complex under alkaline conditions. The Cu²⁺ is then reduced to Cu¹⁺ where the amount of protein present is proportional to the amount reduction. The reduction has been shown to be mediated by amino acids such as cysteine, cystine, tryptophan, and tyrosine as well as the peptide bond. The result of the assay is a purple-blue complex with Cu²⁺ under alkaline conditions. The color complex is stable, even in the presence of other components possibly present with the proteins, such as detergents. The amount of reduction can be monitored by absorbance at 562 nm. The BCA assay is sensitive and accurate over a broad range of protein concentrations.

### 111 Compositions

### A. General

Compositions of the disclosure include a microalgal polysaccharide or homogenate as described herein. In embodiments relating to polysaccharides, including exopolysaccharides, the composition may comprise a homogenous or a heterogeneous population of polysaccharide molecules, including sulfated polysaccharides. Non-limiting examples of homogenous populations include those containing a single type of polysaccharide molecule, such as that with the same structure and molecular weight. Non-limiting examples of heterogeneous populations include those containing more than one type
of polysaccharide molecule, such as a mixture of polysaccharides having a molecular weight (MW) within a range or a MW above or below a MW value. For example, the *Porphyridium sp.* exopolysaccharide is typically produced in a range of sizes from 3-5 million Daltons. Of course a polysaccharide containing composition of the invention may be optionally protease treated, or reduced in the amount of protein, as described above.

In some embodiments, a composition of the disclosure may comprise one or more polysaccharides produced by microalgae that have not been recombinantly modified. The microalgae may be those which are naturally occurring or those which have been maintained in culture in the absence of alteration by recombinant DNA techniques or genetic
engineering.

In other embodiments, the polysaccharides are those from modified microalgae, such as, but not limited to, microalgae modified by recombinant techniques. Non-limiting examples of such techniques include introduction and/or expression of an exogenous nucleic acid sequence encoding a gene product; genetic manipulation to decrease or inhibit expression of an endogenous microalgal gene product; and/or genetic manipulation to
increase expression of an endogenous microalgal gene product. Recombinant modification of the various microalgae species described herein is contemplated. In some embodiments, the microalgae is from the genus *Porphyridium.*

Polysaccharides provided by the disclosure that are produced by genetically modified microalgae or microalgae that are provided with an exogenous carbon source can be distinct from those produced by microalgae cultured in minimal growth media under photoautotrophic conditions (ie: in the absence of a fixed carbon source) at least in that they
contain a different monosaccharide content relative to polysaccharides from unmodified microalgae or microalgae cultured in minimal growth media under photoautotrophic conditions. Non-limiting examples include polysaccharides having an increased amount of arabinose (Ara), rhamnose (Rha), fucose (Fuc), xylose (Xyl), glucuronic acid (GlcA), galacturonic acid (GalA), mannose (Man), galactose (Gal), glucose (Glc), N-acetyl
galactosamine (GalNAc), N-acetyl glucosamine (GlcNAc), and/or N-acetyl neuraminic acid (NANA), per unit mass (or per mole) of polysaccharide, relative to polysaccharides from either non-genetically modified microalgae or microalgae cultured photoautotrophically. An increased amount of a monosaccharide may also be expressed in terms of an increase relative to other monosaccharides rather than relative to the unit mass, or mole, of polysaccharide.
An example of genetic modification leading to production of modified polysaccharides is transforming a microalgae with a carbohydrate transporter gene, and culturing a transformant in the presence of a monosaccharide which is transported into the cell from the culture media by the carbohydrate transporter protein encoded by the carbohydrate transporter gene. In some instances the culture can be in the dark, where the monosaccharide, such as glucose, is
used as the sole energy source for the cell. In other instances the culture is in the light, where the cells undergo photosynthesis and therefore produce monosaccharides such as glucose in the chloroplast and transport the monosaccharides into the cytoplasm, while additional exogenously provided monosaccharides are transported into the cell by the carbohydrate transporter protein. In both instances monosaccharides from the cytoplasm are transported
into the endoplasmic reticulum, where polysaccharide synthesis occurs. Novel
polysaccharides produced by non-genetically engineered microalgae can therefore be generated by nutritional manipulation, ie: exogenously providing carbohydrates in the culture media that are taken up through endogenous transport mechanisms. Uptake of the exogenously provided carbohydrates can be induced, for example, by culturing the cells in
the dark, thereby forcing the cells to utilize the exogenously provided carbon source. For example, Porphyridium cells cultured in the presence of 7% glycerol in the dark produce a novel polysaccharide because the intracellular carbon flux under these nutritionally manipulated conditions is different from that under photosynthetic conditions.. Insertion of carbohydrate transporter genes into microalgae facilitates, but is not strictly necessary for,
polysaccharide structure manipulation because expression of such genes can significantly increase the concentration of a particular monosaccharide in the cytoplasm of the cell. Many carbohydrate transporter genes encode proteins that transport more than one monosaccharide, albeit with different affinities for different monosaccharides (see for example Biochimica et Biophysica Acta 1465 (2000) 263-274). In some instances a microalgae species can be
transformed with a carbohydrate transporter gene and placed under different nutritional conditions, wherein one set of conditions includes the presence of exogenously provided galactose, and the other set of conditions includes the presence of exogenously provided xylose, and the transgenic species produces structurally distinct polysaccharides under the
two conditions. By altering the identity and concentration of monosaccharides in the
cytoplasm of the microalgae, through genetic and/or nutritional manipulation, the methods provide novel polysaccharides. Nutritional manipulation can also be performed, for example, by culturing the microalgae in the presence of high amounts of sulfate, as described herein. In some instances nutritional manipulation includes addition of one or more
exogenously provided carbon sources as well as one or more other non-carbohydrate culture component, such as 50mM MgSO₄.

In some embodiments, the increase in one or more of the above listed monosaccharides in a polysaccharide may be from below to above detectable levels and/or by at least about 5%, to at least about 2000%, relative to a polysaccharide produced from the same microalgae in the absence of genetic or nutritional manipulation. Therefore an increase
in one or more of the above monosaccharides, or other carbohydrates listed in Tables 2 or 3, by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about
75%, at least about 80%, at least about 85%, at least about 90%, at least about 95%, at least
about 100%, at least about 105%, at least about 110%, at least about 150%, at least about 200%, at least about 250%, at least about 300%, at least about 350%, at least about 400%, at least about 450%, at least about 500%, at least about 550%, at least about 600%, at least about 650%, at least about 700%, at least about 750%, at least about 800%, at least about
850%, at least about 900%, at least about 1000%, at least about 1100%, at least about 1200%,
at least about 1300%, at least about 1400%, at least about 1500%, at least about 1600%, at least about 1700%, at least about 1800%, or at least about 1900%, or more, may be used in the practice of the disclosure. In cases wherein the polysaccharides from unmodified microalgae do not contain one or more of the above monosaccharides, the presence of the monosaccharide in a microalgal polysaccharide indicates the presence of a polysaccharide distinct from that in unmodified microalgae. Thus using particular strains of *Porphyridium sp.* and *Porphyridium cruentum* as non-limiting examples, the disclosure includes modification of these microalgae to
incorporate arabinose and/or fucose, because polysaccharides from two strains of these species do not contain detectable amounts of these monosaccharides (see Example 5 herein). In another non-limiting example, the modification of *Porphyridium sp.* to produce polysaccharides containing a detectable amount of glucuronic acid, galacturonic acid, or N-acetyl galactosamine, or more than a trace amount of N-acetyl glucosamine, is specifically included in the instant disclosure. In a further non-limiting example, the modification of *Porphyridium cruentum* to produce polysaccharides containing a detectable amount of rhamnose, mannose, or N-acetyl neuraminic acid, or more than a trace amount of N-acetyl-glucosamine, is also specifically included in the instant disclosure.
Put more generally, the disclosure includes a method of producing a polysaccharide
comprising culturing a microalgae cell in the presence of at least about 0.01 micromolar of an exogenously provided fixed carbon compound, wherein the compound is incorporated into the polysaccharide produced by the cell. In some embodiments, the compound is selected from Table 2 or 3. The cells may optionally be selected from the species listed in Table I,
and cultured by modification, using the methods disclosed herein, or the culture conditions also listed in Table 1.

The methods may also be considered a method of producing a glycopolymer by culturing a transgenic microalgal cell in the presence of at least one monosaccharide, wherein the monosaccharide is transported by the transporter into the cell and is incorporated into a microalgal polysaccharide.

In some embodiments, the cell is selected from Table 1, such as where the cell is of the genus *Porphyridium,* as a non-limiting example. In some cases, the cell is selected from *Porphyridium sp.* and *Porphyridium cruentum.* Embodiments include those wherein the polysaccharide is enriched for the at least one monosaccharide compared to an endogenous polysaccharide produced by a non-transgenic cell of the same species. The monosaccharide may be selected from Arabinose, Fructose, Galactose, Glucose, Mannose, Xylose, Glucuronic acid, Glucosamine, Galactosamine, Rhamnose and N-acetyl glucosamine.

These methods of the disclosure are facilitated by use of non-transgenic cell expressing a sugar transporter, optionally wherein the transporter has a lower Kₘ for glucose than at least one monosaccharide selected from the group consisting of galactose, xylose, glucuronic acid, mannose, and rhamnose. In other embodiments, the transporter has a lower Kₘ for galactose than at least one monosaccharide selected from the group consisting of glucose, xylose, glucuronic acid, mannose, and rhamnose. In additional embodiments, the transporter has a lower Kₘ for xylose than at least one monosaccharide selected from the group consisting of glucose, galactose, glucuronic acid, mannose, and rhamnose. In further embodiments, the transporter has a lower Kₘ for glucuronic acid than at least one monosaccharide selected from the group consisting of glucose, galactose, xylose, mannose, and rhamnose. In yet additional embodiments, the transporter has a lower Kₘ for mannose
than at least one monosaccharide selected from the group consisting of glucose, galactose, xylose, glucuronic acid, and rhamnose. In yet further embodiments, the transporter has a lower Kₘ for rhamnose than at least one monosaccharide selected from the group consisting of glucose, galactose, xylose, glucuronic acid, and mannose. Manipulation of the concentration and identity of monosaccharides provided in the culture media, combined with
use of transporters that have a different Kₘ for different monosaccharides, provides novel polysaccharides. These general methods can also be used in cells other than microalgae, for example, bacteria that produce polysaccharides.

In alternative embodiments, the cell is cultured in the presence of at least two monosaccharides, both of which are transporter by the transporter. In some cases, the two monosaccharides are any two selected from glucose, galactose, xylose, glucuronic acid, rhamnose and mannose.

In one non-limiting example, the method comprises providing a transgenic cell containing a recombinant gene encoding a monosaccharide transporter; and culturing the cell in the presence of at least one monosaccharide, wherein the monosaccharide is transported by the transporter into the cell and is incorporated into a polysaccharide of the cell. It is pointed out that transportation of a monosaccharide from the media into a microalgal cell allows for the monosaccharide to be used as an energy source, as disclosed below, and for the monosaccharide to be transported into the endoplasmic reticulum (ER) by cellular transporters. In the ER, polysaccharide production and glycosylation, occurs such that in the
presence of exogenously provided monosaccharides, the sugar content of the microalgal polysaccharides change.

In some aspects, the disclosure includes a novel microalgal polysaccharide, such as from microalgae of the genus *Porphyridium,* comprising detectable amounts of xylose, glucose, and galactose wherein the molar amount of one or more of these three monosaccharides in the polysaccharide is not present in a polysaccharide of *Porphyridium* that is not genetically or nutritionally modified. An example of a non-nutritionally and non-genetically modified *Porphyridium* polysaccharide can be found, for example, in Jones R., Journal of Cellular Comparative Physiology 60; 61-64 (1962). In some embodiments, the amount of glucose, in the polysaccharide, is at least about 65% of the molar amount of
galactose in the same polysaccharide. In other embodiments, glucose is at least about 70%, at least about 75%, at least about 80%, at least about 85%, at least about 90%, at least about 95%, at least about 100%, at least about 105%, at least about 110%, at least about 120%, at least about 130%, at least about 140%, at least about 150%, at least about 200%, at least
about 250%, at least about 300%, at least about 350%, at least about 400%, at least about
450%, at least about 500%, or more, of the molar amount of galactose in the polysaccharide. Further embodiments disclosed herein include those wherein the amount of glucose in a microalgal polysaccharide is equal to, or approximately equal to, the amount of galactose (such that the amount of glucose is about 100% of the amount of galactose). Moreover, the disclosure includes microalgal polysaccharides wherein the amount of glucose is more than the amount of galactose.

Alternatively, the amount of glucose, in the polysaccharide, is less than about 65% of the molar amount of galactose in the same polysaccharide. The disclosure thus provides for polysaccharides wherein the amount of glucose is less than about 60%, less than about 55%, less than about 50%, less than about 45%, less than about 40%, less than about 35%, less than about 30%, less than about 25%, less than about 20%, less than about 15%, less than about 10%, or less than about 5% of the molar amount of galactose in the polysaccharide.

In other aspects, the invention includes a microalgal polysaccharide, such as from microalgae of the genus *Porphyridium,* comprising detectable amounts of xylose, glucose, galactose, mannose, and rhamnose, wherein the molar amount of one or more of these five monosaccharides in the polysaccharide is not present in a polysaccharide of non-genetically modified and/or non-nutritionally modified microalgae. In some embodiments, the amount of rhamnose in the polysaccharide is at least about 100% of the molar amount of mannose in the same polysaccharide. In other embodiments, rhamnose is at least about 110%, at least about 120%, at least about 130%, at least about 140%, at least about 150%, at least about
200%, at least about 250%, at least about 300%, at least about 350%, at least about 400%, at least about 450%, or at least about 500%, or more, of the molar amount of mannose in the polysaccharide. Further embodiments of the present diclosure include those wherein the amount of rhamnose in a microalgal polysaccharide is more than the amount of mannose on a molar basis.

Alternatively, the amount of rhamnose, in the polysaccharide, is less than about 75% of the molar amount of mannose in the same polysaccharide. The disclosure thus provides for polysaccharides wherein the amount of rhamnose is less than about 70%, less than about 65%, less than about 60%, less than about 55%, less than about 50%, less than about 45%, less than about 40%, less than about 35%, less than about 30%, less than about 25%, less than about 20%, less than about 15%, less than about 10%, or less than about 5% of the molar amount of mannose in the polysaccharide.
In additional aspects, the disclosure includes a microalgal polysaccharide, such as
from microalgae of the genus *Porphyridium,* comprising detectable amounts of xylose, glucose, galactose, mannose, and rhamnose, wherein the amount of mannose, in the polysaccharide, is at least about 130% of the molar amount of rhamnose in the same polysaccharide. In other embodiments, mannose is at least about 140%, at least about 150%,
at least about 200%, at least about 250%, at least about 300%, at least about 350%, at least about 400%, at least about 450°4, or at least about 500%, or more, of the molar amount of rhamnose in the polysaccharide.

Alternatively, the amount of mannose, in the polysaccharide, is equal to or less than the molar amount of rhamnose in the same polysaccharide. The disclosure thus provides for polysaccharides wherein the amount of mannose is less than about 95%, less than about 90%, less than about 85%, less than about 80%, less than about 75%, less than about 70%, less than about 65%, less than about 60%, less than about 60%, less than about 55%, less than about 50%, less than about 45%, less than about 40%, less than about 35°4, less than about 30%, less than about 25%, less than about 20%, less than about 15%, less than about 10%, or less than about 5% of the molar amount of rhamnose in the polysaccharide.

In further aspects, the disclosure includes a microalgal polysaccharide, such as from microalgae of the genus *Porphyridium,* comprising detectable amounts of xylose, glucose, and galactose, wherein the amount of galactose in the polysaccharide, is at least about 100% of the molar amount of xylose in the same polysaccharide. In other embodiments, rhamnose
is at least about 105%, at least about 110%, at least about 120%, at least about 130%, at least
about 140%, at least about 150%, at least about 200%, at least about 250%, at least about 300%, at least about 350%, at least about 400%, at least about 450%, or at least about 500%, or more, of the molar amount of mannose in the polysaccharide. Further embodiments disclosed herein
include those wherein the amount of galactose in a microalgal polysaccharide is more than the amount of xylose on a molar basis.

Alternatively, the amount of galactose, in the polysaccharide, is less than about 55% of the molar amount of xylose in the same polysaccharide. The disclosure thus provides for polysaccharides wherein the amount of galactose is less than about 50%, less than about 45%, less than about 40%, less than about 35%, less than about 30%, less than about 25%, less than about 20%, less than about 15%, less than about 10%, or less than about 5% of the molar amount of xylose in the polysaccharide.

In yet additional aspects, the disclosure includes a microalgal polysaccharide, such as from microalgae of the genus *Porphyridium,* comprising detectable amounts of xylose, glucose, glucuronic acid and galactose, wherein the molar amount of one or more of these five monosaccharides in the polysaccharide is not present in a polysaccharide of unmodified microalgae. In some embodiments, the amount of glucuronic acid, in the polysaccharide, is at least about 50% of the molar amount of glucose in the same polysaccharide. In other
embodiments, glucuronic acid is at least about 55%, at least about 60%, at least about 65%, at least about 70%, at least about 75%, at least about 80%, at least about 85%, at least about 90%, at least about 95%, at least about 100%, at least about 105%, at least about 110%, at least about 120%, at least about 130%, at least about 140%, at least about 150%, at least about 200%, at least about 250%, at least about 300%, at least about 350%, at least about
400%, at least about 450%, or at least about 500%, or more, of the molar amount of glucose in the polysaccharide. Further embodiments disclosed herein include those wherein the amount of glucuronic acid in a microalgal polysaccharide is more than the amount of glucose on a molar basis.

In other embodiments, the exopolysaccharide, or cell homogenate polysaccharide, comprises glucose and galactose wherein the molar amount of glucose in the exopolysaccharide, or cell homogenate polysaccharide, is at least about 55% of the molar amount of galactose in the exopolysaccharide or polysaccharide. Alternatively, the molar amount of glucose in the exopolysaccharide, or cell homogenate polysaccharide, is at least about 60%, at least about 65%, at least about 70%, at least about 75%, at least about 80%, at
least about 85%, at least about 90%, or at least about 100% of the molar amount of galactose in the exopolysaccharide or polysaccharide.

In yet further aspects, the disclosure includes a microalgal polysaccharide, such as from microalgae of the genus *Porphyridium,* comprising detectable amounts of xylose, glucose, glucuronic acid, galactose, at least one monosaccharide selected from arabinose,
fucose, N-acetyl galactosamine, and N-acetyl neuraminic acid, or any combination of two or more of these four monosaccharides.

In one embodiment, a method of determining the amount of phycoerythrin per dry gram of cells in a formulated skin care composition is to quantify the amount of certain molecules known to be present at certain levels or ranges of levels in *Porphyridium* cells is disclosed.
Such measurement is an indication of how many grams of dry Porphyridium biomass per gram of formulated skin care product are in a given formulation. For example, compounds listed in Figure 7 are known to be present in *Porphyridium* cells at certain ranges of levels. The amounts of compounds, such as those listed in Figure 7, were determined by analysis of *Porphyridium* cell biomass grown in nitrogen-replete media under outdoor daylight
conditions, ie: deep red cells containing quantities of phycoerythrin typically seen in cells grown in artificial seawater media such as those described in Example 1. Given a certain quantity of a compound in a formulated skin care composition, the skilled artisan can determine the number of dry grams of Porphyridium biomass are present per gram of
formulated skin care product. The number of dry grams of *Porphyridium* cells present in the composition can then be used to determine if the cells contain less than or more than a certain amount of phycoerythrin per dry gram of cells.

### IV Cosmeceutical Compositions and Topical Application

### A. General

Compositions, comprising polysaccharides, whole cell extracts, or mixtures of polysaccharides and whole cell extracts, are provided for topical application or non-systemic administration. The polysaccharide may be any one or more of the microalgal polysaccharides disclosed herein, including those produced by a species, or a combination of
two or more species, in Table 1. Similarly, a whole cell extract may be that prepared from a microalgal species, or a combination of two or more species, in Table 1. In some embodiments, polysaccharides, such as exopolysaccharides, and cell extracts from microalgae of the genus *Porphyridium* are used in the practice of the invention. A composition of the invention may comprise from between about 0.001% and about 100%,
about 0.01% and about 90%, about 0.1% and about 80%, about 1% and about 70%, about 2% and about 60%, about 4% and about 50%, about 6% and about 40%, about 7% and about 30%, about 8% and about 20%, or about 10% polysaccharide, cell extract, by weight.

In other embodiments, the composition comprises a carrier suitable for topical administration and/or a preservative suitable for topical administration; and Rhodophyte cells, optionally of the genus *Porphyridium.* The cells may contain reduced amounts of the red pigmentation by preparation methods described herein. In some cases, an aqueous extract of a composition comprising the *Porphyridium* cells contains no more than 75% to no more than about 1% of the absorbance per gram at 545nm of a second composition formulated in identical fashion except containing cells of the same species of *Porphyridium* cells that were grown in a photobioreactor in ATCC 1495 ASW media in the presence of 50 microeinsteins of light per square meter per second. In further embodiments, the carrier is suitable for topical administration to humans, such as to human skin or a skin tissue.

In alternative embodiments, a composition for application to human skin may comprise a polysaccharide isolated from cells of the genus *Porphyridium.* Such a composition may further comprise a carrier and/or preservative suitable for topical administration as described herein. In some cases, the polysaccharide of the composition contains no more than about 10% protein by weight. In other embodiments, the polysaccharide contains no more than about 5%, no more than about 2%, or no more than about 1% protein by weight. Of course the polysaccharide may also be essentially, or completely, free of protein or protein as detectable by assay methods as described herein after treatment to remove protein.

In further embodiments, the polysaccharide may comprise a molar amount of glucose that is at least about 50%, or at least about 60%, of the molar amount of galactose. Alternatively, the molar amount of glucose in the polysaccharide is greater than the molar amount of galactose. In additional embodiments, the polysaccharide contains less than a 0.1%, or less than a 0.01%, molar amount of at least one monosaccharide selected from the group consisting of arabinose, rhamnose, fucose, and N-acetyl glucosamine. Optionally, the polysaccharide contains less than a 0.1%, or less than a 0.01%, molar amount of each of arabinose, rhamnose, fucose, and N-acetyl glucosamine.

In yet additional embodiments, a composition may comprise a polysaccharide comprising a molar amount of glucose that is at least about 30%, or at least about 40%, of the molar amount of xylose. In some cases, the polysaccharide comprises a molar amount of glucose between about 15.8 and about 25.8%; and a molar amount of xylose that is between about 37.5 and about 45.5%. Alternatively, the polysaccharide comprises a molar amount of glucose between about 17.8 and about 23.8%; and a molar amount of xylose that is between about 39.5 and about 43.5%.

In yet further embodiments, the polysaccharides are sulfated exopolysaccharides containing at least 3.0% about sulfur, at least about 3.5% sulfur, at least about 4.0% sulfur, at least about 4.5% sulfur, at least about 4.6% sulfur, at least about 4.75% sulfur, at least about 5.0% sulfur, at least about 5.25% sulfur, at least about 5.5% sulfur, at least about 5.75% sulfur, at least about 6.0% sulfur, at least about 6.25% sulfur, at least about 6.5% sulfur, at least about 6.75% sulfur, or at least about 7.0% sulfur by weight of the polysaccharide. The amount or level of sulfation in the polysaccharides may be analyzed and compared to the amount of sulfates used to culture the microalgae. Thus the amount or level of sulfation in the polysaccharides of cells grown at about 100 mM, about 200 mM, about 300 mM, about 400 mM, about 500 mM, about 600 mM, or about 700 mM or higher, sulfate (SO₄²") may be determined by routine and repetitive methods disclosed herein. The amount or level of sulfur by weight in the polysaccharides of a sample of cells or cell material may be determined without knowledge of the amount of sulfate used to culture the cells.

As a further alternative, a composition for topical application to human skin may comprise microalgal cells. The cells may be those of genus *Porphyridium* or any other species or strain as disclosed herein. Optionally, the composition further comprising a carrier
and/or preservative suitable for topical administration as described herein. In alternative embodiments, the cells are homogenized (such as by methods described herein) to generate or form a microalgal cell homogenate. In some cases, the cells or homogenate thereof, and therefore the composition, is essentially free of red and/or green coloration. Optionally, the cells or homogenate thereof, and therefore the composition, is completely free of red
coloration. Thus in some embodiments, the cells (and therefore the homogenate thereof)
contains less than about 15, less than about 10, less than about 5, less than about 2, less than about 1, less than about 0.5, or less than about 0.1 milligrams of phycoerythrin per dry gram of cells.

Alternatively, the cells (and therefore a homogenate thereof) contains a sulfated polysaccharide having an amount of sulfur by weight of at least 3.0%, at least 3.5%, at least 4.0%, at least 4.5%, at least 4.6%, at least 4.75%, at least 5.0%, at least 5.25%, at least 5.5%, at least 5.75%, at least 6.0%, at least 6.25%, at least 6.5%, at least 6.75%, or at least 7.0% sulfur by weight of the polysaccharide as described herein. In additional embodiments, the microalgal cell homogenate contains at least two, at least three, at least five, at least ten, or at
least twenty times the amount of solvent-available polysaccharide present in a quantity of unhomogenized cells needed to generate the microalgal cell homogenate.

In further embodiments, the disclosed includes a composition comprising particulate polysaccharides, such as microbeads or nanobeads comprising a disclosed polysaccharide. In some embodiments the polysaccharide particles are referred to as Marine NanobeadsTM. The composition may be for improving the appearance of skin, such as human skin. The polysaccharides may have any level of sulfation described herein. The composition may be sterile and/or non-pyrogenic and optionally substantially free of endotoxins and/or proteins. In other embodiments, the composition further comprises hyaluronic acid or another agent suitable or desirable for treatment of skin. Non-limiting examples of such an agent include aloe vera, urea, alpha hydroxyl acid, vitamin E, glycyrrhizinic acid, methylsulfonylmethane (MSM), and collagen.

In some embodiments, the composition comprises an algal polysaccharide, wherein the polysaccharide: (a) has been made completely or partially insoluble in water through drying; and (b) has been homogenized or otherwise milled or disrupted to generate particles of the invention as defined in the claims.

The polysaccharide may of course be that of a variety of microalgal cells, such as those described in Table 1 and those of the genus *Porphyridium.* In some cases, the polysaccharide is contained in a non-aqueous material. As non-limiting examples, the material may be contained in an oil suitable for topical administration, with hexadecanoic
acid or oil that is contained in an emulsion as representative examples. The composition may also comprise a carrier and/or preservative suitable for topical administration. Of course the composition may also be substantially free of endotoxins and/or protein as well as sterile and/or non-pyrogenic. In further embodiments, the polysaccharide is encapsulated by a timed-release coating, such as one suitable for topical application to human skin.
The composition is prepared by a manufacturing or preparation method as defined in the claims. So in some cases, the composition comprises a polysaccharide that is partially or completely insoluble in water, by heating an aqueous suspension of the polysaccharide as defined in the claims thereby removing water from the suspension. The polysaccharide particulates
may be partially soluble such that they are less than about 70%, less than about 60%, less
than about 50%, less than about 40%, less than about 30%, less than about 20%, less than about 10%, less than about 5%, or less than about 2% soluble in water.

In other embodiments of the present disclosure, the polysaccharide has been made partially or completely insoluble by a method selected from the group consisting of chemical cross-linking, chemical dehydration through displacement of bound water by an alcohol, precipitation from solution using an alcohol or a ketone or pH, and coating of particles by microencapsulation. Non-limiting examples of these methods are known to the skilled person and may be used in the practice of the invention. For examples, see Biomacromolecules. 2005 Nov-Dec;6(6):3202-8 ; Arterioscler Thromb Vase Biol. 2004 Mar;24(3):613-7; J Biomed Mater Res. 2001 Sep 15;56(4):478-86; Dalton Trans. 2004 Sep 7;(17):2621-34. Epub 2004 Jul 28; Biomacromolecules. 2004 Jan-Feb;5(1):126-36; Contraception. 2002 Aug;66(2):137-40; Biomacromolecules. 2006 May;7(5):1471-80; Biopolymers. 1999 Sep;50(3):227-37; Biomaterials. 2003 May;24(12):2083-96; Int J Pharm. 2003 Nov 28;267(1-2):13-25; Med Biol Eng Comput. 1998 Jan;36(1):129-34 and Reprod Fertil Dev. 2002;14(5-6):307-14. A representative example is chemical cross-linking to a pharmaceutically or cosmetically acceptable insoluble solid phase material, such as a polymer, microbead, or nanobead. The insoluble material need not precipitate when in a solution but includes a material that remains in suspension when in solution. Dehydration or precipitation with alcohol may be practiced with any alcohol suitable for pharmaceutical or cosmetic use. Non-limiting examples include ethanol or a fatty alcohol such as cetyl, stearyl, cetearyl, or lanolin alcohol. A non-limiting method of microencapsulating a cosmetic is described in U.S. Patent 4,752,496.

The use of a disclosed method of the invention also includes milling of dried polysaccharide material (such as a film) into particles by any suitable method. Non-limiting examples of such methods are disclosed herein, and they produce particles with an average size that may range between about 400 and about 0.1 microns.

In some embodiments, the composition comprises polysaccharide particles that increase in volume on contact with water compared to their anhydrous or partially hydrated volume. In some embodiments, the particles increase in volume by an amount selected from at least about 5%, at least about 25%, at least about 50%, at least about 100%, at least about 200%, at least about 300%, by at least about 500%, at least about 1000%, or at least about 5000%.

In some embodiments, the polysaccharide of the method is associated with a fusion protein as described herein. In some cases, the fusion protein comprises a first protein with at least 60% amino acid identity with the protein of SEQ ID NO: 15, and a second protein. Alternatively, the polysaccharide of the method contains an amount of sulfur by weight from at least about 3.0% sulfur to at least about 7.0% sulfur by weight as described herein, and optionally is also associated with a fusion protein. An example of an expression vector for expression in Porphyridium of a polysaccharide binding protein:superoxide dismutase fusion can be found in SEQ ID NO: 36. In some embodiments a spacer of 1-15 amino acids is placed between the glycoprotein and second protein to enable flexibility between the two proteins. Expression of a fusion protein, wherein the second heterologous protein is a dimerizing or multimerizing protein (such as superoxide dismutase) can be advantageous when a higher viscosity or gelling property of the polysaccharide is desired because the dimers serve to crosslink the polysaccharide. The reversibility of the crosslinking is in part dictated by the strength of the dimerization in such fusion proteins provided by the disclosure. Topical compositions are usually formulated with a carrier, such as in an ointment or a cream, and may optionally include a fragrance. One non-limiting class of topical compositions is that of cosmeceuticals. Other non-limiting examples of topical formulations
include gels, solutions, impregnated bandages, liposomes, or biodegradable microcapsules as well as lotions, sprays, aerosols, suspensions, dusting powder, impregnated bandages and dressings, biodegradable polymers, and artificial skin. Another non-limiting example of a topical formulation is that of an ophthalmic preparation. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, the composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and
water.

In some embodiments, the polysaccharides contain fucose moieties. In other embodiments, the polysaccharides are sulfated, such as exopolysaccharides from microalgae of the genus *Porphyridium.* In some embodiments, the polysaccharides will be those from a *Porphyridium* species, such as one that has been subject to genetic and/or nutritional manipulation to produce polysaccharides with altered monosaccharide content and/or altered sulfation.

In additional embodiments, a composition of the disclosure comprises a microalgal cell homogenate and a topical carrier. In some embodiments, the homogenate may be that of a species listed in Table 1 or may be material produced by a species in the table.

In further embodiments of the disclosure, a composition comprising purified microalgal polysaccharide and a carrier suitable for topical administration also contains a fusion (or chimeric) protein associated with the polysaccharide. In some embodiments, the fusion protein comprises a first protein, or polypeptide region, with at least about 60% amino acid identity with the protein of SEQ ID NO: 15. In other embodiments, the first protein has at least about 70%, at least about 75%, at least about 80%, at least about 85%, at least about 90%, at least about 95%, or at least about 98%, or higher, amino acid identity with the sequence of SEQ ID NO:15. Preferably the fusion protein binds to a sulfated exopolysaccharide from a cell of the genus Porphyridium. It is preferable that the binding of the fusion protein to the polysaccharide be selective and high affinity, though such is not required to practice the invention.

The fusion protein may comprise a second protein, or polypeptide region, with a homogenous or heterologous sequence. Non-limiting examples of the second protein include an antibody, an enzyme, or a structural protein of skin or a skin tissue, such as that of a human being. In optional embodiments, the enzyme is superoxide dismutase, such as that has at least about 60% amino acid identity with the sequence of SEQ ID NO: 12, SEQ ID NO: 13, or a protein from Table 21 and exhibit superoxide dismutase activity as non-limiting examples. In some embodiments, the superoxide dismutase has at least about 70%, at least
about 75%, at least about 80%, at least about 85%, at least about 90%, at least about 95%, or at least about 98%, or higher, amino acid identity with the sequence of SEQ ID NO:12 or 13. In other embodiments, the second protein is a structural skin protein selected from the group consisting of elastin and a collagen chain, such as that of human skin. Sequences encoding elastin and a chain of collagen are known to the skilled person and may be incorporated into a
fusion protein via routine methods. Examples of such human skin proteins are also disclosed herein in the sequence listing. Assays for superoxide dismutase activity are well known in the art. For examples see Song et al., Clin Sci (Lond). 2007 Jan 8; Epub ahead of print, Oxidative stress, antioxidant status and DNA damage in patients with impaired glucose regulation and newly-diagnosed Type II diabetes; and Liu et al., Phytomedicine. 2006 Dec 15
Protection of PC 12 cells from hydrogen peroxide-induced cytotoxicity by salvianolic acid B,
a new compound isolated from Radix Salviae miltiorrhizae. The presence of any exogenous or endogenous protein expressed in microalgae can be assayed for example, using well known methods such as western blotting and ELISA assays.

In other embodiments, the second protein is an antibody. Non-limiting examples of antibodies for use include an antibody that selectively binds to
an antigen from a pathogen selected from HIV, Herpes Simplex Virus, gonorrhea, Chlamydia, Human Papillomavirus, and Trichomoniasis. In some embodiments, the antibody is a humanized antibody.

### B. Methods of Formulation

Polysaccharide compositions for topical application can be formulated by first preparing a purified preparation of polysaccharide. As a non-limiting example, the polysaccharide from aqueous growth media is precipitated with an alcohol, resuspended in a dilute buffer, and mixed with a carrier suitable for application to human skin or mucosal tissue, including the vaginal canal. Alternatively, the polysaccharide can be purified from growth media and concentrated by tangential flow filtration or other filtration methods, and formulated as described above. Intracellular polysaccharides can be also formulated in a similar or identical manner after purification from other cellular components.

As a non-limiting example, the invention includes a method of formulating a cosmeceutical composition, said method comprising culturing microalgal cells in suspension under conditions to allow cell division; separating the microalgal cells from culture media, wherein the culture media contains exopolysaccharide molecules produced by the microalgal cells; separating the exopolysaccharide molecules from other molecules present in the culture media; homogenizing the microalgal cells; and adding the separated exopolysaccharide molecules to the cells before, during, or after homogenization. In some embodiments, the microalgal cells are from the genus *Porphyridium.*

In other embodiments, the invention includes a method of manufacturing a composition comprising particles, the method comprising isolating a polysaccharide from microalgae; drying an aqueous suspension of the polysaccharide to a solid film wherein at least some proportion of the film has been made completely or partially insoluble in water; homogenizing or otherwise milling or disrupting the film into particles; and formulating the particles into a non-aqueous material.

The method may of course be practiced with a variety of microalgal cells, such as those described in Table 1 and those of the genus *Porphyridium.*

As described herein, the resulting composition may be for improving the appearance of skin, such as human skin. In some embodiments, the formulating may be into the oil phase of an oil-in-water emulsion. In other embodiments, the non-aqueous material is an oil suitable for topical administration, with hexadecanoic acid and oil that is contained in an emulsion as non-limiting examples. In further embodiments, the method further comprises formulating the particles into a carrier and/or preservative suitable for topical administration. The resulting composition may also be substantially free of endotoxins and/or protein. In many embodiments, the composition is also made sterile and/or non-pyrogenic. Alternatively, the method further comprises formulating hyaluronic acid into the composition.

In other embodiments, the polysaccharide after the drying step is partially or completely insoluble in water. Optionally, the polysaccharide after the drying step is soluble in water at a percentage selected from the list consisting of less than about 70%, less than about 60%, less than about 50%, less than about 40%, less than about 30%, less than about 20%, less than about 10%, less than about 5%, and less than about 2%.

Embodiments of the drying step include drying performed at between about 40 and about 180 °C, such as between about 80 and about 170, or between about 100 and about 160, between about 125 and about 155, between about 135 and about 152, between about 140 and about 150, or between about 145 and about 148 °C as non-limiting examples. Optionally, the drying is performed in two steps, wherein the first step comprises heating the suspension of the polysaccharide to no more than about 60 °C for a first period of time to produce a solid film followed by heating the solid film for a second period of time to no more than about 160 °C. In alternative embodiments, the first and second steps comprise heating to no more than about 80 and no more than about 150, or to approximately 100 and no more than 148 °C, respectively. In some embodiments, the suspension of the polysaccharide is heated during the first period of time in the presence of air to produce a solid film and the solid film is heated during the second period of time in at least a partial vacuum or otherwise under reduced pressure.

After the drying step, milling may be by any suitable method. Non-limiting examples include a method selected from the list consisting of jet milling, ball milling, Retsch® milling, and milling in a Quadro® device. The resulting particles of the composition may have an average size between about 400 and about 0.1 microns. In some embodiments, the particles of the composition have an average size between about 100 and about 0.1 microns, between about 50 and about 0.1 microns, between about 10 and about 0.1 microns, between about 10 and about 0.5 microns, or between about 5 and about 0.5 microns.

In some embodiments, the polysaccharide of the method is associated with a fusion protein as described herein. In some cases, the fusion protein comprises a first protein with at least 60% amino acid identity with the protein of SEQ ID NO: 15, and a second protein. Alternatively, the polysaccharide of the method contains an amount of sulfur by weight from at least about 3.0% sulfur to at least about 7.0% sulfur by weight as described herein, and in some embodiments is associated with a fusion protein.

Examples of polysaccharides, both secreted and intracellular, that are suitable for formulation with a carrier for topical application are listed in Table 1.

In further embodiments, polysaccharide is associated with a fusion (or chimeric) protein comprising a first protein (or polypeptide region) with at least about 60% amino acid identity with the protein of SEQ ID NO: 15. In some cases, the first protein has at least about 70%, at least about 75%, at least about 80%, at least about 85%, at least about 90%, at least about 95%, or at least about 98%, or higher, amino acid identity with the sequence of SEQ ID NO:15.

The fusion protein may comprise a second protein, or polypeptide region, with a homogenous or heterologous sequence. One non-limiting example of the second protein is a superoxide dismutase enzyme.

Examples of carriers suitable for formulating polysaccharide are described above. Ratios of homogenate:carrier are typically in the range of about 0.001:1 to about 1:1 (volume:volume), although the methods disclosed herein comprise ratios outside of this range, such as, but not limited to, about 0.01:1 and about 0.1:1.
Microalgal cellular extracts can also be formulated for topical administration. It is preferable but not necessary that the cells are physically or chemically disrupted as part of the formulation process. For example, cells can be centrifuged from culture, washed with a buffer such as 1.0 mM phosphate buffered saline, pH 7.4, and sonicated. Preferably the cells are sonicated until the cell walls have been substantially disrupted, as can be determined
under a microscope. For example, *Porphyridium sp.* cells can be sonicated using a Misonix sonicator as described in Example 3.

Cells can also be dried and ground using means such as mortar and pestle, colloid milling, ball milling, or other physical method of breaking cell walls.

After cell disruption, cell homogenate can be formulated with carrier and fragrance as described above for polysaccharides.

The compositions according to the present invention can also be used as hair treating agents such as hair dressings (e.g., hair creams, hair sprays, hair tonics, hair gels, hair lotions, hair oils, hair essences, hair waters, hair waxes, and hair mousses), shampoos, finishing rinses, hair treatments, hair creams, hair mousses, hair setting lotions, hair colors, hair dyes (e.g., hair colors, one-part hair dyes, and two-part hair dyes), perm solutions (e.g., permanent wave solutions, hair straightening solutions, and permanent wave holding agents), blood flow enhancers, scalp lotions, and anti-hair loss agents. Other application of the compositions according to the present invention include, for example, skin care cosmetics such as toners, serums, whitening toners, milky lotions, whitening milky lotions, creams, whitening creams,
ointments, whitening ointments, lotions, whitening lotions, oils, facial packs. Furthermore, still other applications of the compositions according to the present invention includes, for example, makeup cosmetics such as foundations, liquid foundations, lipsticks, lip glosses, eye shadows, powders, face powders, blushers, eye shadows, eye liners, mascaras, and eyebrow pencils. Other applications of the compositions according to the present invention include,
for example, skin cleaners such as soap, cleansing creams, cleansing lotions, cleansing milks, cosmetic compositions, facial washes, and body shampoos. Moreover, another application of the compositions according to the present invention include finishing cosmetics such as manicures. Other applications of the compositions according to the present invention include, for example, cosmetic compositions in the form of bath agents, patches, perfumes, toothpastes, tooth washes, and mouthwashes.

### C. Co-Administered Compositions

Topical compositions can comprise a portion of a complete composition sold as a single unit. Other portions of the complete compositions can comprise an oral supplement intended for administration as part of a regime for altering skin appearance. Because the top layers of the skin contain dead cells, nutrients delivered via capillaries cannot reach the outer layers of cells. The outer layers of cells must be provided with nutrients though topical administration. However, topical administration is not always an effective method of providing nutrients to deep layers of skin that contain living cells. The compositions provided herein comprise both topical compositions that contain algal polysaccharides and/or cellular extracts as well as oral compositions comprising nutraceutical molecules such as purified polysaccharides, whole cell extracts, carotenoids, polyunsaturated fatty acids, and other molecules that are delivered to the skin via capillaries. The combined effect of the topical and oral administration of these molecules and extracts provides a benefit to skin health that is additive or synergistic compared to the use of only a topical or only an orally delivered product.

Examples of the topical components of the composition include exopolysaccharide from *Porphyridium cruentum*, *Porphyridium sp.,* list others. Other components of the topical composition can include polysaccharides and/or cell extracts from species listed in Table I.

Cellular extracts for topical administration can also include cellular homogenates from microalgae that have been genetically engineered. For example, homogenates of *Porphyridium sp.* that have been engineered to express an exogenous gene encoding superoxide dismutase can be formulated for topical administration. Other genes that can be expressed include carotenoid biosynthesis enzymes and polyunsaturated fatty acid biosynthesis enzymes.

Examples of compositions for oral administration include one or more of the following: DHA, EPA, ARA, lineoileic acid, lutein, lycopene, beta carotene, braunixanthin, zeaxanthin, astaxanthin, linoleic acid, alpha carotene, vitamin C and superoxide dismutase. Compositions for oral administration usually include a carrier such as those described above. Oral compositions can be formulated in tablet or capsule form. Oral compositions can also be formulated in an ingestible form such as a food, tea, liquid, etc. Oral compositions can, for example, comprise at least 50 microgram, at least 100 microgram, at least 50 milligrams, at least 100 milligrams, at least 500 milligrams, and at least one gram of a small molecule such as a carotenoids or a polyunsaturated fatty acid.

Disclosed herein are orally administered nutraceutical compositions comprising one or more polysaccharides, or microalgal cell extract or homogenate. A nutraceutical composition serves as a nutritional supplement upon consumption. In one disclosure, a nutraceutical may be bioactive and serve to affect, alter, or regulate a bioactivity of an organism.

A nutraceutical may be in the form of a solid or liquid formulation. In one disclosure, a solid formulation includes a capsule or tablet formulation as described above.

In one disclosure, a solid nutraceutical may simply be a dried microalgal extract or homogenate, as well as dried polysaccharides *per se.* Liquid formulations, includes suspensions, as well as aqueous solutions, of polysaccharides, extracts, or homogenates. In one disclosure the nutraceutical is derived from microalgae, while in other example the nutraceutical is derived from other sources such as, for example, plants, plant extracts, and chemically synthesized molecules. In a preferred embodiment a topical composition and an oral composition contain at least one molecule in common.

The methods disclosed herein include a method of producing a nutraceutical
composition. Such a method may comprise drying a microalgal cell homogenate or cell extract. The homogenate may be produced by disruption of microalgae which has been separated from culture media used to propagate (or culture) the microalgae. Thus in one non-limiting example, a method comprises culturing red microalgae; separating the microalgae from culture media; disrupting the microalgae to produce a homogenate; and drying the homogenate. In similar embodiments, a method may comprise drying one or more polysaccharides produced by the microalgae.

In one disclosures, a method comprises drying by tray drying, spin drying, rotary drying, spin flash drying, or lyophilization. In another disclosure, methods comprise disruption of microalgae by a method selected from pressure disruption, sonication, and ball milling

In an additional disclosure, a method further comprises formulation of the homogenate, extract, or polysaccharides with a carrier suitable for human
consumption. As described herein, the formulation may be that of tableting or encapsulation of the homogenate or extract.

In a further disclosure, the methods comprise the use of microalgal homogenates, extracts, or polysaccharides wherein the cells contain an exogenous nucleic acid sequence, such as in the case of modified cells described herein. The exogenous sequence may encode a gene product capable of being expressed in the cells or be a sequence which increases expression of one or more endogenous microalgal gene product.

In a further disclosure, the topical composition and the oral composition both contain at least one molecule in common. For example, the topical composition contains homogenate of *Porphyridium* cells that contain zeaxanthin, and the oral composition contains zeaxanthin. in another embodiment, the topical composition contains homogenate of *Porphyridium* cells that contain polysaccharide, and the oral composition contains polysaccharide purified from *Porphyridium* culture media.

Some of the compositions described herein are packaged for sale as a single unit. For example, a unit for sale comprises a first container holding a composition for topical administration, a second container holding individual doses of a composition for oral administration, and optionally, directions for co-administration of the topical and oral composition.

Further disclosures include a combination product comprising 1) a first composition comprising a microalgal extract and a carrier suitable for topical application to skin; and 2) a second composition comprising at least one compound and a carrier suitable for human consumption; wherein the first and second compositions are packaged for sale as a single unit. Thus the disclosure includes co-packaging of the two compositions, optionally with a instructions and/or a label indicating the identity of the contents and/or their proper use.

In some disclosures, the first composition may be a topical formulation or non-systemic formulation, optionally a cosmeceutieal, as described herein. Preferably, the first composition comprises a carrier suitable for topical application to skin, such as human skin. Non-limiting examples of the second composition include a food composition or nutraceutical as described herein. Preferably, the second composition comprises at least one carrier suitable for human consumption, such as that present in a food product or composition. Combination products may be packaged separately for subsequent use together by a user or packaged
together to facilitate purchase and use by a consumer. Packaging of the first and second compositions may be for sale as a single unit.

### D. Methods of Cosmetic Enhancement

In a further aspect, the disclosure includes a method to cosmetically enhance skin or its appearance or texture. In some cases, the enhancement is due to increased or improved skin elasticity. The skin may be that of a human being, such as the skin of the face, hands, feet, or other parts of the human body. In other embodiments, the enhancement may be in the appearance or texture of human lips. The method may comprise administration of a
polysaccharide composition suitable for injection into skin or lip tissue to improve the appearance thereof. The composition may be any as described herein suitable for the method of administration or application. In some embodiments, the injection is made to alleviate or eliminate wrinkles. In other embodiments, the treatment reduces the visible signs of aging and/or wrinkles. As known to the skilled person, human skin, as it ages, gradually loses skin
components that keep skin pliant and youthful-looking. The skin components include collagen, elastin, and hyaluronic acid, which have been the subject of interest and use to improve the appearance of aging skin.
The present disclosure includes compositions of microalgal polysaccharides, microalgal cell
extracts, and microalgal cell homogenates for use in the same manner as collagen and hyaluronic acid. In some embodiments, the polysaccharides will be those of from a *Porphyridium* species, such as one that has been subject to genetic and/or nutritional manipulation to produce polysaccharides with altered monosaccharide content and/or altered sulfation. In some embodiments, the polysaccharides are formulated as a fluid, optionally elastic and/or viscous, suitable for injection. The compositions may be used as injectable dermal fillers as one non-limiting example. The injections may be made into skin to fill-out facial lines and wrinkles. In other embodiments, the injections may be used for lip enhancement. These applications of polysaccharides are non-limiting examples of non-pharmacological therapeutic methods of the disclosure.
In further embodiments, the microalgal polysaccharides, cell extracts, and cell
homogenates of the present disclosure may be co-formulated with collagen and/or hyaluronic acid (such as the Restylane® and Hylaform® products) and injected into facial tissue. Non-limiting examples of such tissue include under the skin in areas of wrinkles and the lips. In a preferred embodiment, the polysaccharide is substantially free of protein. The injections may
be repeated as deemed appropriate by the skilled practitioner, such as with a periodicity of about three, about four, about six, about nine, or about twelve months. In another preferred embodiment, a hyaluronic acid material is mixed with a polysaccharide from the genus Porphyridium prior to co-administration. The present disclosure in this particular embodiment provides longer half-life to the hyaluronic acid due to the potent inhibition of hyaluronidase by polysaccharides isolated from microalgae from the genus Porphyridium. This allows for less injections to a patient. Preferably the polysaccharide from the genus Porphyridium is at least substantially free of protein. Preferably the mixture of polysaccharide from the genus Porphyridium and hyaluronic acid is sterile.
Thus the disclosure includes a method of cosmetic enhancement comprising injecting
a polysaccharide produced by microalgae into mammalian skin. The injection may be of an effective amount to produce a cosmetic improvement, such as decreased wrinkling or decreased appearance of wrinkles as non-limiting examples. Alternatively, the injection may be of an amount which produces relief in combination with a series of additional injections.
In some methods, the polysaccharide is produced by a microalgal species, or two or more
species, listed in Table 1. In one non-limiting example, the microalgal species is of the genus *Porphyridium* and the polysaccharide is substantially free of protein.

The present disclosure further includes a method to inhibit hyaluronidase activity comprising contacting the hyaluronidase with a disclosed polysaccharide. In some embodiments, the hyaluronidase activity is in the skin or a skin tissue of a human subject and the contacting comprises administering the polysaccharide to the subject. The administering may comprise injection of the polysaccharide, or a polysaccharide containing composition of the present disclosure, to the skin or skin tissue and/or to the lips or a lip tissue. The amount of polysaccharide administered may be any that is sufficient or effective to inhibit hyaluronidase activity to a level as desired by a skilled person. The level of reduction in hyaluronidase activity may be determined by routine methods, including a comparative method wherein the activity in the presence of polysaccharide is compared to the activity in the absence thereof. Thus the amount of reduction may be at least about 10%, about 20%, about 30%, about 40%, about 50%, about 60%, about 70%, about 80%, or about 90% or higher than that observed in the
absence of polysaccharide. In a preferred embodiment the polysaccharide used to inhibit hyaluronidase is from a species of the genus Porphyridium.

The present disclosure also includes a method to stimulate procollagen and/or collagen synthesis or production in a cell, such as a human fibroblast, by contacting the cell with a disclosed polysaccharide. In some embodiments, the cell is in the skin of a human subject and the contacting comprises administering the polysaccharide to the subject. The administering may comprise injection of the polysaccharide, or a polysaccharide containing composition of the present disclosure, to the skin or a skin tissue and/or to the lips or a lip tissue. The amount of polysaccharide administered may be any that is sufficient or effective to stimulate
procollagen or collagen synthesis to a level desired by a skilled person, such as an increase of at least about 5%, 10%, about 20%, or about 30% or higher than that observed in the absence of polysaccharide. In a related manner, the polysaccharide may be used to inhibit collagenase activity. The inhibition may be sufficient to result in an increase of procollagen or collagen levels as described above.
Additionally, the present disclosure includes a method to stimulate elastin synthesis or
production in a cell, such as a fibroblast, by contacting the cell with a disclosed polysaccharide. In a related manner, the polysaccharide may also inhibit elastase activity produced by a cell, such as, but not limited to, a fibroblast. In some embodiments, the cell is in the skin of a human subject and the contacting comprises administering the polysaccharide
to the subject. The administering may comprise injection of the polysaccharide, or a polysaccharide containing composition of the present disclosure, to the skin or a skin tissue. The amount of polysaccharide administered may be any that is sufficient or effective to stimulate elastin synthesis to a level desired by a skilled person, such as an increase of at least about 50%, 100%, about 200%, or about 300% or higher than that observed in the absence of polysaccharide. In a preferred embodiment the polysaccharide stimulating elastin secretion contains at least 5.0% sulfur by weight. Similarly, the polysaccharide may decrease elastase activity by about 10%, about 20%, about 30%, about 40%, about 50%, or about 60% or higher than that observed in the absence of polysaccharide.
The present disclosure further includes the use of the disclosed polysaccharides based on their observed anti-oxidant activity. Thus the disclosure includes a method of providing anti-oxidant activity to skin or a skin tissue, such as that of a human subject, by administering a polysaccharide. In some embodiments, the method inhibits reactive oxygen species (ROS) formation and/or activity in the skin. The disclosure thus includes a method to prevent or treat a disease or unwanted condition associated with ROS or oxidative stress. Non-limiting
examples of such a disease or unwanted condition include reducing inflammation or irritation
of human skin or lips. In some embodiments, the polysaccharide composition comprises one or more other agents or compounds with anti-oxidant activity. Non-limiting examples of other agents include vitamin A (retinyl palmitate), vitamin C (such as one or more of ascorbyl palmitate, sodium ascorbyl palmitate, and tetrahexyldecyl ascorbate), vitamin D
(cholecalcipherol), vitamin E (such as tocopheryl acetate and tocopherol/D-alpha tocopherol), alpha lipoic acid, coenzyme, L-selenomethionine, and beta glucan.

In a related manner, a polysaccharide is used based on its anti-inflammatory in skin or a skin tissue. In some embodiments, the method inhibits polymorphonuclear (PMN) leukocytes in chemotaxis, such as to sites of inflammation in skin. The level of inhibition may be about 10%, about 20%, about 30%, about 40%, or about 50% or more than that seen in the absence of polysaccharide. In other embodiments, the method inhibits the synthesis or release of a pro-inflammatory cytokinc, such as interferon-gamma or interleukin-1-alpha. With interferon-gamma as an example, the inhibition may be at least about 10%, about 20%,
about 30%, about 40%, about 50%, about 60%, about 70%, about 80%, or about 90% or more than that observed in the absence of polysaccharide. With interleukin- 1 -alpha as an example, the inhibition may be at least about 10%, about 20%, about 30%, about 40%, about 50%, about 60%, about 70%, or about 80% or more than that observed in the absence of polysaccharide. In further embodiments, the method inhibits proliferation of peripheral blood
mononuclear cells, including lymphocytes, monocytes, and macrophages. The level of inhibition may be about 10%, about 20%, about 30%, about 40%, about 50%, about 60%, about 70%, or about 80% or more than that observed in the absence of polysaccharide.

The above described methods may be individually part of a method to reduce the
signs of aging or reduce the appearance of aging in human skin as described herein. The methods may also be based upon the insight that the microalgal biomass and polysaccharides of the present disclosure also reduce the effects of UV light or radiation. In some embodiments, the polysaccharide reduces thymidine dimer formation in DNA caused by exposure to UVB irradiation. The reduction may be at least about 10%, about 20%, about 30%, about 40%, about 50%, about 60%, about 70%, or about 80% or more than that observed in the absence
of polysaccharide.

In a related manner, the disclosed methods can be used to shield human skin or lip tissue from UV light radiation. The UV radiation may comprise UVA and/or UVB. The method may comprise applying a composition of the disclosed to skin or a skin tissue in an effective or sufficient amount to shield, at least in part, the skin from UV
radiation. In some embodiments, the amount is that which reduces thymidine dimer
formation and/or sunburn. In an alternative embodiment, a composition of the disclosure may be applied in an effective or sufficient amount, such as that which reduces further UV-mediated damage, to treat skin that has been damaged by UV radiation. An additional non-limiting example is a method of for treating skin to reduce the risk of skin cancer induced by sunlight or UV radiation.

The polysaccharide compositions may be in the form of a sterile and/or non-pyrogenic injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a nontoxic parenterally-acceptable diluent or solvent, for example, as a solution in 1,3-butanediol. Among the acceptable vehicles and solvents that may be employed are mannitol, water, Ringers solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

Sterile injectable polysaccharide compositions preferably contain less than 1% protein as a function of dry weight of the composition, more preferably less than 0.1% protein, more preferably less than 0.01% protein, less than 0.001% protein, less than 0.0001% protein, more preferably less than 0.00001% protein, more preferably less than 0.000001% protein.

### V Gene Expression in Microalgae

Genes can be expressed in microalgae by providing, for example, coding sequences in operable linkage with promoters.

An exemplary vector design for expression of a gene in microalgae contains a first gene in operable linkage with a promoter active in algae, the first gene encoding a protein that imparts resistance to an antibiotic or herbicide. Optionally the first gene is followed by a 3' untranslated sequence containing a polyadenylation signal. The vector may also contain a second promoter active in algae in operable linkage with a second gene. The second gene can encode any protein, for example an enzyme that produces small molecules or a mammalian growth hormone that can be advantageously present in a nutraccutical.

It is preferable to use codon-optimized cDNAs: for methods of recoding genes for expression in microalgae, see for example US patent application 20040209256.

It has been shown that many promoters in expression vectors are active in algae, including both promoters that are endogenous to the algae being transformed algae as well as promoters that are not endogenous to the algae being transformed (ie: promoters from other algae, promoters from plants, and promoters from plant viruses or algae viruses). Example of methods for transforming microalgae, in addition to those demonstrated in the Examples section below, including methods comprising the use of exogenous and/or endogenous promoters that are active in microalgae, and antibiotic resistance genes functional in microalgae, have been described. See for example; Curr Microbiol. 1997 Dec;35(6):356-62 (Chlorella vulgaris); Mar Biotechnol (NY). 2002 Jan;4(1):63-73 (Chlorella ellipsoidea); Mol Gen Genet. 1996 Oct 16;252(5):572-9 (Phaeodactylum tricornutum); Plant Mol Biol. 1996 Apr;31(1):1-12 (Volvox carteri); Proc Natl Acad Sci USA. 1994 Nov 22;91(24):11562-6 (Volvox carteri); Falciatore A, Casotti R, Leblanc C, Abrescia C, Bowler C, PMID: 10383998, 1999 May;1(3):239-251 (Laboratory of Molecular Plant Biology, Stazione Zoologica, Villa Comunale, 1-80121 Naples, Italy) (Phaeodactylum tricomutum and Thalassiosira weissflogii); Plant Physiol. 2002 May;129(1):7-12. (Porphyridium sp.); Proc Natl Acad Sci USA. 2003 Jan 21;100(2):438-42. (Chlamydomonas reinhardtii); Proc Natl Acad Sci USA. 1990 Feb;87(3):1228-32. (Chlamydomonas reinhardtii); Nucleic Acids Res. 1992 Jun 25;20(12):2959-65; Mar Biotechnol (NY). 2002 Jan;4(1):63-73 (Chlorella); Biochem Mol Biol Int. 1995 Aug;36(5):1025-35 (Chlamydomonas reinhardtii); J Microbiol. 2005 Aug;43(4):361-5 (Dunaliella); Yi Chuan Xue Bao. 2005 Apr;32(4):424-33 (Dunaliella); Mar Biotechnol (NY). 1999 May;1(3):239-251. (Thalassiosira and Phaedactylum); Koksharova, Appl Microbiol Biotechnol 2002 Feb;58(2):123-37 (various species); Mol Genet Genomics. 2004 Feb;271(1):50-9 (Thermosynechococcus elongates); J. Bacteriol. (2000), 182, 211-215; FEMS Microbiol Lett. 2003 Apr 25;221(2):155-9; Plant Physiol. 1994 Jun;105(2):635-41; Plant Mol Biol. 1995 Dec;29(5):897-907 (Synechococcus PCC 7942); Mar Pollut Bull. 2002;45(1-12):163-7 (Anabaena PCC 7120); Proc Natl Acad Sci USA. 1984 Mar;81(5):1561-5 (Anabaena (various strains)); Proc Natl Acad Sci USA. 2001 Mar 27;98(7):4243-8 (Synechocystis); Wirth, Mol Gen Genet 1989 Mar;216(1):175-7 (various species); Mol Microbiol, 2002 Jun;44(6):1517-31 and Plasmid, 1993 Sep;30(2):90-105 (Fremyella diplosiphon); Hall et al. (1993) Gene 124: 75-81 (Chlamydomonas reinhardtii); Gruber et al. (1991). Current Micro. 22: 15-20; Jarvis et al. (1991) Current Genet. 19: 317-322 (Chlorella); for additional promoters see also Table 1 from US Patent 6,027,900).

Suitable promoters may be used to express a nucleic acid sequence in microalgae. In some embodiments, the sequence is that of an exogenous gene or nucleic acid. In some embodiments the exogenous gene can encode a superoxide dismutase (SOD) or an SOD fusion. In cases of an exogenous nucleic acid coding sequence, the codon usage may be optionally optimized in whole or in part to facilitate expression in microalgae.

The disclosure includes cells of the genus Porphyridium that
have been stably transformed with a vector containing a selectable marker gene in operable linkage with a promoter active in microalgae. The disclosure includes cells of the genus Porphyridium that have been stably transformed with a vector containing a selectable marker gene in operable linkage with a promoter endogenous to a member of the Rhodophyte order. Such promoters include SEQ ID NOs: 6, 20 and 21, promoters from the
genome of Chondrus crispus (Genbank accession number Z47547), promoters from the
genome of Cyanidioschyzon merolae (see for example Matsuzaki, M. et al. Nature 428, 653657 (2004); Plant Physiology 137:567-585 (2005); entire sequence available at http://merolae.biol.s.u-tokyo.ac.jp/db/chromosome.cgi). The disclosure includes cells of the genus Porphyridium that have been stably transformed with a vector
containing a selectable marker gene in operable linkage with a promoter other than a CMV promoter such as that found in PCT application WO2006013572.

The disclosure provides for the expression of a protein sequence found to be tightly associated with microalgal polysaccharides. One non-limiting example is the protein of SEQ ID NO: 15, which has been shown to be tightly associated with, but not
covalently bound to, the polysaccharide from *Porphyridium sp.* (see J. Phycol. 40: 568-580 (2004)). When *Porphyridium* culture media is subjected to tangential flow filtration using a filter containing a pore size well in excess of the molecular weight of the protein of SEQ ID NO: 15, the polysaccharide in the retentate contains detectable amounts of the protein, indicating its tight association with the polysaccharide. The calculated molecular weight of
the protein is approximately 58kD, however with glycosylation the protein is approximately 66 kD.

Such a protein may be expressed directly such that it will be present with the polysaccharides as disclosed or expressed as part of a fusion or chimeric protein as described herein. As a fusion protein, the portion that is tightly associated with a microalgal
polysaccharide effectively links the other portion(s) to the polysaccharide. A fusion protein may comprise a second protein or polypeptide, with a homogenous or heterologous sequence. A homogenous sequence would result in a dimer or multimer of the protein while a heterologous sequence can introduce a new functionality, including that of a bioactive protein or polypeptide.

Non-limiting examples of the second protein include an enzyme. In optional embodiments, the enzyme is superoxide dismutase, such as that has at least about 60% amino acid identity with the sequence of SEQ ID NO: 12, SEQ ID NO: 13, and proteins from Table 21 as non-limiting examples. In some embodiments, the superoxide dismutase has at least about 70%, at least about 75%, at least about 80%, at least about 85%, at least about 90%, at least about 95%, or at least about 98%, or higher, amino acid identity with the sequence of SEQ ID NO:12 or 13 or a protein from Table 21. In other embodiments, the enzyme is a phytase (such as GenBank accession number CAB91845 and US Patents 6,855,365 and 6,110,719). Other examples of second proteins include structural proteins from mammalian
skin such as collagen and elastin. Assays such as western blot and ELISAs can be used to confirm the presence of the second protein in the biomass as well as when it is attached to a purified polysaccharide. Polysaccharides with fusion proteins bound can be purified as in Example 2. activity assays for proteins such as phytases and superoxide dismutase are well known in the art.
One advantage to a fusion is that the bioactivity of the polysaccharide and the
bioactivity from the protein can be combined in a product without increasing the manufacturing cost over only purifying the polysaccharide. As a non-limiting example, the potent antioxidant properties of a *Porphyridium* polysaccharide can be combined with the potent antioxidant properties of superoxide dismutase in a fusion, however the
polysaccharide:superoxide dismutase combination can be isolated to a high level of purity using tangential flow filtration. In another non-limiting example, the potent antiviral properties of a *Porphyridium* polysaccharide can be added to the potent neutralizing activity of recombinant antibodies fused to the protein (SEQ ID NO:15) that tightly associates with the polysaccharide.
Preferred carbohydrate transporters for expression in Porphyridium are SEQ ID NOs: 33-35 and 38-40.

In other embodiments, the method includes genetic expression methods comprising the use of an expression vector. In one method, a rnicroalgal cell, such as a *Porphyridium* cell, is transformed with a dual expression vector under conditions wherein vector mediated
gene expression occurs. The expression vector may comprise a resistance cassette
comprising a gene encoding a protein that confers resistance to an antibiotic such as zeocin, operably linked to a promoter active in microalgae. The vector may also comprise a second expression cassette comprising a second protein to a promoter active in microalgae. The two cassettes are physically linked in the vector. The transformed cells may be optionally
selected based upon the ability to grow in the presence of the antibiotic or other selectable marker under conditions wherein cells lacking the resistance cassette would not grow, such as in the dark. The resistance cassette, as well as the expression cassette, may be taken in whole or in part from another vector molecule.

In one non-limiting example, a method of expressing an exogenous gene in a cell of the genus *Porphyridium* is provided. The method may comprise operably linking a gene encoding a protein that confers resistance to the antibiotic zeocin to a promoter active in microalgae to form a resistance cassette; operably linking a gene encoding a second protein to a promoter active in microalgae to form a second expression cassette, wherein the resistance cassette and second expression cassette are physically connected to form a dual expression vector; transforming the cell with the dual expression vector; and selecting for the ability to survive in the presence of at least 2.5 ug/ml zeocin, preferably at least 3.0 ug/ml zeocin, and more preferably at least 3.5 ug/ml zeocin, more preferably at least 5.0 ug/ml zeocin.

In additional aspects, the expression of a protein that produces small molecules in microalgae is included and described. Some genes that can be expressed using the methods provided herein encode enzymes that produce nutraceutical small molecules such as lutein, zeaxanthin, and DHA. Preferably the genes encoding the proteins are synthetic and are created using preferred codons on the microalgae in which the gene is to be expressed. For example, enzyme capable of turning EPA into DHA are cloned into the microalgae *Porphyridium sp.* by recoding genes to adapt to *Porphyridium sp.* preferred codons. For examples of such enzymes see Nat Biotechnol. 2005 Aug;23(8):1013-7. For examples of enzymes in the carotenoid pathway see SEQ ID NOs: 18 and 19 and Table 22. The advantage to expressing such genes is that the nutraceutical value of the cells increases without increasing the manufacturing cost of producing the cells.

For sequence comparison to determine percent nucleotide or amino acid identity, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel *et al., supra).*
Another example of algorithm that is suitable for determining percent sequence
identity and sequence similarity is the BLAST algorithm, which is described in Altschul et al., J. Mol. Biol. 215:403-410 (1990). Software for performing BLAST analyses is publicly available through the National Center for Biotechnology Information (http://www.nebi.nlm.nih.gov/). This algorithm involves first identifying high scoring
sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra.).* These initial neighborhood word hits act as seeds for initiating searches to find longer HSPs containing them. The word hits are then extended in
both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when:
the cumulative alignment score falls off by the quantity X from its maximum achieved value; the cumulative score goes to zero or below, due to the accumulation of one or more negative-scoring residue alignments; or the end of either sequence is reached. For identifying whether a nucleic acid or polypeptide is appropriate, the default parameters of the BLAST programs are suitable. The BLASTN program (for nucleotide sequences) uses as
defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix. The TBLATN program (using protein sequence for nucleotide sequence) uses as defaults a word length (W) of 3, an expectation (E) of 10, and a BLOSUM 62 scoring matrix. (see Henikoff & Henikoff, Proc. Natl. Acad. Sci. USA 89:10915 (1989)).

In addition to calculating percent sequence identity, the BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

### EXAMPLES

### EXAMPLE 1

Growth of Porphyridium cruentum and Porphyridium sp.

Porphyridium sp. (strain UTEX 637) and Porphyridium cruentum (strain UTEX 161) were inoculated into autoclaved 2 liter Erlenmeyer flasks containing an artificial seawater media:
1495 ASW medium recipe from the American Type Culture Collection
(components are per 1 liter of media)

| | |
|---|---|
| NaCl | 27.0 g |
| MgSO₄ . 7H_{2O} | 6.6 g |
| MgCl₂ . 6H₂O | 5.6 g |
| CaCl₂ . 2H₂O | 1.5 g |
| KNO₃ | 1.0 g |
| KH₂PO₄ | 0.07 g |
| NaHCO₃ | 0.04g |
| 1.0 M Tris-HCl buffer, pH 7.6 | 20.0 ml |
| Trace Metal Solution (see below) | 1.0 ml |
| Chelated Iron Solution (see below) | 1.0 ml |
| Distilled water | bring to 1.0 L |

### Trace Metal Solution:

| | |
|---|---|
| ZnCl₂ | 4.0mg |
| H₃BO₃ | 60.0mg |
| CoCl₂ . 6H₂O | 1.5mg |
| CuCl2 . 2H₂O | 4.0 mg |
| MnCl₂ . 4H₂O | 40.0 mg |
| (NH₄)₆Mo₇O₂₄ .4H₂O | 37.0 mg |
| Distilled water | 100.0 ml |

### Chelated Iron Solution:

| | |
|---|---|
| FeCl₃ . 4H₂O | 240.0 mg |
| 0.05 M EDTA, pH7.6 | 100.0 ml |

Media was autoclaved for at least 15 minutes at 121°C.

Inoculated cultures in 2 liter flasks were maintained at room temperature on stir plates. Stir bars were placed in the flasks before autoclaving. A mixture of 5% CO₂ and air was bubbled into the flasks. Gas was filter sterilized before entry. The flasks were under 24 hour illumination from above by standard fluorescent lights (approximately 150 uE/m⁻¹ /s⁻¹). Cells were grown for approximately 12 days, at which point the cultures contained approximately of 4 X 10⁶ cells/mL.

### EXAMPLE 2

Dense *Porphyridium sp.* and *Porphyridium cruentum* cultures were centrifuged at 4000 rcf. The supernatant was subjected to tangential flow filtration in a Millipore Pellicon 2 device through a 1000kD regenerated cellulose membrane (filter catalog number P2C01MC01). Approximately 4.1 liters of *Porphyridium cruentum* and 15 liters of *Porphyridium sp.* supernatants were concentrated to a volume of approximately 200 ml in separate experiments. The concentrated exopolysaccharide solutions were then diafiltered with 10 liters of 1mM Tris (pH 7.5). The retentate was then flushed with 1mM Tris (pH 7.5), and the total recovered polysaccharide was lyophilized to completion. Yield calculations were performed by the dimethylmethylene blue (DMMB) assay. The lyophilized polysaccharide was resuspended in deionized water and protein was measured by the bicinchoninic acid (BCA) method. Total dry product measured after lyophilization was 3.28g for *Porphyridium sp.* and 2.0g for *Porphyridium cruentum.* Total protein calculated as a percentage of total dry product was 12.6% for *Porphyridium sp.* and 15.0% for *Porphyridium cruentum.*

### EXAMPLE 3

### Increasing solvent-available polysaccharide

A measured mass (approximately 125 grams) of freshly harvested *Porphyridium sp.* cells, resuspended in a minimum amount of dH₂O sufficient to allow the cells to flow as a liquid, was placed in a container. The cells were subjected to increasing amounts of sonication over time at a predetermined sonication level. Samples were drawn at predetermined time intervals, suspended in measured volume of dH₂O and diluted appropriately to allow visual observation under a microscope and measurement of polysaccharide concentration of the cell suspension using the DMMB assay. A plot was made of the total amount of time for which the biomass was sonicated and the polysaccharide concentration of the biomass suspension. Two experiments were conducted with different time intervals and total time the sample was subjected to sonication. The first data set from sonication experiment 1 was obtained by subjecting the sample to sonication for a total time period of 60 minutes in 5 minute increments. The second data set from sonication experiment 2 was obtained by subjecting the sample to sonication for a total time period of 6 minutes in 1-minute increments. The data, observations and experimental details are described below. Standard curves were generated using TFF-purified, lyophilized, weighed, resuspended *Porphyridium sp.* exopolysaccharide.

### General parameters of sonication experiments I and 2

Cells were collected and volume of the culture was measured. The biomass was separated from the culture solution by centrifugation. The centrifuge used was a Forma Scientific Centra-GP8R refrigerated centrifuge. The parameters used for centrifugation were 4200rpm, 8 minutes, rotor# 218. Following centrifugation, the biomass was washed with dH₂O. The supernatant from the washings was discarded and the pelleted cell biomass was collected for the experiment.

A sample of 100µL of the biomass suspension was collected at time point 0 (0TP) and suspended in 900µL dH₂O. The suspension was further diluted ten-fold and used for visual observation and DMMB assay. The time point 0 sample represents the solvent-available polysaccharide concentration in the cell suspension before the cells were subjected to sonication. This was the baseline polysaccharide value for the experiments.

The following sonication parameters were set: power level=8, 20 seconds ON / 20 seconds OFF (Misonix 3000 Sonicator with flat probe tip). The container with the biomass was placed in an ice bath to prevent overheating and the ice was replenished as necessary. The sample was prepared as follows for visual observation and DMMB assay: 100 µL of the biomass sample + 900µL dH₂O was labeled as dilution 1. 100 µL of (i) dilution 1 + 900µL dH₂O for cell observation and DMMB assay.

### Sonication Experiment 1

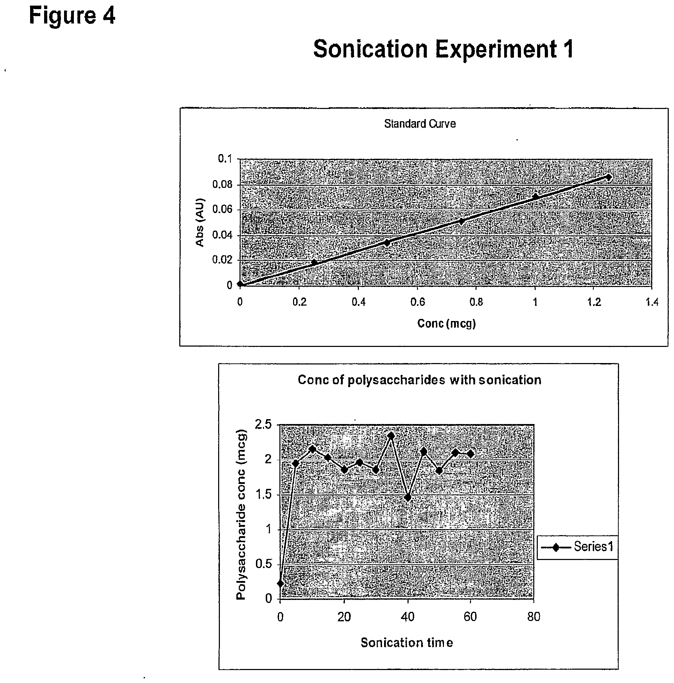

In the first experiment the sample was sonicated for a total time period of 60 minutes, in 5-minute increments (20 seconds ON / 20 seconds OFF). The data is presented in Tables 4, 5 and 6. The plots of the absorbance results are presented in Figure 4.

**TABLE 4: SONICATION RECORD - EXPERIMENT 1**

| Ser# | Time point (min) | Observations |
|---|---|---|
| 1 | 0 | Healthy red cells |
| 2 | 5 | Red color disappeared, small greenish circular particles |
| 3 | 10 | Small particle, smaller than 5 minute TP |
| 4 | 15 | Small particle, smaller than 10 minute TP. Same observation as 10 minute time |
| 5 | 20 | Similar to 15 minute TP. Small particles; empty circular shells in the field of vision |
| 6 | 25 | Similar to 20 minute TP |
| 7 | 30 | Similar to 25 minute TP, particles less numerous |
| 8 | 35 | Similar to 30 minute TP |
| 9 | 40 | Similar to 35 minute TP |
| 10 | 45 | Similar to 40 minute TP |
| 11 | 50 | Very few shells, mostly fine particles |
| 12 | 55 | Similar to 50 minute TP. |
| 13 | 60 | Fine particles, hardly any shells |

| | | |
|---|---|---|
| TP = time point. | | |

**TABLE 5: STANDARD CURVE RECORD - - SONICATION EXPERIMENT 1**

| Absorbance (AU) | Concentration (µg) |
|---|---|
| 0 | Blank, 0 |
| 0.02 | 0.25 |
| 0.03 | 0.5 |
| 0.05 | 0.75 |
| 0.07 | 1.0 |
| 0.09 | 1.25 |

**Table 6: Record of Sample Absorbance versus Time Points - Sonication Experiment 1**

| SAMPLE TIME POINT (MIN) | Sol vent-A vailable Polysaccharide (µg) |
|---|---|
| 0 | 0.23 |
| 5 | 1.95 |
| 10 | 2.16 |
| 15 | 2.03 |
| 20 | 1.86 |
| 25 | 1.97 |
| 30 | 1.87 |
| 35 | 2.35 |
| 40 | 1.47 |
| 45 | 2.12 |
| 50 | 1.84 |
| 55 | 2.1 |
| 60 | 2.09 |

The plot of polysaccharide concentration versus sonication time points is displayed above and in Figure 4. Solvent-available polysaccharide concentration of the biomass (cell) suspension reaches a maximum value after 5 minutes of sonication. Additional sonication in 5-minute increments did not result in increased solvent-available polysaccharide concentration.

Homogenization by sonication of the biomass resulted in an approximately 10-fold increase in solvent-available polysaccharide concentration of the biomass suspension, indicating that homogenization significantly enhances the amount of polysaccharide available to the solvent. These results demonstrate that physically disrupted compositions of *Porphyridium* for oral or other administration provide novel and unexpected levels or polysaccharide bioavailability compared to compositions of intact cells. Visual observation of the samples also indicates rupture of the cell wall and thus release of insoluble cell wall-bound polysaccharides from the cells into the solution that is measured as the increased polysaccharide concentration in the biomass suspension.

### Sonication Experiment 2

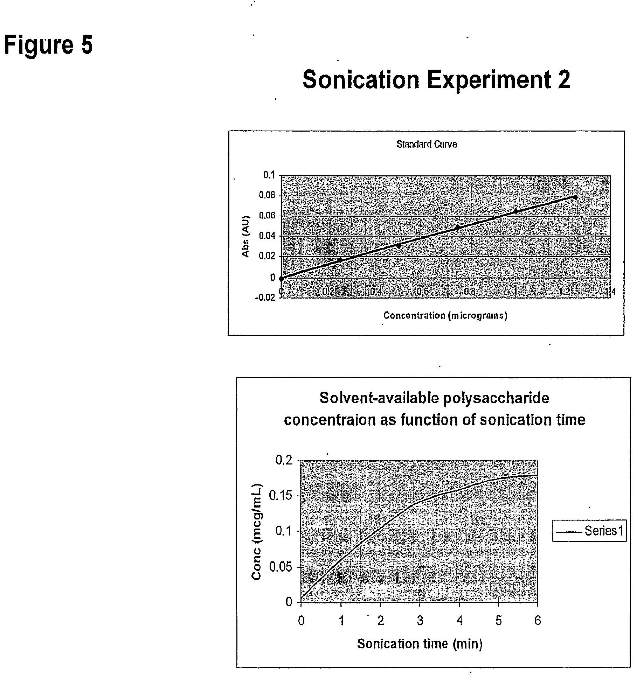

In the second experiment the sample was sonicated for a total time period of 6 minutes in 1-minute increments. The data is presented in Tables 7, 8 and 9. The plots of the absorbance results are presented in Figure 5.

**TABLE 7: SONICATION EXPERIMENT 2**

| Ser# | Time point (min) | Observations |
|---|---|---|
| 1 | 0 | Healthy red-brown cells appear circular |
| 2 | 1 | Circular particles scattered in the field of vision with few healthy cells. Red color has mostly disappeared from cell bodies. |
| 3 | 2 | Observation similar to time point 2 minute. |
| 4 | 3 | Very few healthy cells present. Red color has disappeared and the concentration of particles closer in size to whole cells has decreased dramatically. |
| 5 | 4 | Whole cells are completely absent. The particles are smaller and fewer in number. |
| 6 | 5 | Observation similar to time point 5 minute. |
| 7 | 6 | Whole cells are completely absent. Large particles are completely absent. |

**TABLE 8: STANDARD CURVE RECORD - SONICATION EXPERIMENT 2**

| Absorbance (AU) | Concentration (µg) |
|---|---|
| - 0.001 | Blank, 0 |
| 0.017 | 0.25 |
| 0.031 | 0.5 |
| 0.049 | 0.75 |
| 0.0645 | 1.0 |
| 0.079 | 1.25 |

**Table 9: Record of Sample Absorbance versus Time Points - Sonication Experiment 2**

| SAMPLE TIME POINT (MIN) | Solvent-Available Polysaccharide (µg) |
|---|---|
| 0 | 0.063 |
| 1 | 0.6 |
| 2 | 1.04 |
| 3 | 1.41 |
| 4 | 1.59 |
| 5 | 1.74 |
| 6 | 1.78 |

The value of the solvent-available polysaccharide increases gradually up to the 5 minute time point as shown in Table 9 and Figure 5.

### EXAMPLE 4

### Alcohol precipitation

*Porphyridium sp.* culture was centrifuged at 4000 rcf and supernatant was collected. The supernatant was divided into six 30ml aliquots. Three aliquots were autoclaved for 15 min at 121°C. After cooling to room temperature, one aliquot was mixed with methanol (58.3% vol/vol), one was mixed with ethanol (47.5% vol/vol) and one was mixed with isopropanol (50% vol/vol). The same concentrations of these alcohols were added to the three supernatant aliquots that were not autoclaved. Polysaccharide precipitates from all six samples were collected immediately by centrifugation at 4000 rcf at 20°C for 10 min and pellets were washed in 20% of their respective alcohols. Pellets were then dried by lyophilization and resuspended in 15 ml deionized water by placement in a 60°C water bath. Polysaccharide pellets from non-autoclaved samples were partially soluble or insoluble. Polysaccharide pellets from autoclaved ethanol and methanol precipitation were partially soluble. The polysaccharide pellet obtained from isopropanol precipitation of the autoclaved supernatant was completely soluble in water.

### EXAMPLE 5

### Monosaccharide analysis

Approximately 10 milligrams of purified polysaccharide from *Porphyridium sp.* and *Porphyridium cruentum* (described in Example 3) were subjected to monosaccharide analysis.

Monosaccharide analysis was performed by combined gas chromatography/mass spectrometry (GC/MS) of the per-O-trimethylsilyl (TMS) derivatives of the monosaccharide methyl glycosides produced from the sample by acidic methanolysis.

Methyl glycosides prepared from 500µg of the dry sample provided by the client by methanolysis in 1 M HCl in methanol at 80°C (18-22 hours), followed by re-N-acetylation with pyridine and acetic anhydride in methanol (for detection of amino sugars). The samples were then per-O-trimethylsilylated by treatment with Tri-Sil (Pierce) at 80°C (30 mins). These procedures were carried out as previously described described in Merkle and Poppe (1994) Methods Enzymol. 230: 1-15; York, et al. (1985) Methods Enzymol. 118:3-40. GC/MS analysis of the TMS methyl glycosides was performed on an HP 5890 GC interfaced to a 5970 MSD, using a Supelco DB-1 fused silica capillary column (30m 0.25 mm ID). Monosaccharide compositions were determined as follows:

**Table 10: Porphyridium sp. monosaccharide analysis**

| Glycosyl residue | Mass (µg) | Mole % |
|---|---|---|
| Arabinose (Ara) | n.d. | n.d. |
| Rhamnose (Rha) | 2.7 | 1.6 |
| Fucose (Fuc) | n.d. | n.d. |
| Xylose (Xyl) | 70.2 | 44.2 |
| Glucuronic acid (GlcA) | n.d. | n.d. |
| Galacturonic acid (GalA) | n.d. | n.d. |
| Mannose (Man) | 3.5 | 1.8 |
| Galactose (Gal) | 65.4 | 34.2 |
| Glucose (Glc) | 34.7 | 18.2 |
| *N*-acetyl galactosamine (GalNAc) | n.d. | n.d. |
| *N*-acetyl glucosamine (GlcNAc) | trace | trace |
| | ∑=176.5 | |

**Table 11: Porphyridium cruentum monosaccharide analysis**

| Glycosyl residue | Mass (µg) | Mole % |
|---|---|---|
| Arabinose (Ara) | n.d. | n.d. |
| Rhamnose (Rha) | n.d. | n.d. |
| Fucose (Fuc) | n.d. | n.d. |
| Xylose (Xyl) | 148.8 | 53.2 |
| Glucuronic Acid (GlcA) | 14.8 | 4.1 |
| Mannose (Man) | n.d. | n.d. |
| Galactose (Gal) | 88.3 | 26.3 |
| Glucose (Glc) | 55.0 | 16.4 |
| N-acetyl glucosamine (GlcNAc) | trace | trace |
| N-acetyl neuraminic acid (NANA) | n.d. | n.d. |
| | ∑= 292.1 | |

| | | |
|---|---|---|
| Mole % values are expressed as mole percent of total carbohydrate in the sample. n.d.= none detected. | | |

### EXAMPLE 6

### Protein measurement

*Porphyridium sp.* was grown as described. 2 liters of centrifuged *Porphyridium sp.* culture supernatant were autoclaved at 121°C for 20 minutes and then treated with 50% isopropanol to precipitate polysaccharides. Prior to autoclaving the 2 liters of supernatant contained 90.38 mg polysaccharide. The pellet was washed with 20% isopropanol and dried by lyophilization. The dried material was resuspended in deionized water. The resuspended polysaccharide solution was dialyzed to completion against deionized water in a Spectra/Por cellulose ester dialysis membrane (25,000 MWCO). 4.24% of the solid content in the solution was proteins as measured by the BCA assay.

### EXAMPLE 7

### Generation of protein-free polysaccharide

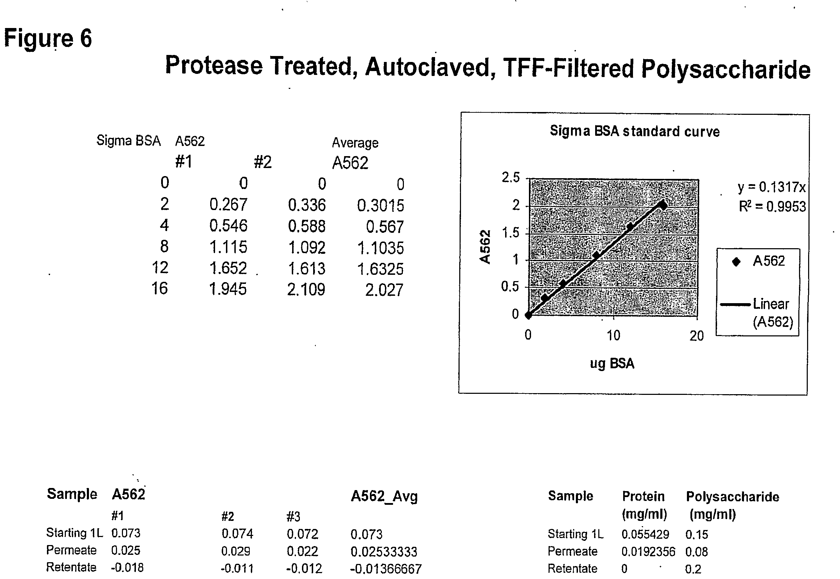

*Porphyridium sp.* was grown as described. 1 liters of centrifuged *Porphyridium sp.* culture supernatant was autoclaved at 121°C for 15 minutes and then treated with 10% protease (Sigma catalog number P-5147; protease treatment amount relative to protein content of the supernatant as determined by BCA assay). The protease reaction proceeded for 4 days at 37°C. The solution was then subjected to tangential flow filtration in a Millipore Pellicon® cassette system using a 0.1 micrometer regenerated cellulose membrane. The retentate was diafiltered to completion with deionized water. No protein was detected in the diafiltered retentate by the BCA assay. See Figure 6.

Optionally, the retentate can be autoclaved to achieve sterility if the filtration system is not sterile. Optionally the sterile retentate can be mixed with pharmaceutically acceptable carrier(s) and filled in vials for injection.

Optionally, the protein free polysaccharide can be fragmented by, for example, sonication to reduce viscosity for parenteral injection as, for example, an antiviral compound. Preferably the sterile polysaccharide is not fragmented when prepared for injection as a joint lubricant.

### EXAMPLE 8

### Heterotrophic growth of Porphyridium

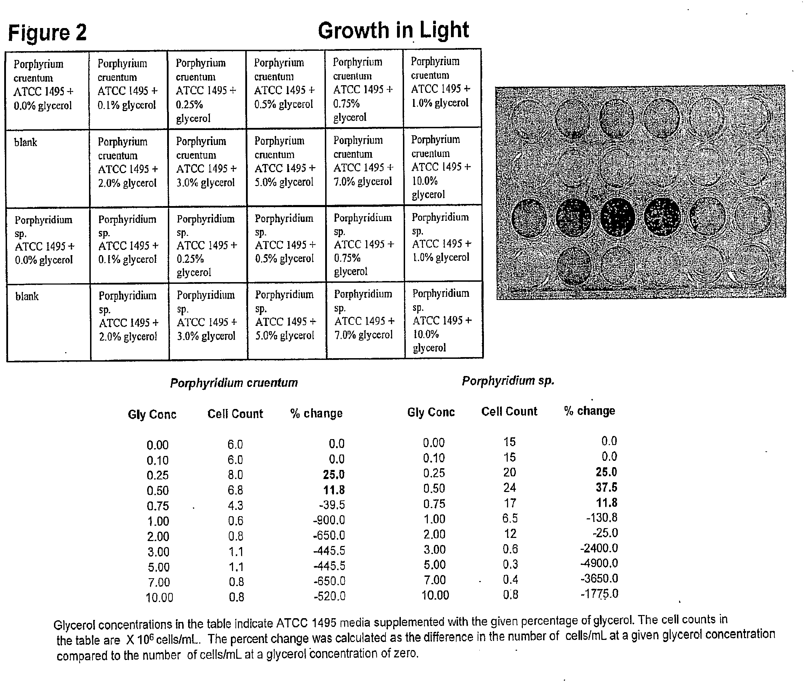

Cultures of *Porphyridium sp.* (UTEX 637) and *Porphyridium cruentum* (strain UTEX 161) were grown, to a density of 4 X 10⁶ cells/mL, as described in Example 1. For each strain, about 2 X 10⁶ cells/mL cells per well (∼500 uL) were transferred to 11 wells of a 24 well microtiter plate. These wells contained ATCC 1495 media supplemented with varying concentration of glycerol as follows: 0%, 0.1%, 0.25%, 0.5%, 0.75%, 1%, 2 %, 3%, 5%, 7% and 10%. Duplicate microtiter plates were shaken (a) under continuous illumination of approximately 2400 lux as measured by a VWR Traceable light meter (cat # 21800-014), and (b) in the absence of light. After 5 days, the effect of increasing concentrations of glycerol on the growth rate of these two species of *Porphyridium* in the light was monitored using a hemocytometer. The results are given in Figure 2 and indicate that in light, 0.25 to 0.75 percent glycerol supports the highest growth rate, with an apparent optimum concentration of 0.5%.

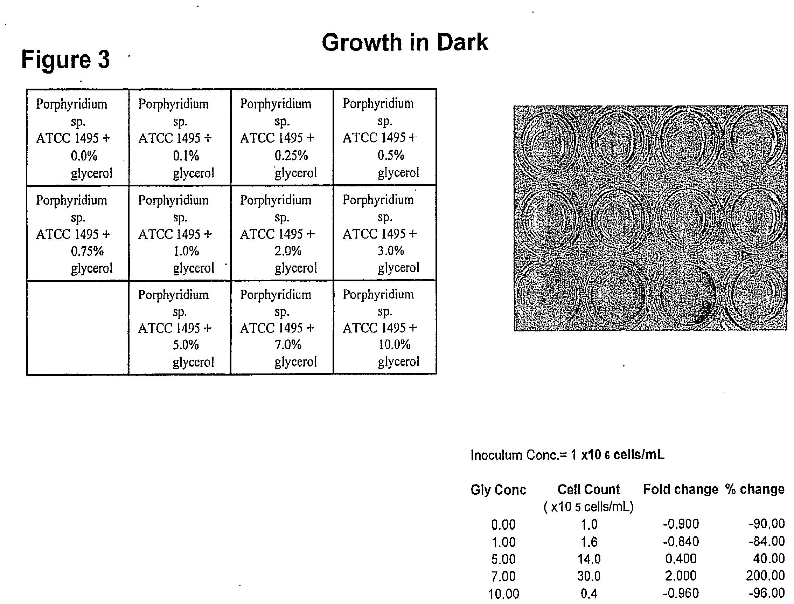

Cells in the dark were observed after about 3 weeks of growth. The results are given in Figure 3 and indicate that in complete darkness, 5.0 to 7.0% glycerol supports the highest growth rate, with an apparent optimum concentration of 7.0%.

### EXAMPLE 9

### Cosmeceutical Compositions

*Porphyridium sp.* (UTEX 637) was grown to a density of approximately 4 X 10⁶ cells/mL, as described in Example 1. Approximately 50 grams of wet pelleted, and washed cells were completely homogenized using approximately 20 minutes of sonication as described. The homogenized biomass was mixed with carriers including, water, butylene glycol, mineral oil, petrolatum, glycerin, cetyl alcohol, propylene glycol dicaprylate/dicaprate, PEG-40 stearate, C11-13 isoparaffin, glyceryl stearate, tri (PPG-3 myristyl ether) citrate, emulsifying wax, dimethicone, DMDM hydantoin, methylparaben, carbomer 940, ethylparaben, propylparaben, titanium dioxide, disodium EDTA, sodium hydroxide, butylparaben, and xanthan gum. The mixture was then further homogenized to form a composition suitable for topical administration. The composition was applied to human skin daily for a period of one week.

### EXAMPLE 10

### Antibiotic sensitivity

Approximately 4500 cells (300ul of 1.5x10⁵ cells per ml) of *Porphyridium sp.* and *Porphyridium cruentum* cultures in liquid ATCC 1495 ASW media were plated onto ATCC 1495 ASW agar plates (1.5% agar). The plates contained varying amounts of zeocin, sulfometuron, hygromycin and spectinomycin. The plates were put under constant artificial fluorescent light of approximately 480 lux. After 14 days, plates were checked for growth. Results were as follows:

**Zeocin**

| Conc.(ug/ml) | Growth |
|---|---|
| 0.0 | ++++ |
| 2.5 | + |
| 5.0 | - |
| 7.0 | - |

**Hygromycin**

| Conc.(ug/ml) | Growth |
|---|---|
| 0.0 | ++++ |
| 5.0 | ++++ |
| 10.0 | ++++ |
| 50.0 | ++++ |

**Specinomycin**

| Conc.(ug/ml) | Growth |
|---|---|
| 0.0 | ++++ |
| 100.0 | ++++ |
| 250.0 | ++++ |
| 750.0 | ++++ |

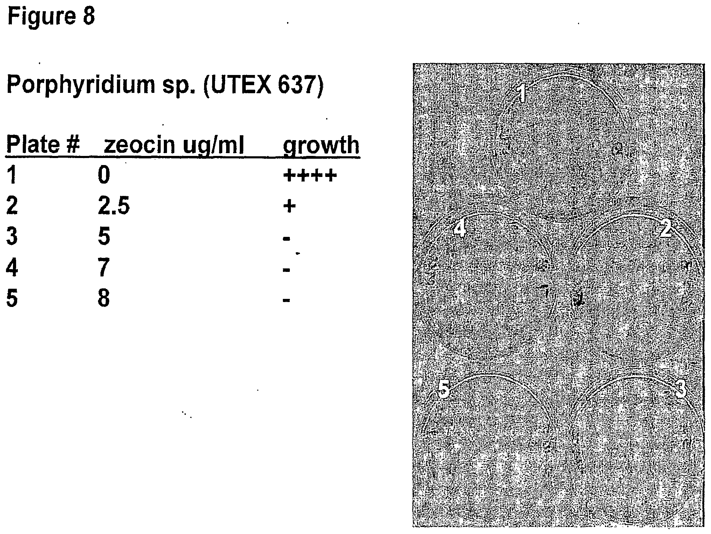

After the initial results above were obtained, a titration of zeocin was performed to more accurately determine growth levels of Porphyridium in the presence of zeocin. Porphyridium sp. cells were plated as described above. Results are shown in Figure 8.

### EXAMPLE 11

### Nutritional manipulation to generate novel polysaccharides

Cells expressing an endogenous monosaccharide transporter, containing a monosaccharide transporter and capable of importing glucose, are cultured in ATCC 1495 media in the light in the presence of 1.0% glucose for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing xylose, are cultured in ATCC 1495 media in the light in the presence of 1.0% xylose for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing galactose, are cultured in ATCC 1495 media in the light in the presence of 1.0% galactose for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing glucuronic acid, are cultured in ATCC 1495 media in the light in the presence of 1.0% glucuronic acid for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing glucose, are cultured in ATCC 1495 media in the dark in the presence of 1.0% glucose for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing xylose, are cultured in ATCC 1495 media in the dark in the presence of 1.0% xylose for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing galactose, are cultured in ATCC 1495 media in the dark in the presence of 1.0% galactose for approximately 12 days. Exopolysaccharide is purified as described in Example 2. Monosaccharide analysis is performed as described in Example 5.

Cells expressing an endogenous monosaccharide transporter, containing a monosaccharide transporter and capable of importing glucuronic acid, are cultured in ATCC 1495 media in the dark in the presence of 1.0% glucuronic acid for approximately 12 days. Exopolysaccharide is purified as described in Example 2. Monosaccharide analysis is performed as described in Example 5.

### EXAMPLE 12

### Increasing solvent-available polysaccharide

128mg of intact lyophilized *Porphyridium sp.* cells were ground with a mortar/pestle. The sample placed in the mortar pestle was ground for 5 minutes. 9.0 mg of the sample of the ground cells was placed in a micro centrifuge tube and suspended in 1000µL of dH2O. The sample was vortexed to suspend the cells. 3.

A second sample of 9.0mg of intact, lyophilized *Porphyridium sp.* cells was placed in a micro centrifuge tube and suspended in 1000µL of dH₂O. The sample was vortexed to suspend the cells.

The suspensions of both cells were diluted 1:10 and polysaccharide concentration of the diluted samples was measured by DMMB assay. Upon grinding, the suspension of ground cells resulted in an approximately 10-fold increase in the solvent-accesible polysaccharide as measured by DMMB assay over the same quantity of intact cells.

**Table 12**

| Sample Description | Read 1 (AU) | Read 2 (AU) | Avg. Abs (AU) | Conc. (µg/mL) |
|---|---|---|---|---|
| Blank | 0 | -0.004 | -0.002 | 0 |
| 50ng/µL Std., 10µL; 0.5µg | 0.03 | 0.028 | 0.029 | NA |
| 100ng/µL Std., 10µL; 1.0µg | 0.056 | 0.055 | 0.0555 | NA |
| Whole cell suspension | 0.009 | 0.004 | 0.0065 | 0.0102 |
| Ground cell suspension | 0.091 | 0.072 | 0.0815 | 0.128 |

Reduction in the particle size of the lyophilized biomass by homogenization in a mortar/pestle results in better suspension and increase in the solvent- available polysaccharide concentration of the cell suspension.

### EXAMPLE 13

### Decolorization of biomass

A *Porphyridium* culture (UTEX 637) was grown as described in Example 1 except that the culture media contained no Tris and 0.125g/L potassium nitrate, pH 7.6. The low nitrate culture was grown under approximately 130 µE m⁻² s⁻¹ until the color changed from red to yellow-brown, which took approximately 3 weeks. After waiting for a further three days, the yellow-brown cells were harvested by centrifugation, hereinafter referred to as "decolorized biomass" or "decolorized cell pellet". A deep red cell pellet generated from cells grown in normal ATCC 1495 ASW media was also generated as described in Example 1. The cell pellets were washed with 0.5L of distilled water, shell frozen in a dry ice acetone bath and lyophilized.

### Determination of Color

The decolorized cell pellet had a yellow-brown appearance (as opposed to cells grown in full ATCC 1495 ASW media which have a deep red appearance). The lyophilized decolorized cells and lyophilized red cells grown in full ATCC 1495 ASW media were treated identically. 100mg of lyophilized cell-pellets were resuspended in 4ml of 1M pH 7.6 Tris buffer by vigorous vortexing. The suspensions were sonicated on ice using a Misonix 3000 sonicator equipped with a micro-tip probe set at a power level of 6.5 for 90 seconds, pulsing for 30 seconds on 20 seconds off (3 cycles). The cell debris was pelleted by centrifugation in a microcentrifuge at 14,000 rpm for 5 minutes and the supernatant decanted. This procedure was repeated twice more with 1M pH 7.6 Tris buffer, and finally with 6M urea. No red color was observed in cell pellets or supernatant from the decolorized biomass after the second extraction or from the cells grown in full ATCC 1495 ASW media after the fourth extraction. The respective supernatants were combined and brought to a final volume of 75ml with distilled water.

Absorbance spectra of the supernatants from the decolorized pellet and the pellet from cells grown in full ATCC 1495 ASW media were recorded between 510 and 600nM with a Pharmacia Ultraspec III spectrophotometer and a 1cm path length cuvette.

The extinction coefficient for phycoerythrin is 5.26 ml.mg/cm at 545nm (see for example Gantt and Lipschultz, Phycobilisomes of Porphyridium cruentum; Biochemistry, 13, 2960, 1974). The concentration phycoerythrin was calculated from the optical density at 545nm (after subtracting the background due to scatter measured at 600nm) as 46mg/g dry-weight in cells grown in ATCC 1495 ASW media and 4.7mg/g in the decolorized cells.

### EXAMPLE 14

### Homogenization of biomass

After Porphyridium biomass grown as described in Example 1 was recovered by centrifugation and washed in deionizer water, nitrogen was bubbled through the paste for 30 minutes to displace dissolved oxygen and minimize subsequent oxidation. The paste was then passed through a model 110Y Microfluidics Microfluidizer® at 22,000 PSI with cooling, and the process repeated until cell breakage was at least 50% as determined by microscopic examination. Nitrogen was once again bubbled through the paste, which wais then lyophilized after shell freezing in dry ice ethanol. The dried cell mass was then ground to a fine powder with a Braun® kitchen homogenizer. This process can be performed with decolorized biomass generated as described herein. Optionally, preservative(s) and/or carrier(s) suitable for topical administration are added to the material, as well as fragrances and other materials used in the art of skin care product formulation.

### EXAMPLE 15

### Hyaluronidase inhibition

Biotinylated hyaluronic acid (bHA) was covalently attached to the wells of a 96-well plate. Samples containing hyaluronidase and various test materials were then added to the wells. The hyaluronidase degrades the bound hyaluronic acid, resulting in a decrease in the amount of biotin covalently linked to the well plate. At the end of the incubation period the reaction was stopped and the well plate was washed to remove the hyaluronidase. The remaining bHA was detected using an avidin bound peroxidase enzyme. When an appropriate substrate is added, the peroxidase enzyme generated a color signal in proportion to the amount of bHA. The color signal was measured spectrophotometrically, and was inversely proportional to the amount of hyaluronidase activity in the sample. Thus, materials that inhibited hyaluronidase resulted in a greater color signal, since more of the bHA remained intact. Also see Frost, G.,I., Stern, R. A Microtiter-Based Assay for Hyaluronidase Activity Not Requiring Specialized Reagents. Analytical Biochemistry 251, 263-269: 1997.

### Preparation of Test Material Extracts

Test Material A was supplied as a powder type material. For this study, 100 mg of this material was combined with either 5 ml of ethanol or 5 ml of ultrapure water in 15 ml centrifuge tubes. After combining, the mixtures were vortexed, then placed onto a rocking platform for approximately 30 minutes at room temperature, and then centrifuged at 1,000 RPM for 5 minutes. The supernatants were then used at the final concentrations listed in the results section. Test Material B was supplied as a thick, viscous solutions (3%).

### Anti-Hyaluronidase Assay: Immobilization of bHA onto 96-well plates

A solution of sulfo-NHS (0.184 mg/ml) and bHA (0.2 mg/ml) was prepared in distilled water. 50 µl of this solution was then added to the wells of a 96-well Covalink-NH plate. A solution of EDC (0.123 mg/ml) was then prepared in distilled water and 50 µl of this solution was added to each well (which resulted in a final concentration of 10 µg/well bHA and 6.15 µg/well of EDC). The well plate was then incubated overnight at 4±2°C or for 2 hours at room temperature. After the incubation the plate was washed three times with PBS containing 2 M NaCl and 50 mM MgSO₄.

### Anti-Hyaluronidase Assay

Prior to the assay, the well plate was equilibrated with assay buffer (0.1 M formate [pH 4.5], 0.1 M NaCl, 1% Triton X-100, 5 mM saccharolactone). The test materials were prepared in assay buffer at 2x their final concentration (heparin was used as a positive control, 1 mg/ml final concentration). After removing the assay buffer from the well plate used for equilibration, 50 µl of each of the prepared test materials was added to three wells on the well plate, followed by the addition of 50 µl of assay buffer containing hyaluronidase will be added to each well (0.1 mg/ml). Three additional wells were treated with 100 µl of assay buffer alone (without test materials and without hyaluronidase) and served as an index of zero hyaluronidase activity. After the addition of the test materials and enzyme, the plate was incubated for 30 minutes at room temperature. At the end of the incubation period, 200 µl of 6 M guanidine-HCl was added to each well to terminate the reaction. The plate was then washed three times with PBS containing 2 M NaCl, 50 mM MgSO₄ and 0.05% Tween 20.

During the 30 minute incubation, an avidin/biotin-peroxidase complex was prepared in 10.5 ml of PBS containing 0.1% Tween 20 using an ABC kit. This mixture was incubated for at least 30 minutes prior to use. After the plate was washed, 100 µl of the avidin/biotin-peroxidase solution was added to each well and the plate was incubated for another 30 minutes at room temperature. The plate was washed three times with PBS containing 2 M NaCl, 50 mM MgSO₄ and 0.05% Tween 20. After the final wash, 100 µl of substrate solution (one 10 mg tablet of OPD in 10 ml of 0.1 M citrate-PO₄ buffer supplemented with 7.5 µl of 30% H₂O₂) was added to each well. The plate was incubated in the dark for 10-20 minutes and then read at 460 nm using a plate reader. The substrate solution was also added to three wells that were not treated with test materials or the avidin/biotin-peroxidase solution and were used as a blank for the absorbance measurements.

**Table 13**

| Treatment | Percent Inhibition |
|---|---|
| 10% A Water Extract | 86 |
| 5% MATERIAL A Water Extract | 67 |
| 1% MATERIAL A Water Extract | 29 |
| 1.5% MATERIAL B | 93 |
| 0.5% MATERIAL B | 81 |
| 0.1% MATERIAL B | 70 |
| 0.1% Heparin | 74 |
| Negative Control | 0 |

| | |
|---|---|
| Test Material Identification: | MATERIAL A: Porphyridium sp. biomass homogenized (Quadro F10); cells grown as described in Example 1 |
| Physical Description: | Red/Purple powder |
| Concentrations Tested: | 10%, 5%, 1% (Extracted in either ethanol or water) |
| | |
| Test Material Identification: | 3% MATERIAL B: Exopolysaccahride from Porphyridium sp. purified as described in Example 2 |
| Physical Description: | Light tan, viscous liquid |
| Concentrations Tested: | 1.5%, 1%, 0.5%, 0.1% |

### EXAMPLE 16

### Sulfated derivative polysaccharides

Porphyridium cruentum and Porphyridium sp. were grown in artificial seawater media essentially as described in Example 1 except that the amount of MgSO₄ was varied. Porphyridium sp.cells were grown in 17mM MgSO₄. Porphyridium cruentum was grown in 120mM, 600mM, 750 mM, 1M, and 2M MgSO₄. Cell division occurred at all concentrations. Polysaccharide was purified essentially as described in Example 2 from the 120 and 600mM cultures, to the point where all soluble protein and small molecules were removed . Sulfur content was analyzed according to US EPA SW846, Method 6010B, Inductively Coupled Plasma-Atomic Emission Spectrometry. The polysaccharide purified from the 17, 120 and 600mM cultures contained 3.57, 4.23 and 5.57% sulfur, respectively. It was observed that polysaccharides with higher percent sulfate by weight exhibited stronger gelling properties than polysaccharides with a lower percent sulfate by weight when the two preparations were generated at the same polysaccharide concentration. For example, at a 1% concentration the polysaccharide containing 5.57% sulfur held its shape and moved as a gelatinous unit whereas the polysaccharide with a 3.57 percent sulfur by weight at 1% flowed as a viscous liquid. The increased gelling properties provide added benefits for skin care compositions as they can form gels in products at lower concentrations.

### EXAMPLE 17

### Monosaccharide analysis

Porphyridium cruentum was cultured as described in Example 1 except that (a) the amount of KNO₃ per liter of media was approximately 150g/L; (b) the media contained no Tris; and (c) the media contained approximately 0.14g/L KH₂PO₄. The cells lost all detectable red coloration after approximately three weeks of growth, and turned to a yellow shade. Exopolysaccharide was purified essentially as described in Example 2. Monosaccharide analysis was performed essentially as described in Example 5. Monosaccharide composition of the exopolysaccharide was as follows:

| Glycosyl residue | Mass (µg) | Mole % |
|---|---|---|
| Arabinose (Ara) | n.d. | n.d. |
| Rhamnose (Rha) | n.d. | n.d. |
| Fucose (Fuc) | n.d. | n.d. |
| Xylose (Xyl) | 137.8 | 41.5 |
| Glucuronic Acid (GlcA) | 18.7 | 4.3 |
| Mannose (Man) | trace | trace |
| Galactose (Gal) | 133.2 | 33.4 |
| Glucose (Glc) | 83.2 | 20.8 |
| Unknown Sugar | n.d. | n.d. |
| N-acetyl glucosamine (GlcNAc) | n.d. | n.d. |
| N-acetyl neuraminic acid (NANA) | n.d. | n.d. |
| | ∑= 372.9 | |

The exopolysaccharide contained significantly different and more advantageous monosaccharide composition from those grown under standard conditions shown in Example 5. The composition is a preferred composition for skin care products. For example, the ratio of glucose to xylose is higher in the polysaccharide from bleached cells.

### EXAMPLE 18

### Polysaccharide bead production

Two solutions of polysaccharide from Porphyridium cruentum (0.5% w/v) in water, prepared as described in Example 2 except flushed with distilled water rather than 1mM Tris, were dried under air flow at 60°C until converted to a solid translucent film. One sample was isolated from Porphyridium cruentum grown in ATCC 1495 media, while the other was from Porphyridium cruentum grown in ATCC 1495 media with the exception that the KNO₃ was approximately 0.15g/L (labeled as "1495 low N"). The resulting films were then heated under vacuum (≥25 in Hg) at approximately 150°C overnight to form dried polysaccharide. The dried polysaccharide was then ground in a pestle and mortar. A third polysaccharide sample (0.5% w/v) in water, isolated from Porphyridium cruentum grown in ATCC 1495 media, prepared as described in Example 2 except flushed with distilled water rather than 1mM Tris, was lyophilized and not ground.

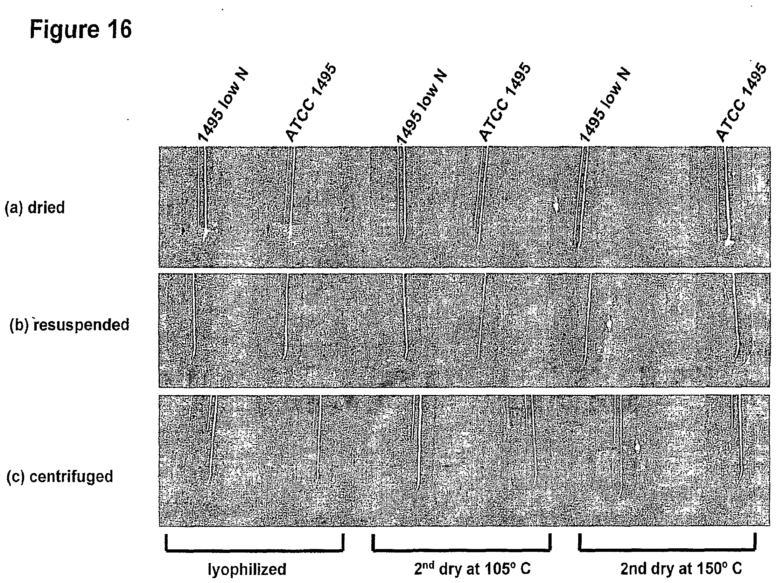

100mg of dried, ground polysaccharide from each sample was resuspended in 2.5ml of water. 100mg of lyophilized polysaccharide was also resuspended in 2.5ml of water. The suspensions were centrifuged at 4,400 rpm in a Forma Scientific Centra-GP8R refrigerated centrifuge. The parameters used for centrifugation were 4200rpm, rotor# 218, 20 minute spin. As shown in Figure 16(c), there was a swollen, insoluble gel layer and a clear supernatant in the samples that were dried at 150°C but not in the samples that were lyophilized or dried at 105°C. The polysaccharide dried at 150°C did not go into solution, but rather stayed insoluble despite significant swelling in size.

The percentage insoluble polysaccharide in the 150°C dried samples was measured by separating the insoluble and supernatant fractions, lyophilizing the separated fractions, and weighing the dried residual polysaccharide from each fraction. The percentage insoluble polysaccharide was then calculated as a percentage of the total polysaccharide from both fractions. While the polysaccharide from the samples that were originally dried by lyophilization and drying at 105°C were 100% soluble, the low N and ATCC 1495 polysaccharide samples dried at 150°C were 75% and 86% insoluble, respectively. Independent experiments demonstrated that material dried at 125°C was completely soluble.

### EXAMPLE 19

### Polysaccharide bead properties

Insoluble polysaccharide preparations, prepared essentially as described in Example 18, were tested for (a) the ability to swell in size over time and (b) the ability to bind soluble polysaccharide and remove it from solution.

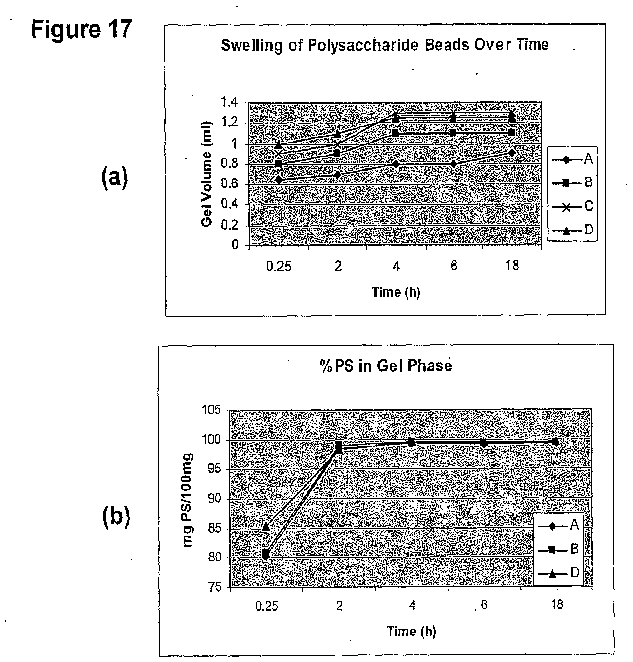

Samples were resuspended in distilled water as described in Example 18. Samples were centrifuged as described in Example 18 at various time points. The volume of the insoluble gel layer was measured, followed by resuspension of the material and incubation at room temperature until the next centrifugation point. Results are shown in Figure 17. The results demonstrate that the dried polysaccharide beads continue to swell in volume for at least 4 hours and as long as 18 hours after initial exposure to water.

The concentration of polysaccharide in solution in the supernatant was measured at each time point using the DMMB assay as described in Example 3. Results are shown in Figure 17(b). The results demonstrate that the polysaccharide beads bind soluble polysaccharide and remove it from solution for at least 4 hours after initial exposure to water. The swelling and binding of soluble polysaccharide is a useful property for topical application to human skin and is stimulated by transepidermal water loss.

### EXAMPLE 20

### Transformation of Porphyridium and Genotyping

The Porphyridum glycoprotein promoter, SEQ ID NO:21, was cloned in operable linkage with a zeocin resistance ble cDNA with small amounts of flanking sequence (SEQ ID NO:37), with the far 5' region of the glycoprotein promoter directly adjacent to the pBlusecript SacI site and the far 3' region of the CMV 3'UTR (SEQ ID NO:32) adjacent to the pBlusecript KpnI site. The CMV 3'UTR was also in operable linkage with the ble cDNA. The plasmid was linearized by KpnI, which does cut in the promoter, ble cDNA, or 3'UTR, prior to transformation.

The biolistic particle delivery system PDS 1000/ He (Bio-Rad, USA) was used for transformation. *Porphyridium sp.* culture was grown to logarithmic phase (∼2 x 10⁶ cells/mL) in liquid ATCC 1495 media under continuous light (approximately 75 umol/photons/m²). Cells from this culture were harvested at 4,000 rpm at room temperature.

The cell pellet was washed twice with sterile distilled water. Cells were resuspended in fresh ATCC 1495 media to the same cell density i.e.. ∼2 x 10⁶ cells/mL and incubated in the dark for 16 hours. The dark adapted cells were then harvested at 4000 rpm at room temperature, resuspended in fresh ATCC 1495 media to a density of ∼2 x 10⁸ cells/mL. Approximately 1 x 10⁸ cells were transferred to each ATCC 1495 agarose plate. Filter sterilized DNA from the plasmids was coated onto 550 nm gold particles (catalog number S04e, Seashell Technology, USA) according to the manufacturer's protocol. For each of the particle bombardments, 1 ug of plasmid DNA was used. The negative controls were bombarded in identical fashion with gold particles coated with a plasmid containing the Porphyridum glycoprotein promoter, SEQ ID NO:21, and the CMV 3'UTR, (SEQ ID NO:32), with no zeocin resistance gene. Each of the particle bombardments were performed using 1350 psi rupture disks, at bombardment distance of 9 cm, and under 28 in. Hg vacuum. The bombarded cells were scraped off the plates, and transferred to 100 ml of fresh ATCC 1495 media, and shaken under continuous light (approximately 75 umol/ m²) for 3 days. Following recovery, the cells were harvested at 4,000 rpm at room temperature, and plated onto ATCC 1495 plates supplemented with 30 ug/mL Zeocin (Invitrogen, Carlsbad, CA, USA) at a cell density of 1 x 10⁷ cells/plate. These plates were incubated under light (approximately 25 umol/ m²) for 4-5 weeks. Zeocin resistant colonies growing on these plates were scored as transformants and transferred onto fresh ATCC 1495 plates supplemented with 30 ug/mL Zeocin (Invitrogen, Carlsbad, CA, USA) for growth and analysis.

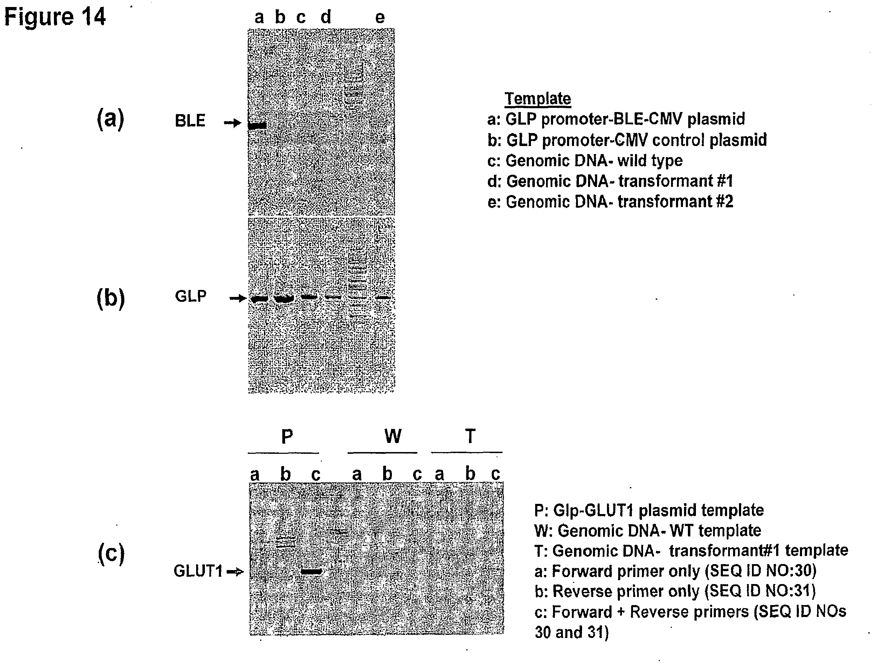

Zeocin resistant colonies appeared after 2-3 weeks. Genotyping with primers specific to the zeocin resistance gene was performed on genomic DNA isolated from zeocin resistant colonies. Results from genotyping of one strain (referred to herein and labeled as "transformant #2" in Figure 14) indicated that the zeocin resistance gene was present. A band of the correct size was amplified. Results are shown in Figure 14 and discussed in more detail in Example 20.

### EXAMPLE 21

### Transformation of Porphyridium, Genotyping, and Southern Blot Analysis

The zeocin resistance plasmid described in Example 20 and a second plasmid that was identical with the exception that it contained a cDNA for a human GLUT1 glucose transporter (SEQ ID NO:25) instead of the ble cDNA were combined in a co-transformation experiment carried out essentially as described in Example 20 except that both the zeocin resistance and GLUT1 plasmids were both adhered to the gold beads. A zeocin resistant colony (referred to herein as transformant#1) was selected for further analysis. Genomic DNA was extracted from wild type Porphyridium sp. and transformant#1.

Genotyping was performed on genomic DNA extracted from wild type, transformant#1, and transformant#2 DNA with plasmid DNA used as a template positive control and water in place of DNA as a template negative control. A segment of the Porphyridium glycoprotein (GLP) gene promoter was used as a target positive control. The following primer sets were used for the genotyping PCR: Ble- FWD (SEQ ID NO: 26) and Ble-REV (SEQ ID NO: 27), GLP-FWD (SEQ ID NO: 28) and (SEQ ID NO: 29), GLUT1-FWD (SEQ ID NO: 30) and GLUT1-REV (SEQ ID NO: 31). The PCR profile used was as follows: 94°C denaturation for 5 min; 35 cycles of 94°C for 30 sec, 51°C or 60°C (51°C for glycoprotein gene & GLUT1 and 60°C for ble) for 30 sec, 72°C for 2 min; 72°C for 5 min. Results are shown in Figure 14. Figure 14(a) demonstrates that the ble gene was present in both transformants, as the expected 300bp product was generated. Figure 14(b) demonstrates that the genomic DNA extraction and amplification was working, as the expected 948bp glycoprotein promoter fragment was generated. Figure 14(c) demonstrates that the GLUT1 gene was present transformant #1, as the expected 325bp product was generated. DNA ladder was from BioNexus, Inc., All Purpose Hi-Lo DNA Marker, Catalog No: BN2050.

Specific bands can be amplified from residual plasmid DNA adhered to the outside of cells on transformation plates. Additionally, plasmids that have not been linearized can be maintained as episomes for a period of time before being degraded and can serve as template during PCR despite not having been integrated into a chromosome of a host organism. In both cases, microalgal strains may genotype positive despite the absence of stable chromosomal integration of the vector. Antibiotic resistant strains are known to arise due to mutagenesis caused by chromosomal damage from biolistic particles, electroporation conditions, and random genetic variation that is known to occur in microbial organisms. Southern blot analysis was performed to conclusively confirm the integration of the GLUT1 construct into the genome of transformant #1.

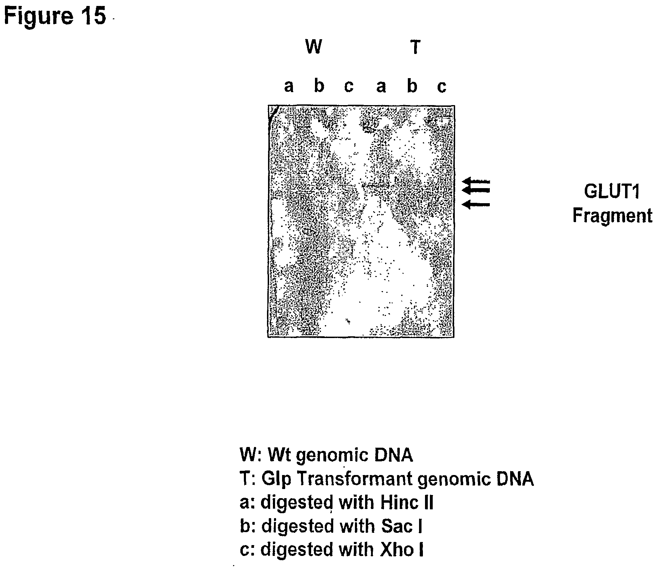

Southern blot analysis was performed on transformant #1. 20 µg genomic DNA from wild type and transformant#1 were individually digested with Hinc II, Sac I , Xho I and separated on a 1% agarose gel. DNA was transferred onto Nylon membrane (Hybond N+, Amersham Biosciences). A 1495 bp fragment containing the entire coding region of the GLUT1 gene was used as a probe. DIG labeled probes were generated for each probe fragment using the DIG High Prime DNA labeling and detection Kit according to the manufacturers instructions (Roche). The hybridizing bands were detected using the colorimetric detection reagents provided by the manufacturer. Figure 15 demonstrates that the GLUT1 plasmid was stably integrated into the genome of transformant#1 while no detectable signal arose from wild type genomic DNA. As would be expected for a plasmid integrating into a chromosome of an organism, the specific band was in a different position for each different restriction enzyme used to digest the genomic DNA. This data conclusively demonstrates a species of the genus Porphyridium containing an exogenous gene encoding a carbohydrate transporter integrated into a chromosome of the organism. In this embodiment the carbohydrate transporter gene is in operable linkage with a promoter endogenous to a species of the genus Porphyridium. In some other embodiments embodiment the carbohydrate transporter gene is in operable linkage with a promoter endogenous to a Rhodophyte.

### EXAMPLE 22

### Production of materials from microalgae

Materials from Porphyridium were generated to assess their utility as components of skin care compositions. Materials BM, LS PS, and HS PS were generated and tested as referenced in this and following Examples. BM is Porphyridium sp. biomass, grown essentially as described in Example 1, which was washed once with ditilled water, run twice through a microfluidizer (Microfluidics Inc, Newton, MA., U.S.A.), and lyophilized as follows: using a Microfluidics Microfluidizer® (model # 110Y) pressurized with nitrogen, washed biomass material was pumped through an 87um orifice at 22,000 psi twice. The product was kept on ice at all times. Nitrogen gas was bubbled through the final product while mixing for 10min. Snap freeze for storage or shell freeze and lyophilize. The BM material was then treated as described in each example.

LS PS was polysaccharide from Porphyridium sp. HS PS was polysaccharide from Porphyridium cruentum. Both were purified essentially as described in Example 2. The cells grown for preparation of LS PS and HS PS were grown essentially as described in Example 1 except that the sulfate in the media was 17mM for the LS PS and 600mM for the HS PS (also described in Example 16).

### EXAMPLE 23

### UVB protective properties of materials from microalgae

TT dimer-UV exposure assay: The testing system used for this assay was the MatTek EpiDerm®, a skin model that consists of normal human-derived epidermal keratinocytes cultured to form a multilayered, highly differentiated model of the human epidermis. For this study, the tissues were treated topically overnight with either test materials, 1 mM Trolox (positive control), or left untreated (negative control). On the following day, the tissues were exposed to UVB (300 mJ/cm²). Following the exposures the DNA was extracted from the EpiDerm tissues and assayed for thymine dimer content. Samples of the DNA were immobilized on a solid membrane support and incubated with an antibody that recognizes thymidine dimers in double stranded DNA. The primary antibody was detected using a secondary antibody conjugated to an alkaline phosphatase enzyme followed by the addition of a substrate that the alkaline phosphatase enzyme uses to generate a chemiluminescent signal. The light generated by this reaction was captured using film with the intensity of the light signal being proportional to the amount of the thymine dimers present in the sample.

Test material BM was Porphyridium sp. biomass that had been homogenized with a Microfluidizer® twice and then lyophilized. For this study, 100 mg of this material was combined with 5 ml of ultrapure water in 15 ml centrifuge tubes. After combining, the mixture was vortexed, then placed onto a rocking platform for approximately 30 minutes at room temperature, and then centrifuged at 1,000 RPM for 5 minutes. The supernatant was then used at a final concentration of 10% and 5%. The two remaining test materials, LS PS and HS PS were supplied as thick, viscous solutions (3g/100mL). LS PS53 was tested at the final concentrations of 1.5%, 0.5%, and 0.1%, while PS133 was tested at the final concentration of 0.1 %.

Prior to use, the MatTek EpiDerm® tissues were removed from the agarose-shipping tray and placed into a 6-well plate containing 0.9 ml of cuture medium (MatTek EPI-100 culture media) according to the manufacturer. The tissues were allowed to incubate for at least 1 hour at 37± 2°C and 5±1% CO₂. After this initial incubation, the culture media was replaced with fresh, pre-warmed EPI-100 media and 100 µl of test material, 1 mM Trolox or PBS (negative control) was applied. The tissues were then incubated overnight at 37± 2°C and 5±1% CO₂. On the following day, the tissues were exposed to 300 mJ/cm² of UVB energy at 300nm. After the UVB exposure the tissues were collected and DNA was immediately extracted.

DNA extraction was performed using the Qiagen, DNEasy Kit. Single tissues were placed into 1.5 ml centrifuge tubes containing 180 µl of Lysis Buffer One. After mincing the tissues with a pair of fine tipped scissors, 20 µl of Proteinase K was added to the tube and the tube was incubated overnight at 55 ± 2°C with occasional mixing/vortexing. After the Proteinase K digestion, 200 µl of Lysis Buffer Two was added to the tube and the tube was incubated at 70 ± 2°C for approximately 10 minutes. Next, the DNA was precipitated by the addition of 200 µl of 100% ethanol. The precipitated DNA was washed to remove cellular debris by applying the mixture to a DNEasy Spin Column and centrifuging the sample in a 2 ml collection tube at 8,000 RPM for 1 minute. The flow through and collection tube was discarded, and 500 µl of Wash Buffer One was added to the spin column and the column was placed into a new collection tube and centrifuged at 8,000 RPM for 1 minute. The flow through and collection tube was again discarded, and 500 µl of Wash Buffer Two was added to the spin column and the column was placed into a new collection tube and centrifuged at 14,000 RPM for 3 minutes. The spin column was then placed into a new 1.5 ml centrifuge tube and 110 µl of Elution Buffer was added to the column. The column was incubated for 1 minute at room temperature and then centrifuged at 8,000 RPM for 1 minute.

Extracted DNA was quantified via a fluorometric assay. A 10 µl aliquot of the DNA sample was mixed with 1.0 ml of Assay Buffer (2 M NaCl, 50 mM sodium phosphate, pH 7.4) and 100 µl of this diluted sample was transferred to a well in a black 96-well plate. A series of DNA standards (0, 100, 200, 300, 400 and 500 ng) was also transferred to wells in a 96-well plate (in duplicate). Finally, 100 µl of dilute Hoechst 33258 dye (0.006 mg/ml in Assay Buffer) was added to each well and the fluorescence intensity of each well was determined using an excitation wavelength of 355 nm and an emission wavelength of 485 nm.

Aliquots of DNA (400 ng in 2x SSC [20x stock SSC: 3 M NaCl, 0.3 M sodium citrate, pH 7.0]) was loaded onto a membrane via microfiltration blotting. After loading, the membrane was washed once in 2x SSC and then baked for 30 minutes at 80°C to crosslink the DNA to the membrane. The membrane was then incubated for 1 hour in blocking solution (TBS [20 mM Tris, pH 7.5, 500 mM NaCl] supplemented with 5% non-fat milk protein, 0.2% polyvinylpyrolidone, and 0.2% ficol), and then briefly washed twice in TBS-T (TBS with 0.1% non-fat milk protein and 0.1% Tween 20). The membrane was then incubated overnight (4°C) with an antibody that recognizes thymine dimers diluted in TBS-T. On the following day, the membrane was washed 3 times with TBS-T (20 minutes per wash) and then incubated with an AP-conjugated secondary antibody for 1-2 hours at room temperature. After this incubation period the membrane was washed as described above. Near the end of the final wash the chemiluminescence reagent was prepared. At the end of the last wash, all of the TBS-T was drained from the membrane and the chemiluminescent substrate was applied to the membrane and it was allowed to sit for approximately 1 minute. The membrane was then wrapped in Saran foil and taped inside of a film cassette. In a dark room a piece of film was inserted into the cassette and exposed for various amounts of time, starting with 10 seconds and increasing or decreasing in appropriate increments, until obtaining the necessary exposures. After exposure, the films were analyzed via densitometry. To quantify the amount of DNA present, a standard curve was generated using known concentrations of DNA and their respective fluorescence intensity (measured in RFUs or relative fluorescence units). A regression analysis was performed to establish the line that best fits these data points. The RFUs for each unknown sample were used to estimate the amount DNA. Mean densitometric values, expressed in optical density units, were determined for each treatment.

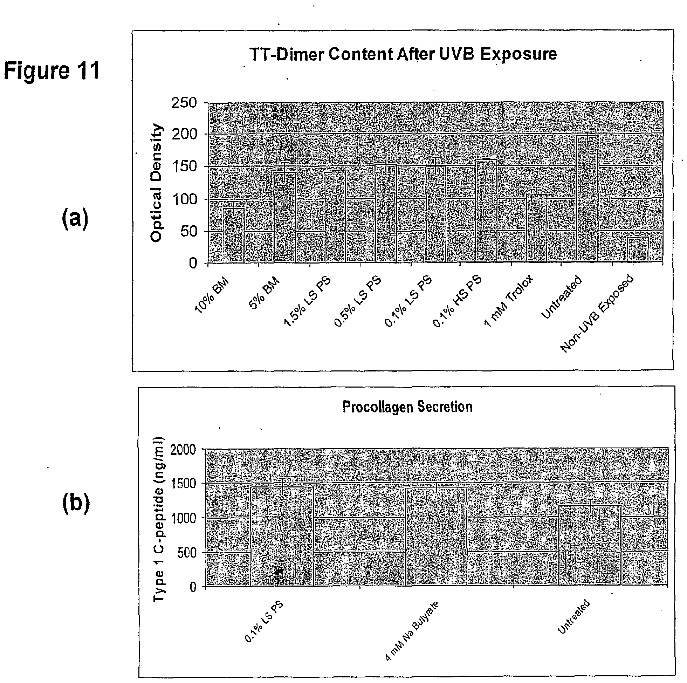

The results for the thymidine dimer assay are presented in Table 14 and Figure 11(a). The values are expressed as optical density units. The values are presented as mean values ± standard deviation.

**Table 14. Thymidine Dimer Assay**

| Treatment | Optical Density | | |
|---|---|---|---|
| 10% BM | 85 | ± | 11 |
| 5% BM | 140 | ± | 15 |
| 1.5% LS PS | 140 | ± | 3 |
| 0.5% LS PS | 152 | ± | 16 |
| 0.1% LS PS | 150 | ± | 13 |
| 0.1% HS PS | 160 | ± | 2 |
| 1 mM Trolox | 107 | ± | 1 |
| Untreated | 196 | ± | 6 |
| Non-UVB Exposed | 39 | ± | 4 |

The results of this study indicate that all three of the test materials significantly reduced the amount of TT dimer formation.

### EXAMPLE 24

### Procollagen synthesis stimulating properties of materials from microalgae

A fibroblast cell culture model was used to assess the ability of the polysaccharide from Porphyridium to exert an effect on procollagen synthesis. Fibroblasts are the main source of the extracelluar matrix peptides, including the structural protein collagen. Procollagen is a large peptide synthesized by fibroblasts in the dermal layer of the skin and is the precursor for collagen. As the peptide is processed to form a mature collagen protein, the propeptide portion is cleaved off (type I C-peptide). Both the mature collagen protein and the type I C-peptide fragment are then released into the extracellular environment. As collagen is synthesized the type I C-peptide fragment accumulates into the tissue culture medium. Since there is a 1:1 stoichiometric ratio between the two parts of the procollagen peptide, assaying for type I C-peptide reflects the amount of collagen synthesized. Type 1 C-peptide was assayed via an ELISA based method. Test material LS PS was supplied as a thick, viscous solution (3g/100mL). This material was used at a 0.1% final concentration. The anti-collagen antibody used was Procollagen Type I: N-18 antibody, catalog number sc-8785, Santa Cruz Biotechnology. The secondary antibody used was anti-goat conjugated with alkaline phosphatase catalog number sc-2355, Santa Cruz Biotechnology.

Fibroblasts were seeded into the individual wells of a 12 well plate in 1.0 ml of Fibroblast Growth Media (FGM: DMEM supplemented with 2% FBS, 5 ng/ml human recombinant growth factor, 5 ug/ml insulin, 50 ug/ml gentamicin, and 0.5 ug/ml Amphotericin-B) and incubated overnight at 37±2°C and 5±1% CO₂. On the following day the media was removed via aspiration to eliminate any non-adherent cells and replaced with 1.0 ml of fresh FGM. The cells were grown until confluent with a media change every 48 to 72 hours. Upon reaching confluency the cells were treated for 24 hours with DMEM supplemented with 1.5% FBS to wash out any effects from the growth factors included in the normal culture media. After this 24-hour wash out period the cells were treated with 0.1% LS PS dissolved in FGM with 1.5% FBS. 4 mM sodium butyrate was used as a positive control. Untreated cells (negative controls) received FGM with 1.5% FBS. The cells were incubated for 48 hours and at the end of the incubation period cell culture medium was collected and either stored frozen (-75°C) or assayed immediately. Materials were tested in triplicate.

A series of type I C-peptide standards was prepared ranging from 0 ng/ml to 640 ng/ml. Next, an ELISA microplate was prepared by removing any unneeded strips from the plate frame followed by the addition of 100 µl of peroxidase-labeled anti procollagen type I-C peptide antibody to each well used in the assay. Twenty (20) µl of either sample (collected tissue culture media) or standard was then added to appropriate wells and the microplate was covered and allowed to incubate for 3 ± 0.25 hours at 37°C. After the incubation the wells were aspirated and washed three times with 400 µl of wash buffer. After the last wash was removed 100 µl of peroxidase substrate solution (hydrogen peroxide + tetramethylbenzidine as a chromagen) was added to each well and the plate was incubated for 15 ± 5 minutes at room temperature. After the incubation 100 µl of stop solution (1 N sulfuric acid) was added to each well and the plate was read using a microplate reader at 450 nm.

To quantify the amount of procollagen present, a standard curve was generated using known concentrations of procollagen. A regression analysis was be performed to establish the line that best fit the data points. Absorbance values for the test and positive control samples was used to determine the amount of procollagen present. The values are presented in mean ng/ml ± the standard deviation of the mean. 0.1% LS PS increased procollagen synthesis compared to untreated cells, as seen below in Table 15 and in Figure 11(b).

**Table 15. Procollagen Assay**

| Treatment | Type I C-Peptide ng/ml | | |
|---|---|---|---|
| 0.1% LS PS | 1448 | ± | 113 |
| 4 mM Na Butyrate | 1425 | ± | 81 |
| Untreated | 1151 | ± | 96 |

### EXAMPLE 24

### Elastin synthesis stimulating properties of materials from microalgae

A fibroblast cell culture model was used to assess elastin synthesis. Elastin is the main component of a network of elastic fibers that give tissues their ability to recoil after a transient stretch. This protein is released by fibroblasts (soluble elastin) into the extracellular space where it is then cross-linked to other elastin proteins to form an extensive network of fibers and sheets (insoluble elastin). Soluble elastin can be readily measured from cell culture medium via a competitive ELISA based method.

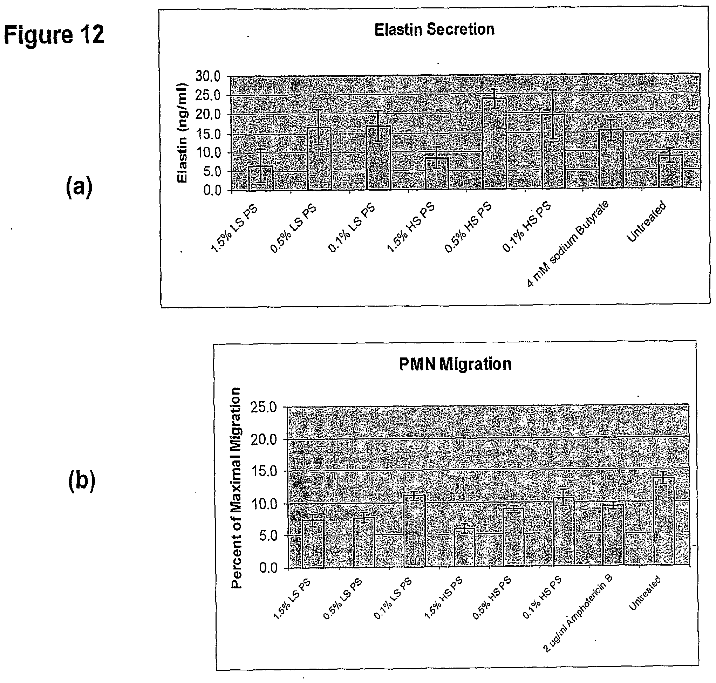

Test material BM was supplied as a powder type material. 100 mg of BM was combined with 5 ml of ultrapure water in 15 ml centrifuge tubes. After combining, the mixtures were vortexed, then placed onto a rocking platform for approximately 30 minutes at room temperature, and then centrifuged at 1,000 RPM for 5 minutes. The supernatant for each mixture was then used at final concentrations indicated in Figure 12(a). LS PS and HS PS was supplied as a thick, viscous solution (3g/100mL). This material was used at final concentrations indicated in Figure 12(a).

Fibroblasts were seeded into the individual wells of a 12 well plate in 1.0 ml of Fibroblast Growth Media (FGM) and incubated overnight at 37+ 2°C and 5±1 % CO₂. On the following day the media was removed via aspiration to eliminate any non-adherent cells and replaced with 1.0 ml of fresh FGM. The cells were grown until confluent, with a media change every 48 to 72 hours. Upon reaching confluency the cells were treated for 24 hours with DMEM supplemented with 1.5% FBS to wash out any effects from the growth factors included in the normal culture media. After this 24-hour wash out period the cells were treated with the test materials at the specified concentrations dissolved in FGM with 1.5% FBS. 4 mM sodium butyrate was used as a positive control for elastin synthesis. Untreated cells (negative controls) received FGM with 1.5% FBS. The cells were incubated for 48 hours and at the end of the incubation period cell culture medium was collected and either stored frozen (-75°C) or assayed immediately. Materials were tested in triplicate.

Soluble α-elastin was dissolved in 0.1 M sodium carbonate (pH 9.0) at a concentration of 1.25 µg/ml. 150 µl of this solution was then applied to the wells of a 96-well maxisorp Nunc plate and the plate was incubated overnight at 4°C. On the following day the wells were saturated with PBS containing 0.25% BSA and 0.05% Tween 20. The plate was then incubated with this blocking solution for 1 hour at 37°C and then washed two times with PBS containing 0.05% Tween 20.

A set of α-elastin standards was generated ranging from 0 to 100 ng/ml. 180 µl of either standard or sample was then transferred to a 650 µl microcentrifuge tube. The anti-elastin antibody was the C-21 antibody, catalog number sc-17581, from Santa Cruz Biotechnology. The secondary antibody used was anti-goat conjugated with alkaline phosphatase catalog number sc-2355, Santa Cruz Biotechnology. An anti-elastin antibody solution was prepared (the antibody was diluted 1:100 in PBS containing 0.25% BSA and 0.05% Tween 20) and 20 µl of the solution was added to the tube. The tubes were then incubated overnight at 4 ± 2°C. On the following day, 150 µl was transferred from each tube to the 96-well elastin ELISA plate, and the plate was incubated for 1 hour at room temperature. The plate was then washed 3 times with PBS containing 0.05% Tween 20. After washing, 200 µl of a solution containing a peroxidase linked secondary antibody diluted in PBS containing 0.25% BSA and 0.05% Tween 20 was added, and the plate was incubated for 1 hour at room temperature. After washing the plate three times as described above, 200 µl of a substrate solution was added and the plate was incubated for 10 to 30 minutes in the dark at room temperature. After this final incubation the plate was read at 460 nm using a plate reader.

To quantify the amount of elastin present, a standard curve was generated using known concentrations of elastin. A regression analysis was performed to establish the line that best fit these data points. Absorbance values for the test and control samples was used to determine the amount of elastin present in each sample. Both the 0.5% and 0.1% concentrations of LS PS and HS PS induced an increase in elastin synthesis.

### EXAMPLE 25

### Antiinflammatory properties of materials from microalgae

Microalgal polysaccharide materials were tested for their ability to influence the migration of polymorphonucelar (PMN) leukocytes in response to chemotractant substances. Leukocyte migration is essential for leukocyte accumulation at sites of inflammation. During the inflammatory response, leukocytes migrate towards the source of a chemotractant substance in a process called chemotaxis. In vitro methods to study chemotaxis often use a membrane based approach, where a chemoattractant is placed on one side of a membrane and PMN leukocytes are placed on the other. Chemotaxis is then measured by quantifying the number of leukocytes which then migrate through the filter towards the chemotractant substance.

For this study, human PMN leukocytes were isolated via density centrifugation from freshly drawn blood. The PMN cells were loaded with a Calcein AM, a fluorescent dye. While the PMN cells were being labeled, the bottom chamber of a chemotaxis chamber was filled with PBS containing FMLP, a chemotractant substance. A membrane filter was placed over the bottom wells, after which a small aliquot of fluorescently labeled PMN cells was placed on top, along with the LS PS and HS PS test materials and positive control materials. The chemotaxis chamber was be incubated and at the end of the incubation period the fluorescence of the bottom wells was read and used as an index of PMN migration. Test material LS PS and HS PS, were supplied as thick, viscous solutions (3g/100mL). These materials were used at a final concentration of 1.5%, 0.5% and 0.1%.

Heparinized whole blood (20-30 ml) was collected from a healthy human donor, layered over a density gradient (Histopaque 1077 and Histopaque 1119) and centrifuged at 400g for 30 min. The PMN rich fraction was removed, washed twice in with phosphate buffered saline (PBS) and then resuspended in 5.0 ml RPMI-1640 without phenol red supplemented with 10% heat-treated fetal calf serum (RPMI-FCS). Calcein AM (5 µg/ml final) was added to the cell suspension and then the cells were incubated for 30 min at 37°C. After the incubation, the PMN cells were washed twice with PBS, counted and resuspended in RPMI-FCS at 1-5 x 10⁶ cells/ml.

A disposable 96-well chemotaxis chamber was used to measure PMN migration (chemotaxis chambers manufactured by Neuroprobe Inc., Gaithersburg, MD, catalog number 101-5). To set up the 96-well chamber, the wells in the microplate (bottom chamber) were filled with 29 µl of FMLP (0.1 µM) diluted in PBS with 0.1% serum albumin. Negative control wells were prepared with PBS and albumin (no FMLP added). Amphotericin B (2 µg/ml) was used as a positive control (inhibits PMN migration). In addition, 3 wells were filled with 12.5 µl of fluorescently labeled PMN cells and 12.5 µl of PBS with 0.1% albumin. These latter wells were used as an index to represent 100% migration by the PMN cells.

Test materials were prepared in RPMI-FCS at 2x their desired final concentration. RPMI-FCS without test materials was used as another negative control. Aliquots of the test materials were combined with an equal volume of the fluorescently labeled PMN cells. A chemotaxis membrane was then placed over the bottom wells of the chemotaxis chamber and anchored into place. 25 µl of the PMN/test material combination was then spotted onto the membrane above each of the bottom wells (materials were tested in triplicate) and the well plate was incubated for 1 hour at 37 ± 2°C and 5% CO₂. At the end of the incubation, the top of the chemotaxis membrane was wiped clean with a tissue to remove any non-migrating cells and the bottom wells of the chemotaxis chamber were read using a fluorescent plate reader (485 nm excitation, 530 nm emission).

The mean fluorescence of the three wells filled directly with fluorescent PMN cells was determined and used to represent 100% migration. The mean fluorescence of the test material treated PMN cells were then determined (Test Material Migration). The fluorescent values were corrected for non-chemotaxis migration by subtracting the mean fluorescent measurements for the wells where FMLP was not present as a chemotractant. Percent PMN migration was then calculated using the following equation: ((Test Material Migration (mean RFU)) / (100% Migration (mean RFU))) x 100

The results for the PMN migration assay are presented in Table 16 and Figure 12(b). The values are expressed as mean percent of maximal (100%) migrating cells ± standard deviation. The LS PS and HS PS samples reduced PMN migration at all concentrations tested.

**Table 16. PMN Migration**

| Treatment | Percent of Maximal Migration | | |
|---|---|---|---|
| 1.5% LS PS | 7.3 | ± | 1.0 |
| 0.5% LS PS | 7.7 | ± | 0.7 |
| 0.1% LS PS | 11.1 | ± | 0.6 |
| 1.5% HS PS | 6.0 | ± | 0.8 |
| 0.5% HS PS | 9.0 | ± | 0.4 |
| 0.1% HS PS | 10.6 | ± | 1.3 |
| 2 ug/ml Amphotericin B | 9.4 | ± | 0.6 |
| Untreated | 13.5 | ± | 0.9 |

### EXAMPLE 26

### Antiinflammatory properties of materials from microalgae

The pro-inflammatory cytokine interferon-gamma, which is released by activated T-lymphocytes, plays a role in human inflammatory responses. Interferon-gamma also stimulates the enzyme indoleamine-2,3-dioxygenase, which degrades tryptophan to kynurenine. In concert with other pro-inflammatory cytokines, interferon-gamma is the most important trigger for the formation and release of reactive oxygen species (ROS). Interleukin-1 alpha (IL1-α) is a cytokine that also plays a major role in inflammatory responses. Microalgal materials were tested for their ability to suppress secretion of gamma interferon and IL1-α by human peripheral blood mononuclear cells.

Heparinized whole blood (20-30 ml) was collected from a healthy human donor, layered over a density gradient (Histopaque 1077 and Histopaque 1119) and centrifuged at 400g for 30 min. The PBMC rich fraction was removed and washed twice in with 5.0 ml RPMI-1640 without phenol red supplemented with 5% heat-treated fetal calf serum (RPMI-FCS). After washing, the cells were resuspended in RPMI-FCS at a final density of 1x10⁶ cells/ml.

Fifty microliters of PBMC cells were added to wells in a 96-well plate (each treatment was tested in triplicate). Next, 50 µl of RPMI-FCS supplemented with 2 µg/ml phytohemagglutinin (PHA) and the respective test materials was added to the wells. Cyclosporin A (2.5 µg/ml) was used as a positive control, while untreated cells served as a negative control (Untreated). One set of wells was not treated with PHA (Untreated-PHA). After the wells had been prepared, the plates were incubated for approximately 68 hours at 37 ± 2°C and 5% CO₂. After the incubation, 20 µl of a 20:1 solution of MTS:PMS (Promega) was added to each well and the 96-well plate was returned to the incubator for an additional 4 hours. The plate was then read at 490 nm using a plate reader. At the end of the incubation period the cell culture supernatant was assayed for IL-1α and gamma interferon.

Prior to the assay, all buffers and reagents were prepared according to the ELISA kit instructions (IL-1α: Cayman Chemicals (catalog number 583301); γ-IFN: R&D Systems (catalog number DIF50)) and allowed to reach room temperature. The 96-well plate was prepared by removing any unneeded well strips and by rinsing the plate once with Wash Buffer followed by blotting it dry. Next, a series of IL-1α standards was prepared ranging from 0 to 250 pg/ml and 100 µl of each of these standards was dispensed into two wells (duplicates) in the 96-well plate. Subsequently, 50 µl of each sample + 50 µl of culture media was added to additional wells (the samples were diluted to bring their IL-1α levels within the range of the standard curve), followed by the addition of 100 µl of acetylcholinesterase:Interleukin-1α FAB' Conjugate solution. After reserving two empty wells to serve as blanks, the 96-well plate was incubated overnight at 2-8°C. On the following day the wells were aspirated and washed 5-6 times with Wash Buffer. After the last wash was removed 200 µl of fresh Ellman's Reagent was added to each well. The plate was incubated at room temperature with periodic checks of the absorbance readings (405 nm) using a plate reader.

A series of gamma interferon standards was prepared ranging from15.6 pg/ml to 1000 pg/ml using human gamma interferon. Next, an ELISA microplate was prepared by removing any unneeded strips from the plate frame. 100 µl of Assay Diluent RD1F was added to each well, followed by the addition of 100 µl of sample (diluted in culture media if necessary to bring the gamma interferon values within the range of the standard curve) or 100 µl of standard per well. The microplate was covered and allowed to incubate for 2 ± 0.25 hours at room temperature. After the incubation the plate was aspirated and washed three times with 400 µl of wash buffer. After the last wash was removed 200 µl of anti-gamma interferon antibody/horseradish peroxidase conjugate solution was added to each well and the plate was incubated for 1 ± 0.25 hour at room temperature. The wells were aspirated and washed again as described above. After the last wash was removed 200 µl of horseradish peroxidase substrate solution (hydrogen peroxide + tetramethylbenzidine as a chromagen) was added to each well and the plate was incubated for 20 ± 5 minutes at room temperature. After the incubation 50 µl of stop solution (2 N sulfuric acid) was added to each well and the plate was read using a microplate reader at 450 nm.

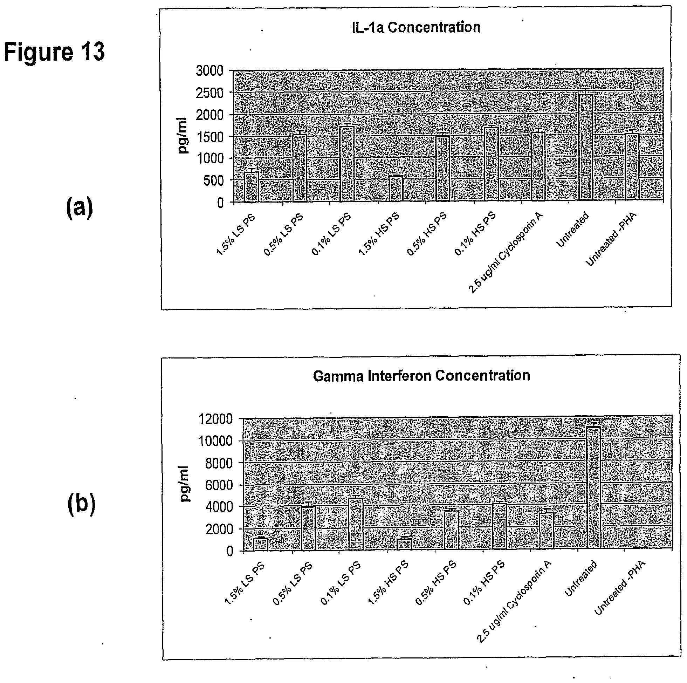

As demonstrated by Tables 17 and 18 and Figures 13(a) and 13(b), all materials tested at all concentrations reduced gamma interferon and IL1-α by human PBMC cells.

**Table 17. IL-1α Assay**

| Treatment | pg/ml | | |
|---|---|---|---|
| 1.5% LS PS | 689 | ± | 69.3 |
| 0.5% LS PS | 1530 | ± | 79.7 |
| 0.1% LS PS | 1715 | ± | 69.3 |
| 1.5% HS PS | 563 | ± | 38.5 |
| 0.5% HS PS | 1462 | ± | 99.9 |
| 0.1% HS PS | 1663 | ± | 54.1 |
| 2.5 ug/ml Cyclosporin A | 1561 | ± | 77.5 |
| Untreated | 2402 | ± | 146.2 |
| Untreated-PHA | 1512 | ± | 88.8 |

**Table 18. Gamma Interferon Assay**

| Treatment | pg/ml | | |
|---|---|---|---|
| 1.5% LS PS | 1116 | ± | 109.7 |
| 0.5% LS PS | 3924 | ± | 425.8 |
| 0.1% LS PS | 4626 | ± | 305.5 |
| 1.5% HS PS | 997 | ± | 159.7 |
| 0.5% HS PS | 3509 | ± | 203.0 |
| 0.1% HS PS | 4125 | ± | 131.4 |
| 2.5 ug/ml Cyclosporin A | 3259 | ± | 326.8 |
| Untreated | 11078 | ± | 315.0 |
| Untreated -PHA | 83 | ± | 7.0 |

### EXAMPLE 27

### Antiinflammatory properties of materials from microalgae

When presented with certain antigens, lymphocytes, which are the main cell type in PBMCs, respond by proliferating. This massive proliferation reaction forms the initial step in the immune response of this cell type. In vitro were used methods to study human lymphocyte proliferation using the antigen phytohemagglutinin (PHA) to stimulate a proliferation response. The ability of microalgal materials to inhibit this proliferation was tested.

Heparinized whole blood (20-30 ml) was collected from a healthy human donor, layered over a density gradient (Histopaque 1077 and Histopaque 1119) and centrifuged at 400g for 30 min. The PBMC rich fraction was removed and washed twice in with 5.0 ml RPMI-1640 without phenol red supplemented with 5% heat-treated fetal calf serum (RPMI-FCS). After washing, the cells were resuspended in RPMI-FCS at a final density of 1x10⁶ cells/ml.

Fifty microliters of PBMC cells were added to wells in a 96-well plate (each treatment was tested in triplicate). Next, 50 µl of RPMI-FCS supplemented with 2 µg/ml PHA and the respective test materials was added to the wells. Cyclosporin A (2.5 µg/ml) was used as a positive control, while untreated cells served as a negative control (Untreated). One set of wells was not treated with PHA and served as an index of non-stimulated proliferation (Untreated-PHA); all samples but this sample were treated with PHA. After the wells had been prepared, the plates were incubated for approximately 68 hours at 37 ± 2°C and 5% CO₂. After the incubation, 20 µl of a 20:1 solution of MTS:PMS (Promega) was added to each well and the 96-well plate was returned to the incubator for an additional 4 hours. The plate was then read at 490 nm using a plate reader.

The mean absorbance of the three wells of PBMC not treated with PHA was determined and used to represent non-stimulated proliferation. The absorbance values for the PBMC from each treatment group were also then determined. A stimulated proliferation index for each treatment was calculated using the following equations: ((Mean absorbance of treatment with PHA) / (Mean Absorbance without PHA)) x 100

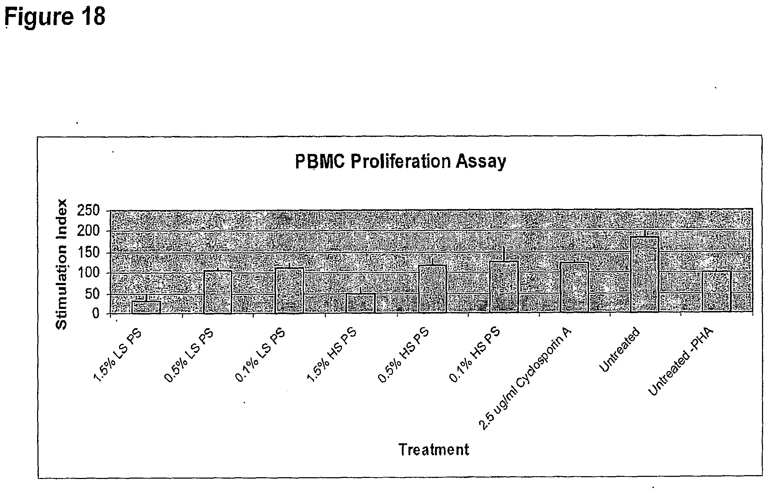

The results for the PBMC proliferation assay are presented in Table 19 and Figure 18. The values are expressed as a stimulation index, using the untreated PBMC not exposed to PHA to represent 100%. As can be seen in Table 19 and Figure 18, all materials tested reduced PBMC proliferation compared to PHA stimulated cells that were untreated by the test materials.

**Table 19. Proliferation Assay**

| Treatment | Stimulation Index | | |
|---|---|---|---|
| 1.5% LS PS | 32 | ± | 16.0 |
| 0.5% LS PS | 106 | ± | 6.9 |
| 0.1% LS PS | 111 | ± | 12.0 |
| 1.5% HS PS | 50 | ± | 13.6 |
| 0.5% HS PS | 117 | ± | 14.4 |
| 0.1% HS PS | 126 | ± | 34.4 |
| 2.5 ug/ml Cyclosporin A | 123 | ± | 6.5 |
| Untreated | 183 | ± | 13.9 |
| Untreated -PHA | 100 | ± | 5.2 |

### EXAMPLE 28

### Elastase Inhibition

Human dermal fibroblasts were cultured and used as a source of the elastase enzyme. This enzyme was be partially purified from the fibroblasts by lysing the cells in an elastase buffer and retaining the soluble portion of the lysate. Portions of this fibroblast lysate were then be incubated with test materials a synthetic elastase substrate, Suc-(Ala₃)-p-Nitroaniline (STANA). Elastase acts upon this substrate to release p-nitroaniline, which is detected with a spectrophotometer by measuring the absorbance at a wavelength of 405 nm. An inhibition of the elastase enzyme is identified by a decrease in the amount of released p-nitroaniline when compared to uninhibited enzyme.

Test material BM was supplied as a powder. 100 mg of BM was combined with either 5 ml of ethanol or 5 ml of ultrapure water in 15 ml centrifuge tubes. After combining, the mixtures were vortexed, then placed onto a rocking platform for approximately 30 minutes at room temperature, and then centrifuged at 1,000 RPM for 5 minutes. The supernatants were then used at the final concentrations listed in Table 20. The two additional materials used in this study, PS53 and PS133, were supplied as thick, viscous solutions (3%). These materials were also used at the final concentrations listed in the results section.

Human neonatal dermal fibroblasts were obtained and cultured per the vendor's specifications using sterile technique at all times (Cascade Biologics, catalog # C-004-5C). The cells were seeded in a 75-cm² flask and grown in Fibroblast Growth Medium (FGM: DMEM supplemented with 2% FBS, 5 ng/ml human recombinant growth factor, 5 ug/ml insulin, 50 ug/ml gentamicin, and 0.5 ug/ml Amphotericin-B) and subcultured until a sufficient number of cells had been grown.

After washing the cells twice with PBS, approximately 7.5 ml of PBS was added to each culture flask and the cells were detached using a cell scraper. The detached cells were transferred to a 15 ml centrifuge tube and the flask was rinsed with a second application of 7.5 ml of PBS, which was also transferred to the 15 ml tube. After centrifuging the tube for 5 minutes at 1,200 RPM (4°C), the supernatant was removed and discarded while the pellet was resuspended in 2 ml of ice cold 2x elastase buffer (400 mM Tris-HCl [pH 8.0], 0.2% Triton X-100) and sonicated 3x for 10 seconds or until the lysate became clear. The lysed cells were centrifuged at 2,200 RPM for 10 minutes (4°C) to remove any cellular debris. The supernatants from all of the preparations were pooled into a single container, mixed, and then aliquoted into 2 ml portions and stored at -75 ± 5°C until used. One of the aliquots was used to determine the protein concentration (BCA Protein Assay) and elastase activity level of the batch.

A Bicinchoninic Acid assay (Pierce, Inc., BCA Protein Assay Kit, catalog#23227) Fifty volumes of Reagent A (Bicinchoninic Acid BCA was combined with 1 volume of Reagent B (4% (w/v) CuSO₄ - 5 H₂O) in a 15-ml centrifuge tube. For the assay, proteins can reduce Cu(II) to Cu(I) in a concentration dependent manner. BCA can then react with the Cu(I) to form a purple colored complex with a maximum absorbance at 562 nm. Two hundred microliters of this combined reagent was dispensed into a 96-well plate. Next, 10 µl of each of the standards was added into their respective wells (standards were made using 2mg/ml bovine serum albumin dissolved in PBS, and then making a series of 50% dilutions down to 0.0078 mg/ml). Next, 10 µl of 2x elastase buffer (used as a blank) or cell lysate sample was added. The plate was covered and incubated at 37± 2°C for 30 ± 5 minutes. At the end of the incubation the absorbance of each well was measured at 540 nm using a microplate reader.

After measuring the protein concentration of the lysate, a small sample was obtained and the elastase activity of the lysate was determined at 100%, 50% and 25% concentrations using 2x elastase buffer to make the dilutions. To determine the activity, 100 µl of the three different lysate concentrations was loaded into a 96 well plate (each concentration tested in triplicate), while 100 ul of 2x Elastase buffer was added to three additional wells to serve as blanks. Next, 100 µl of deionized water was added to all wells and the 96-well plate will be allowed to incubate with the samples for 15 minutes at 37±2°C. Next, 4 µl of STANA (62.5 mM Suc-(Ala₃)-p-Nitroaniline in DMSO) was added to each well, and the 96-well plate was returned to the incubator for 1 hour. After the incubation the well plate was read at 405 nm using a microplate reader. The mean absorbance for each concentration of the lysate was plotted versus its respective concentration. These values were used to estimate a concentration that will produce a spectrophotometric measurement of 0.4 to 0.5 using a linear regression analysis of the best-fit line through all three (100%, 50%, and 25%) data points. The stored fibroblast lysates was diluted to the appropriate concentration to elicit the desired level of activity.

Test materials were prepared at 2x their final desired concentration in deionized water. If solvents were present in the test materials then appropriate solvent controls were also prepared. 100 µl of distilled water served as a negative control while 100 µl of 0.2 mM Phosphoramidon served as a positive control. After all of the wells had been prepared the well plate was sealed with an adhesive cover and incubated for 15 minutes at 37 ± 2°C to allow the inhibitors time to interact with elastase. After this preliminary 15 minute incubation, 4 µl of STANA was added; the plate was resealed and then incubated for 1 hour at 37 ± 2°C. At the end of the incubation the plate was read at 405 nm using a microplate reader.

**Table 20. Anti- Elastase Assay**

| Treatment | Percent Inhibition |
|---|---|
| 10% BM water extraction | 59 |
| 5% BM water extraction | 40 |
| 10% BM Ethanol extraction | 28 |
| 5% BM Ethanol extraction | 16 |
| 1% LS PS | 29 |
| 0.5% LS PS | 23 |
| 1% LS PS | 31 |
| 0.5% HS PS | 19 |
| 100 µM Phosphoramidon | 94 |

### EXAMPLE 29

Microalgal materials were tested to assess their ability to relieve redness and pain associated with sunburn. Informed consent was obtained from human subjects. A 1.5% preparation of polysaccharide from Porphyridium cruentum, purified essentially as described in example 2, was formulated with 1% Germaben II and 0.15% sodium EDTA. A patch of arm skin was exposed to UV radiation from a Solar Ultraviolet Simulator model 14S with a dose controller system model DCS-1 and a 150 watt xenon arc lamp #1122200 (Hanovia) having a continuous emission spectrum in the UV-B range of 290-320 nanometers. The dose was tested on each subject prior to the experiment to determine the minimal erythema dose (MED) necessary to achieve a level 2 response in each subject. Erythema was scored by a trained evaluator using the following scale : 0=no reaction ; +=minimal erythema ; 1= slight erythema ; 2 moderate erythema ; 3=marked erythema with or without edema ; 4= severe erythema with or without edema. Subjects wer thern exposed to a UVB dose sufficient to generate a level 2 erythema at three sites on the skin. One site was treated with the polysaccharide before and after irradiation. One site was treated with polysaccharide only after irradiation. One site was left untreated as a control. After 4, 24, 48, 72, and 96 hours, follow-up examination was performed. Polysaccharide was applied once daily to the two treated sites following daily examinations and was also treated once per day at home by each subject.

Mean erythema scores were the same on all three sites tested through the 4 and 24 hour evaluation points. At the 48 hour evaluation the mean erythema score decreased 5% on the site treated pre and post irradiation and stayed the same on the other two sites. At the 72 hour evaluation the mean erythema scores decrased by 30% on both sites treated with the polysaccharide and decreased 13% on the untreated site. At the 96 hour evaluation the mean erythema scores decrased by 40% on both sites treated with the polysaccharide and decreased 22% on the untreated site. There was no edema observed at any time on any site. The

**Table 21: Superoxide dismutases**

| Genbank accession number |
|---|
| CAA42737 |
| CAA43859 |
| AAA29934 |
| BAA02655 |
| NP_ 625295 |
| AH004200 |
| CAC14367 |
| YP_ 001003721 |
| ABM60577 |
| CAM08755 |
| YP 966270 |
| YP 963716 |
| ABM37237 |
| ABM35060 |
| ABM33234 |
| ABM33141 |
| NP_ 407488 |
| NP_ 405922 |
| YP_ 932026 |
| ZP 01641300 |
| ZP 01638301 |
| ZP 01637188 |
| EAX24391 |
| EAX23794 |
| EAX23720 |
| EAX23627 |
| EAX20859 |
| EAX19390 |
| EAX16596 |
| CAL93139 |
| YP_ 914326 |
| YP_ 747424 |
| AB 159459 |
| ZP_ 01610569 |
| ZP 01605216 |
| ZP 01600343 |
| ZP 01584712 |
| ZP_ 01581863 |
| ZP_ 01581157 |
| ZP_ 01575777 |
| ZP_ 01569848 |
| ZP_ 01559998 |
| EAW01367 |
| EAW01065 |
| EAV97274 |
| EAV95856 |
| EAV80568 |
| EAV73624 |
| EAV73531 |
| EAV70130 |
| EAV66456 |
| EAV61854 |
| ZP_ 01532079 |
| ZP_ 01516088 |
| EAV26209 |
| YP_845889 |
| YP_822355 |
| YP_843115 |
| YP_S36186 |
| ABK17454 |

**Table 22: Beta-carotene hydroxylases**

| Genbank accession number |
|---|
| NP_ 682690 |
| YP_172377 |
| NP _440788 |
| YP_475340 |
| YP_475340 |
| BAB75708 |
| ZP_ 01084736 |
| ZP_ 01080915 |
| ZP_ 01123496 |
| ABB27016 |
| NP_ 895643 |
| NP_ 896386 |
| ABB34062 |
| YP_292794 |
| AAP99312 |
| ZP_01006041 |
| ABB49299 |
| NP_ 848964 |
| ZP_ 01040435 |
| NP_ 049051 |
| YP_457405 |
| AAT38625 |

### SEQUENCE LISTING

SEQ ID NO 1: RBCS2 Chlamydomonas reinhardtii 
cgcttagaagatttcgataaggcgccagaaggagcgcagccaaaccaggatgatgtttgatggggtatttgagcacttgcaacccttat
ccggaagccccctggcccacaaaggctaggcgccaatgcaagcagttcgcatgcagcccctggagcggtgccctcctgataaacc
ggccagggggcctatgttctttactttttacaagagaagtcactcaacatcttaaacggtcttaagaagtctatccgg
SEQ ID NO 2: β-2 tubulin Chlamydomonas reinhardtii 
Ctttcttgcgctatgacacttccagcaaaaggtagggcgggctgcgagacggcttcccggcgctgcatgcaacaccgatgatgcttcg
acccccgaagctccttcggggctgcatgggcgctccgatgccgctccagggcgagcgctgtttaaatagccaggcccccgattgca
aagacattatagcgagctaccaaagccatattcaaacacctagatcactaccacttctacacaggccactcgagcttgtgatcgcactcc
gctaaggggggcgcctcttcctcttcgtttcagtcacaacccgcaaac
SEQ ID NO 3: Chlorella virus promoter #1 
cggggatcgcagggcatgggcattaaaagaactttatggaatcaaaaatcttagtgaatttccaccacaggtatatagtcttcaggacgc
taacgatgatatcaacgattgtatcaaggttatcgtttgaggcactcatatcaggtagtttctacacagaaacttgaacaacgcctggga
aaagatcctgagcatagtaacttatatactagcagatgttgtaacgatgctttatatgaatatgaattagcacaacgacaactacaaaaac
aacttgatgaatttgacgaagatgggtatgatttttttcaggcacgtataaatacattagatccgtcgacctgcagccaagctt
SEQ ID NO 4: Chlorella virus promoter #2 
cccggggatcatcgaaagcaactgccgcattcgaaacttcgactgcctcgttataaaggttagtgaaagccattgtatgttattactgagt
tatttaatttagcttgcttaaatgcttatcgtgttgatatgataaatgacaaatgatacgctgtatcaacatctcaaaagattaatacgaagatc
cgtcgacctgcagccaagctt
SEQ ID NO 5: Chlorella virus promoter #3 
cccggggatctgcgtattgcgggacttttgagcattttccagaacggattgccgggacgtatactgaacctccagtccctttgctcgtcgt
atttcccataatatacatatacactattttaattatttacaccggttgttgctgagtgatacaatgcaaattccctccaccgaggaggatcgcg
aactgtccaaatgtcttctttctgcagctccatacggagtcgttaggaaacattcacttaattataggatccgtcgacctgcagccaagctt
SEQ ID NO 6: Rhodella reticulata phycoerythrin beta subunit promoter from AF114823 
tttttatagatcatccaattattttttcattagatattgtatatcaataatttggcatatgttttgtagtatacgggttatgatattgcaatatatgtac
aacattggtaatttttggacttacatatatatcaattatatcaatgacaatgtaatatattggttgatagatcaataaacatctttaataagatctg
ttaaaattcaaatatagactttctgtattataagtagttttcttatattactatagacgtagaacgatcaaaaaaaataaatatggacatgactt
gattcaatatggaagacggggtatgagaaatatcgtgttgcactcaatatagaattgacgtattttaatgcagtgcccgttatatattgcgt
aacaaagattaaaagtatattatatattataatactagtagaccagcaaatataaaattatgctgaaacaataataccctttaaagttttaagg
agccttttc
SEQ ID NO 7: AHAS promoter from Porphyridium from AJ224709 
attatttaacaattggaaacttagttaattagggtaaattatattaacccttatgaaccaaaataatttggtttcaaaaaaaactaacttatgaat
taaaattgaaatattttctacatcataataattttaattctaaatagaattttagataagggatctaagataacaaaaaaatcaatttaagtaata
aagaaaatgtgattacaaaattttgatattaaactatagtatttacaaattattatcaaaaattacttatccatttgaggaaaagactgaacctt
taaacatatttgtttatgcgattttagatcattcaagttagcgagctgtatgaaatgaaagtttcatgtacagttcttaagtagagatgtatatat
gttaatagaaatattatttgcatcgactataatcaattctgaagacttcaaaataaaacctgttatacgtgctatactagagatggttgatgaa
ataaatcaaccaggtattattacagactgaactgaactaaaaaaattcatataatttagcgtact
SEQ ID NO 8: Carbonic anhydrase pca2 promoter AB040136 from Porphyridium purpureum 
gcacacgagtgttgtggcgttgtcgcagcaggtttgggggcgcgagagcgcacgacgcttgtgtgtgtgtgtgtgtgtggaccgcaa
ccaccctcgcgacgcggcattgccgtgcgtgccgtcgcggctgcgtggttcgtggtgtgatattctaaacgcatgtgggttgggtgtgg
gtgttgttctgtgtccatcaggcgatggacacagccgccactgaagtgtcactgaattaagcgcggtgcattttgcacgtggcttttgtgt
gggtgtgtgtgtatgtgtcctgctcggcttgtatcgacatcctccttcgtttttctcgtacggggcttttgtgtttcctttggtacgtggtgagc
 
gtttttggggtgttgccggacatgatggtgttgtgtttgtgagtttgggagtgtgagactgggagcgacggtgaagccgcatgaatcgt
ggagcgcaaaatgcaagttgactggagccatcgcgatgcttttggcgttttgcgcatgtgatcacaatctcctcggaatggtccaaaatg
gatcgaactggctcgcccccccaatctgtgcgctttcggcctgttcggacatgccggtttcgcggtgcgcagcatgtggctcgcgcatg
gtaggggatgttggcgcggggcataaataggctgcgacaacttgccgcttccccttcatcgcacacctcaggcaggaggaagtggtg
gaaaagactggtgcaggagaggatttgcaggagaggaaggagagggagaggcgtgtcgtgcttgccactgcgatagtcacc
SEQ ID NO 9: Carbonic anhydrase peal promoter AB040135 from Porphyridium purpureum 
gcgtgcgtcaagcacattggggcaactcgggcaaccgacgcagccacgcacacgagtgttgtggcgttgtcgtagcaggtttgggg
gcgcgagagcgcacgacgcgtgtgtgtgtgtgtgtgtggaccgcaaccaccctcgcgacgcggcattgccgtgcctgccgttgc
ggctgcgtggttcgtggtgtgatattctaaacgcatgtgggttgggtgttggtgttgttctgtgtccatcaggcgatggacacagccgcca
ctgaagtgtcactgaattaagcgcggtgcattttgcacgtggcttttgtgtgtgtgtgtttgtgtctatgtgtcctgctcggtttgtatcgacgt
cctccttcgtttttttcgcacggggcttttgtctttcctttggtacgtggtgagcgtttttggggtgttgccggacatgatggtgttgtgtttgt
gagtttgagagtgagactgggagcgacggtgaagccgcatgaatcgtggagcgcaaaatgcaagttgactggagccatcgcgatgc
ttttggcgttttgcgcatgtgatcacaatctcctcggaatggtccaaaatggatcgaactggctcgcccccccaatctgtgcgctttcggcct
gttcggacatgccggtttcgtggtgcgcagcatgtggctcgcgcatggtaggggatgttggcgcgggggcataaataggctgcgacaa
cttgccgcttccccttccctgcacgcctcaggcaggaagaagtggtggaaaagactggtgcaggagaggatcttgcaggagaggaa
ggagagggagaggcgtgtcgtgcttgccactgcaatcgtcacc
SEQ ID NO 10: Sulfometuron resistance- acetohydroxy acid synthase [Porphyridium sp.] 
mthieksnyqeqtgafalldslvrhkvkhifgypggailpiydelykweeqgyikhilvrheqgaahaadgyaratgevgvcfats
gpgatnlvtgiatahmdsipiviitgqvgrsfigtdafqevdifgitlpivkhsyvirdprdiprivaeafsiakqgrpgpvlidvpkd
vgletfeyqyvnpgearipgfrdlvapssrqiihsiqliqeanqpllyvgggaitsgahdliyklvnqykipitttlmgkgiideqnpl
algmlgmhgtayanfavsecdllitlgarfddrvtgkldefacnakvihvdidpaevgknripqvaivgdislvleqwllyldrnlq
lddshlrswherifrwrqeyplivpklvqtlspqeiianisqimpdayfstdvgqhqmwaaqfvktlprrwlsssglgtmgyglp
aaigakiaypespvvcitgdssfqmniqelgtiaqykldikiiiinnkwqgmvrqsqqafygaryshsrmedgapnfvalaksfg
idgqsistrqemdslfntiikykgpmvidckviedencypmvapgksnaqmigldksnneiikike
SEQ ID NO 11: Zeocin resistance- ble protein 
marmakltsavpvltardvagavefwtdrlgfsrdfveddfagvvrddvtlfisavqdqdqvvpdntlawvwvrgldelyaews
evvstnfrdasgpamteigeqpwgrefalrdpagncvhfvaeeqd
SEQ ID NO 12: Superoxide Dismutase From AAB60930 
gyvnglesaeetlaenresgdfgssaaamgnvthngcghylhtlfwenmdpngggepegelldrieedfgsyegwkgefeaaa
saaggwallvydpvakqlrnvpvdkhdqgalwgshpilaldvwehsyyydygpargdfida
SEQ ID NO 13: Superoxide Dismutase from NP_000445
SEQ ID NO 14: Superoxide Dismutase-Polysaccharide binding protein fusion AAV48590-NP_000445 
marmvvaavavmavlsvalaqfipdvditwkvpmtltvqnlsiftgpnqfgrgipspsaigggngldivggggslyisptggqv
qysrgsnnfgnqvaftrvrkngnnesdfatvfvggttpsfvivgdstenevsfwtnnkvvvnsqgfippngnsaggnsqytfvng
itgtagapvggtvirqvsawreifntagncvksfglvvrgtgnqglvqgveydgyvaidsngsfaisgyspavnnapgfgknfaa
artgnffavssesgvivmsipvdnagctlsfsvaytitpgagrvsgvslaqdnefyaavgipgagpgevriyrldgggattlvqtlsp
pddipelpivanqrfgemvrfgansetnyvavgspgyaaeglalfytaepgltpndpdeglltllaysnsseipangglgefmtasn
crqfvfgepsvdsvvtflasigayyedyctcerenifdqgimfpvpnfpgespttcrssiyefrfnclmegapsictyserptyewte
evvdpdntpcelvsriqrrlsqsncfqdyvtlqvvgagagmatkavcvlkgdgpvqgiinfeqkesngpvkvwgsikglteglh
gfhvhefgdntagctsagphfnplsrkhggpkdeerhvgdlgnvtadkdgvadvsiedsvislsgdhciigrtlvvhekaddlgk
ggneestktgnagsrlacgvigiaq
SEQ ID NO 15: Protein associated with polysaccharide from Porphyridium from AAV48590 
marmvvaavavmavlsvalaqfipdvditwkvpmtltvqnlsiftgpnqfgrgipspsaigggngldivggggslyisptggqv
qysrgsnnfgnqvaftrvrkngnnesdfatvfvggttpsfvivgdstenevsfwtnnkvvvnsqgfippngnsaggnsqytfvng
itgtagapvggtvirqvsawreifntagncvksfglvvrgtgnqglvqgveydgyvaidsngsfaisgyspavnnapgfgknfaa
artgnffavssesgvivmsipvdnagctlsfsvaytitpgagrvsgvslaqdnefyaavgipgagpgevriyrldgggattlvqtlsp
pddipelpivanqrfgemvrfgansetnyvavgspgyaaeglalfytaepgltpndpdegllltllaysnsseipangglgefmtasn
crqfvfgepsvdsvvtflasigayyedyctcerenifdqgimfpvpnfpgespttcrssiyefrfnclmegapsictyserptyewte
evvdpdntpcelvsriqrrlsqsncfqdyvtlqvv
SEQ ID NO: 16 RBCS2 promoter from Chlamydomonas reinhardtii 
Gccagaaggagcgcagccaaaccaggatgatgtttgatggggtatttgagcacttgcaacccttatccggaagccccctggcccaca
aaggctaggcgccaatgcaagcagttcgcatgcagcccctggagcggtgccctcctgataaaccggccaggggcctatgttcttta
ctttttacaagagaagtcactcaacatcttaa
SEQ ID NO 17 Superoxide Dismutase-Polysaccharide binding protein fusion AAV48590-NP_000445 
matkavcvlkgdgpvqgiinfeqkesngpvkvwgsikglteglhgfhvhefgdntagctsagphfnplsrkhggpkdeerhvg
dlgnvtadkdgvadvsiedsvislsgdhciigrtlvvhekaddlgkggneestktgnagsrlacgvigiaqmarmvvaavavma
vlsvalaqfipdvditwkvpmtltvqnlsiftgpnqfgrgipspsaigggngldivggggslyisptggqvqysrgsnnfgnqvaft
rvrkngnnesdfatvfvggttpsfvivgdstenevsfwtnnkvvvnsqgfippngnsaggnsqytfvngitgtagapvggtvirq
vsawreifntagncvksfglvvrgtgnqglvqgveydgyvaidsngsfaisgyspavnnapgfgknfaaartgnffavssesgvi
vmsipvdnagctlsfsvaytitpgagrvsgvslaqdnefyaavgipgagpgevriyrldgggattlvqtlsppddipelpivanqrf
gemvrfgansetnyvavgspgyaaeglalfytaepgltpndpdeglltllaysnsseipangglgefmtasncrqfvfgepsvdsv
vtflasigayyedyctcerenifdqgimfpvpnfpgesptterssiyefrfnclmegapsictyserptyewteevvdpdntpcelv
sriqrrlsqsncfqdyvtlqvv
SEQ ID NO 18: Zeaxanthin epoxidase from AAO34404 
mlastytpcgvrqvagrtvavpsslvapvavarslglapyvpvcepsaalpacqqpsgrrhvqtaatlradnpssvaqlvhqngk
gmkviiagagiggl vlavallkqgfqvqvferdltairgegkyrgpiqvqsnalaaleaidpevaaevlregcitgdringlcdgltg
ewyvkfdtfhpavskglpvtrvisrltlqqilakaveryggpgtiqngcnvtefterrndttgnnevtvqledgrtfaadvlvgadgi
wskirkqligetkanysgytcytgisdftpadidivgyrvflgngqyfvssdvgngkmqwygfhkepsggtdpegsrkarllqif
ghwndnvvdlikatpeedvlrrdifdrppiftwskgrvallgdsahamqpnlgqggcmaiedayelaidlsravsdkagnaaav
dvegvlrsyqdsrilrvsaihgmagmaafmastykcylgegwskwveglripphpgrvvgrlvmlltmpsvlewvlggntdhv
aphrtsycslgdkpkafpesrfpefmnndasiirsshadwllvaerdaataaanvnaatgssaaaaaadvnsscqckgiymad
saalvgrcgatsrpalavddvhvaeshaqvwrglaglppssssastaaasasaassaasgtastlgssegywlrdlgsgrgtwvngk
rlpdgatvqlwpgdavefgrhpshevfkvkmqhvtlrsdelsgqayttlmvgkirnndyvmpesrpdggsqqpgrlvta
SEQ ID NO 19: lycopene epsilon cyclase from BAA97033 
mecvgarnfaamavstfpswscrrkfpvvkrysyrnirfglcsvrasgggssgsescvavredfadeedfvkaggseilfvqmq
qnkdmdeqsklvdklppisigdgaldlvvigcgpaglalaaesaklglkvgligpdlpftnnygvwedefndlglqkciehvwre
tivyldddkpitigraygrvsrrllheellrrcvesgvsylsskvdsiteasdglrlvacddnnvipcrlatvasgaasgkllqyevggp
rvcvqtaygvevevenspydpdqmvfmdyrdytnekvrsleaeyptflyampmtksrlffeetclaskdvmpfdllktklmlrl
dtlgirilktyeeewsyipvggslpnteqknlafgaaasmvhpatgysvvrslseapkyasviaeilreettkqinsnisrqawdtlw
pperkrqrafflfglalivqfdtegirsffrtffrlpkwmwqgflgstltsgdlvlfalymfvispnnlrkglinhlisdptgatmiktylk
v
SEQ ID NO 20: Porphyra EF1 promoter 
aagcttcgctgccaggctctccatcagcgacttgcggtcggtgctgtttggggaccggcgggaagcgcaccagaatgtgggggagacaggca
gggctcagagacacgagtggagagcattgatcagtaacagtcgcacgtcagactgggtctgcgtggggccacgaaagcgcaaaaatgggccta
gcaggcgccgtcaaccttctcaagctcagagcgccgccaggctgcacacccccgaggcgacggccagtagcacctgcgccacggagacaccg
tctcctgctaacatacggttgttcgtccgccatcgagaacagctaggtacagaataccacgaggaagggaagaaaagccttgaatggcaaaggt
 
ggtcgaaggcaagccagagagggacgttctacatgcaccaaagccagtaggagcgcactacgcggtaagcggtcgccgaaagagggggggc
gacccacgctcgtcactgcaggttgtattgtgccggtccgcaactgccactggcggtgtttgtcaagcggccaccacgagtctcgagggctcagca
attgcaccgctggcacgcagaatgtggccttccccgagtttacagtggtccttttccccgaaagcatccctaaaaaaggaattgagcgacggcga
cgccgaccgacgtccgcggcggcggccacaggcgagggacggaggaccaccggccttttaaatgggcgtcgaagaccacgtgctcggcaca
ccaccccgtcgacatcgctatcgtacccgtcttccccgccgattgacgtcttgagtttacctgttcacctagcctttcatc
SEQ ID NO 21: Glycoprotein promoter from Porphyridium sp. 
cgtcattagcgaagcgtccgcgttcacagcgttcgatggaggtggcagggacaatgatggtggagcctcttcgcacctcagcgcggcatacacca
caaaaccaccgtctaactcgagggacagcgcctggcgcacgttggatttcagggtcgcgtaccgcgccaggtcaaagtggatcttaccgaccagt
ttttcgttgtcactgctagccgcggtcaccgactttgccgagtctgtgtggtacagctgcagcacggcaaagcgctggtggcgatcttcaaagtttcg
gtccgatgcgcgtctcgacaggcgcgcaggaacgctgaaggtctcgttccacacggccacgttgggctgatggtcgaaactgacgaatgcgcttg
gttttcgtcccgggccgtccgagcacctgctggtctccatacgcgagtcgaaagcgatacgtgccatccactttcatgcgggtcactttctccacttcg
agcgtaaatatgaacgtatgcgagcagaggtgcccaccgccactcacgttcgtgccggtgtcgacgccattcgtcgcccatgacgaggaagaga
gcaagacgacgtttccagccctgctcgccgacttgctctgcgccgacgacgcggacgcctgcgtcacactggaggagtttgaggcgttggacgc
gcgcacgctggactctcgcttcactagcaagccactcccgctgcgatcgagcgacggcgagcgcgagcccttcctactctcactgcgcgacgag
gcggtcgagtccgagcgactcacctggcttaagcgtctctcgctcatgacgcgcttgacgagcaccttgcgagtagccagccgcacctctacccg
cgcatgccaacctcactccagtgtctcgcaagcaaccacgatgctccttcccagtcgaatgcgacacgcgaccacgttcgccgcgcaactttttttc
caccgcagaaattggcgcggacgaagtacagctgacccgcaccaacggtacagtcatgagcttgtaactgccacttcgcacttttgtaacgtataaa
ataacgatacaaaggtggattaattatttcatttttcatgttccagatgtttcaagctctcatgctttgtgagtgtttttggatgtgtagccggcaaccctact
tgttcgggcaacgaaacagattgttcgtgcggttggcgcggttgacgcggttgatgacaactggatgacgaaaaccagtgcgcaagaaggggggg
gggggttaagtatagtgtgtttagtcaacgaacggcgtgtgaatttaaatgctttggggtgtagtcaaacgggtgtggtttacaaggcgctgccaagc
agcgggagtacagcgctgtacgccgccgatttcaaaaggagacacgcgcgagcaacaaaatcacgaatcgggtggatttgcgtggtgcgttcgt
tggggtgtgtgtgtggcgctggcggttggacacgcgtaacaaatgcaggctgttttactataaagtcgggcgagaagcggatggctggtgcggtgt
gatgggcgcgcacgacgccacgcagtgcctctatgcaatcgagagggagatgcgaaaaaaagggggcggtctggtataaagtgcgcgggcac
gtcgtaggtacttaaatgctgtggggacggtgaaaagagtgcgtgagtgaggtgtgtggacgaaaggagagggaagagggagtgggtggccg
ctgtagagaacacggtcgggtgcagtaacgaa
SEQ ID NO 22: Glycoprotein-collagen fusion AAV48590-CAA34683 
marmvvaavavmavlsvalaqfipdvditwkvpmtltvqnlsiftgpnqfgrgipspsaigggngldivggggslyisptggqv
qysrgsnnfgnqvaftrvrkngnnesdfatvfvggttpsfvivgdstenevsfwtnnkvvvnsqgfippngnsaggnsqytfvng
itgtagapvggtvirqvsawreifntagncvksfglvvrgtgnqglvqgveydgyvaidsngsfaisgyspavnnapgfgknfaa
artgnffavsssesgvivmsipvdnagctlsfsvaytitpgagrvsgvslaqdnefyaavgipgagpgevriyrldgggattlvqtlsp
pddipelpivanqrfgemvrfgansetnyvavgspgyaaeglalfytaepgltpndpdeglltllaysnsseipanggglgefmtasn
crqfvfgepsvdsvvtflasigayyedyctcerenifdqgimfpvpnfpgespttcrssiyefrfnclmegapsictyserptyewte
evvdpdntpcelvsriqrrlsqsncfqdyvtlqvvmirlgapqslvllltllvaavlrcqgqdvrqpgpkgqkgepgdikdivgpkg
ppgpqgpageqgprgdrgdkgekgapgprgrdgepgtpgnpgppgppgppgppglggnfaaqmaggfdekaggaqlgv
mqgpmgpmgprgppgpagapgpqgfqgnpgepgepgvsgpmgprgppgppgkpgddgeagkpgkagergppgpqg
argfpgtpglpgvkghrgypgldgakgeagapgvkgesgspgengspgpmgprglpgergrtgpagaagargndgqpgpag
ppgpvgpaggpgfpgapgakgeagptgargpegaqgprgepgtpgspgpagasgnpgtdgipgakgsagapgiagapgfpg
prgppgpqgatgplgpkgqtgepgiagfkgeqgpkgepgpagpqgapgpageegkrgargepggvgpigppgergapgnrg
fpgqdglagpkgapgergpsglagpkgangdpgrpgepglpgargltgrpgdagpqgkvgpsgapgedgrpgppgpqgarg
qpgvmgfpgpkgangepgkagekglpgapglrglpgkdgetgaagppgpagpagergeqgapgpsgfqglpgppgppgeg
gkpgdqgvpgeagapglvgprgergfpgergspgaqglqgprglpgtpgtdgpkgasgpagppgaqgppglqgmpgergaa
giagpkgdrgdvgekgpegapgkdggrgltgpigppgpagangekgevgppgpagsagargapgergetgppgpagfagpp
gadgqpgakgeqgeagqkgdagapgpqgpsgapgpqgptgvtgpkgargaqgppgatgfpgaagrvgppgsngnpgppg
ppgpsgkdgpkgargdsgppgragepglqgpagppgekgepgddgpsgaegppgpqglagqrgivglpgqrgergfpglpg
psgepgkqgapgasgdrgppgpvgppgltgpagepgrqgspgadgppgrdgaagvkgdrgetgavgapgtpgppgspgpa
gptgkqgdrgeagaqgpmgpsgpagargiqgpqgprgdkgeagepgerglkghrgftglqglpgppgpsgdqgasgpagps
gprgppgpvgpsgkdgangipgpigppgprgrsgetgpagppgnpgppgppgppgpgidmsafaglgpr
SEQ ID NO 23: Glycoprotein-elastin fusion AAV48590- NP 000492 
marmvvaavavmavlsvalaqfipdvditwkvpmtltvqnlsiftgpnq fgrgipspsaigggngldivggggslyisptggqv
qysrgsnnfgnqvaftrvrkngnnesdfatvfvggttpsfvivgdstenevsfwtnnkvvvnsqgfippngnsaggnsqytfvng

[?]
 
itgtagapvggtvirqvsawreifntagncvksfglvvrgtgnqglvqgveydgyvaidsngsfaisgyspavnnapgfgknfaa
artgnffavsssesgvivmsipvdnagctlsfsvaytitpgagrvsgvslaqdnefyaavgipgagpgevriyrldgggattlvqtlsp
pddipelpivanqrfgemvrfgansetnyvavgspgyaaeglalfytaepgltpndpdeglltllaysnsseipangglgefmtasn
crqfvfgepsvdsvvtflasigayyedyctcerenifdqgimfpvpnfpgespttcrssiyefrfnclmegapsictyserptyewte
evvdpdntpcelvsriqrrlsqsncfqdyvtlqvvmagltaaaprpgvlllllsilhpsrpggvpgaipggvpggvfypgaglgalg
ggalgpggkplkpvpgglagaglgaglgafpavtfpgalvpggvadaaaaykaakagaglggvpgvgglgvsagavvpqpga
gvkpgkvpgvglpgvypggvlpgarfpgvgvlpgvptgagvkpkapgvggafagipgvgpfggpqpgvplgypikapklp
ggyglpyttgklpygygpggvagaagkagyptgtgvgpqaaaaaakaaakfgagaagvlpgvggagvpgvpgaipgiggia
gvgtpaaaaaaaaakaakygaaaglvpggpgfgpgvvgvpgagvpgvgvpgagipvvpgagipgaavpgvvspeaaaka
aakaakygarpgvgvggiptygvgaggfpgfgvgvggipgvagvpsvggvpgvggvpgvgispeaqaaaakaakygvgt
paaaaakaaakaaqfglvpgvgvapgvgvapgvgvapgvglapgvgvapgvgvapgvgvapgigpggvaaaaksaakva
akaqlraaaglgagipglgvgvgvpglgvgagvpglgvgagvpgfgagadegvrrslspelregdpsssqhlpstpssprvpgal
aaakaakygaavpgvlgglgalggvgipggvvgagpaaaaaakaaakaaqfglvgaaglgglgvgglgvpgvgglggippa
aaakaakygaaglggvlggagqfplggvaarpgfglspifpggaclgkacgrkrk
SEQ ID NO: 24: zeocin resistance gene 
atggccaagttgaccagtgccgttccggtgctcaccgcgcgcgacgtcgccggagcggtcgagttctggaccgaccggctcgggtt
ctcccgggacttcgtggaggacgacttcgccggtgtggtccgggacgacgtgaccctgttcatcagcgcggtccaggaccaggtggt
gccggacaacaccctggcctgggtgtgggtgcgcggcctggacgagctgtacgccgagtggtcggaggtcgtgtccacgaacttcc
gggacgcctccgggccggccatgaccgagatcggcgagcagccgtgggggcgggagttcgccctgcgcgacccggccggcaa
ctgcgtgcacttcgtggccgaggagcaggactga
SEQ ID NO: 25 Human GLUT1 protein in Porphyridium codons 
atggagccgagcagcaagaagctgaccggccgcctgatgctggcggttggcggcgccgttctgggcagcctccagttcggctacaa
caccggcgtgattaacgccccacagaaggtgatcgaggagttctacaaccagacctgggtccaccgctacggcgagagcattctgc
cgaccaccctgaccacgctgtggagcctgagcgtggcgattttcagcgtgggcggcatgattggcagcttctcggtgggcctgttcgt
gaaccgcttcggccgccgcaacagcatgctgatgatgaacctgctggccttcgtgtcggcggtgctgatgggcttcagcaagctggg
caagagcttcgagatgctgattctgggccgcttcattattggcgtgtactgcggcctgaccaccggcttcgtgccgatgtacgtgggcg
aggtgtcgccaacggcgttccgcggcgcgctgggcaccctccatcagctgggcattgttgtgggcattctgattgcccaggtgttcgg
cctggacagcattatgggcaacaaggacctgtggccgctgctgctgtcgattattttcattccggcgctgctccagtgcattgtgctgcc
gttctgcccagagagcccacgcttcctgctgattaaccgcaacgaggagaaccgcgcgaagagcgtgctgaagaagctgcgcggc
acggcggacgttacccacgacctccaggagatgaaggaggagagccgccagatgatgcgcgagaagaaggtgaccattctggag
ctgttccgctcgccagcgtaccgccagccgattctgatcgccgtggtgctccagctgtcccagcagctgtcgggcattaacgccgtgtt
ctactacagcaccagcattttcgagaaggcgggcgtccagcagccagtgtacgccaccattggcagcggcattgtgaacaccgcctt
caccgtggtgtcgctgttcgtggttgagcgcgcgggccgccgcacgctccatctgattggcctggcgggcatggcgggctgcgcgat
tctgatgaccattgccctggcgctgctggagcagctgccgtggatgagctacctgagcattgtggcgatcttcggcttcgtggcgttctt
cgaggttggcccaggcccgattccgtggttcattgtggcggagctgttcagccagggcccacgcccagcggcgattgccgttgccgg
cttctcgaactggaccagcaacttcattgtgggcatgtgcttccagtacgtcgagcagctgtgcggcccgtacgtgttcattatcttcacc
gtgctgctggtcctcttcttcatcttcacctacttcaaggtgccggagaccaagggccgcaccttcgacgagattgccagcggcttccgc
cagggcggcgccagccagagcgacaagaccccggaggagctgttccatccactgggcgccgacagccaggtgtaa
SEQ ID NO: 26 GCCAAGTTGACCAGTGCCGTTCCGGTG
SEQ ID NO: 27 CGGCTGCTCGCCGATCTCGGTCATGG
SEQ ID NO: 28 ATACGCGAGTCGAAAGC
SEQ ID NO: 29 ACAGCGCTGTACTCCC
SEQ ID NO: 30 TGCTGATGATGAACCTGCTGGCC
SEQ ID NO: 31 AGCACAATGCACTGGAGCAGCG
SEQ ID NO:32 CMV 3' UTR 
tttctccataataatgtgtgagtagttcccagataagggaattagggttcctatagggtttcgctcatgtgttgagcatataagaaacccctta
gtatgtatttgtatttgtaaaatacttctatcaataaaatttctaattcctaaaaccaaaatccagtactaaaatccagatcccccgaatta
SEQ ID NO: 33 Cyanidioschyzon merolae Na+/glucose co-transporter c05f0001 
mastapantavalntldwvlvivyfsalvlaiwlstrkssragsgtqskpasemfflagrsttffavgaslfmsnigsehfialaaagatsglavasf
ewmasifvgvvlgrvfapfylrnslhtvpkflelryaagarryhalatimmailtkvsatlysgaiilrvllgwpvwfslimilvlttlytslgglrav
iwtevlqafvllagglalavrslqavgslaglsellaaqnrrqmldllqwpssttpwveypwpgiifglpalevfywctdqvvvqrvlsakseah
arggsllcgflktlvpfmmvipglcafllfpevaanpnqayptavarllphgllglmvsamlaalmsslastfnststvvvydfvieccglsrlsd
ktlvllgrianivlcafslawipivegmgeelyfyiqsvisyiappiavvfvagiawrratatgalctllvggalglvrfvvevalrlahreaplgalg
kiffqsnflyfaifswvfsslllvtvslfteppseqqlgllfqeagasgshvrttagnqigeasskqvvgasryvadepsssdpaarqqtveleiesfs
gtensdeafsvdpehtapsmkrvasrdtllageaasqeplfspqgefsaaqetfssaapsrltsaaldvlsvvlvaeilafyiqfr
SEQ ID NO: 34 Cyanidioschyzon merolae arabinose permease CMK066C 
metvtvrwkrflstfgarpcllscllggllfgyhlavfstvttfrsfqdwfgswpsgeqvllgsyfvgafvgclyqrvlpflahgagtaawrrylwl
rwssvffclgsglpflikrermltgrfqmgaflvllihrlligigagivnvlgpalclevapstsrgafvflyqlaitigilmanlvnlatghedvhrqv
dpvaggagidmrgnflrplryplvpailmcigllryqssmgvsayrdatsadmletgkpmsqrkerehsmvasaralrpdaaeaghsfnvis
rpslqneslslkdvesfamyavlargsspptarsrasvwlllrdpriqicmilqllqqltginvvlvygvqileqvqssamgssrrlarlsgplygavl
lsvmnviatlvavgiidrtcrrklylfstpvlaachlalaratraengsssvaftgflalmlfvavfavshgplavlvanelfspearasansigmvv
navattavsigfpllqrelfgiagtflffalilmggeywlwrylpetrqptsadss
SEQ ID NO: 35 Galdieria sulphuraria myo-inositol transporter EAL84263 
mveksssdpevpslshhessisiekqgdaatarewaqdvnstttntklknplagltreqllndveafakekdlehilddlrkgalvaqdprefeq
mdaltesekellrrekthrwsqpfmmyfmtsessrypptefgfnpacqssvldllscrewirllstvrrsmyssithlsyakqsrfyfaefnvtdt
wmqgllngapylcsavigcwttaplnrwfgrrgcifiscfisfassfwmaaahtwwnlllgrfllgfavgakstttpvygaecspanirgalvm
mwqmwtafgimlgyiasvafmdvthptipgfnwrlmlgstaippffvciqvyfcpesprwymmrnryhdaykalckfrpstfqaardly
yihaalkveeklregkhlfremftiprnrraaqssffvmfmqqfcgvnaimyysssmfreagfdtrmalitslgcgitnwifalpavytidtfgrr
nllltfplmcifllftgfsfyipdqtsrtacvatgiylfmivyspgegpvpftysaeafplyirdigmsfatattwgfnfivsltwpslnksftptgaf
gwyaawnffgwifcyfclpetkalsleeldqvfsvpttkhvnhyramlpwyvkkyllrrdvppqnqlydyy
SEQ ID NO: 36 glp promoter - glp-SOD fusion- CMV 3'UTR 
cgtcattagcgaagcgtccgcgttcacagcgttcgatggaggtggcagggacaatgatggtggagcctcttcgcacctcagcgcggcatacacca
caaaaccaccgtctaactcgaggacagcgcctggcgcacgttggatttcagggtcgcgtaccgcgccaggtcaaagtggatcttaccgaccagt
ttttcgttgtcactgctagccgcggtcaccgactttgccgagtctgtgtggtacagctgcagcacggcaaagcgctggtggcgatcttcaaagtttcg
gtccgatgcgcgtctcgacaggcgcgcaggaacgctgaaggtctcgttccacacggccacgttgggctgatggtcgaaactgacgaatgcgcttg
gttttcgtcccgggccgtccgagcacctgctggtctccatacgcgagtcgaaagcgatacgtgccatccactttcatgcgggtcactttctccacttcg
agcgtaaatatgaacgtatgcgagcagaggtgcccaccgccactcacgttcgtgccggtgtcgacgccattcgtcgcccatgacgaggaagaga
gcaagacgacgtttccagccctgctcgccgacttgctctgcgccgacgacgcggacgcctgcgtcacactggaggagtttgaggcgttggacgc
gcgcacgctggactctcgcttcactagcaagccactcccgctgcgatcgagcgacggcgagcgcgagcccttcctactctcactgcgcgacgag
gcggtcgagtccgagcgactcacctggcttaagcgtctctcgctcatgacgcgcttgacgagcaccttgcgagtagccagccgcacctctacccg
cgcatgccaacctcactccagtgtctcgcaagcaaccacgatgctccttcccagtcgaatgcgacacgcgaccacgttcgccgcgcaactttttttc
caccgcagaaattggcgcggacgaagtacagctgacccgcaccaacggtacagtcatgagcttgtaactgccacttcgcacttttgtaacgtataaa
ataacgatacaaaggtggattaattatttcatttttcatgttccagatgtttcaagctctcatgctttgtgagtgtttttggatgtgtagccggcaaccctact
tgttcgggcaacgaaacagattgttcgtgcggttggcgcggttgacgcggttgatgacaactggatgacgaaaaccagtgcgcaagaaggggggg
gggggttaagtatagtgtgtttagtcaacgaacggcgtgtgaatttaaatgctttggggtgtagtcaaacgggtgtggtttacaaggcgctgccaagc
agcgggagtacagcgctgtacgccgccgatttcaaaaggagacacgcgcgagcaacaaaatcacgaatcgggtggatttgcgtggtgcgttcgt
tggggtgtgtgtgtggcgctggcggttggacacgcgtaacaaatgcaggctgttttactataaagtcgggcgagaagcggatggctggtgcggtgt
gatgggcgcgcacgacgccacgcagtgcctctatgcaatcgagagggagatgcgaaaaaaggggggcggtctggtataaagtgcgcgggcac
gtcgtaggtacttaaatgctgtggggacggtgaaaagagtgcgtgagtgaggtgtgtggacgaaaggagagggaagagggagtgggtggccg
ctgtagagaacacggtcgggtgcagtaacgaaATGGCCCGCATGGTGGTGGCCGCCGTGGCCGTGATG
GCCGTGCTGTCGGTGGCCCTGGCCCAGTTCATTCCGGACGTGGACATTACGTGGA
AGGTGCCGATGACGCTGACGGTGCAGAACCTGTCGATTTTCACGGCCCGAACC
AGTTCGGCCGCGGCATTCCGTCGCCGTCGGCCATTGGCGGCGGCAACGGCCTGG
ACATTGTGGGCGGCGGCGGCTCGCTGTACATTTCGCCGACGGGCGGCCAGGTGC
AGTACTCGCGCGGCTCGAACAACTTCGGCAACCAGGTGGCCTTCACGCGCGTGC
GCAAGAACGGCAACAACGAGTCGGACTTCGCCACGGTGTTCGTGGCGGCACGA
CGCCGTCGTTCGTGATTGTGGGCGACTCGACGGAGAACGAGGTGTCGTTCTGGAC
GAACAACAAGGTGGTGGTGAACTCGCAGGGCTTCATTCCGCCGAACGGCAACTC
 
GGCCGGCGGCAACTCGCAGTACACGTTCGTGAACGGCATTACGGGCACGGCCGG
CGCCCCGGTGGGCGGCACGGTGATTCGCCAGGTGTCGGCCTGGCGCGAGATTTTC
AACACGGCCGGCAACTGCGTGAAGTCGTTCGGCCTGGTGGTGCGCGGCACGGGC
AACCAGGGCCTGGTGCAGGGCGTGGAGTACGACGGCTACGTGGCCATTGACTCG
AACGGCTCGTTCGCCATTTCGGGCTACTCGCCGGCCGTGAACAACGCCCCGGGCT
TCGGCAAGAACTTCGCCGCCGCCCGCACGGGCAACTTCTTCGCCGTGTCGTCGGA
GTCGGGCGTGATTGTGATGTCGATTCCGGTGGACAACGCCGGCTGCACGCTGTCG
TTCTCGGTGGCCTACACGATTACGCCGGGCGCCGGCCGCGTGTCGGGCGTGTCGC
TGGCCCAGGACAACGAGTTCTACGCCGCCGTGGGCATTCCGGGCGCCGGCCCGG
GCGAGGTGCGCATTTACCGCCTGGACGGCGGCGCCACGACGCTGGTGCAGA
CGCTGTCGCCGCCGGACGACATTCCGGAGCTGCCGATTGTGGCCAACCAGCGCTT
CGGCGAGATGGTGCGCTTCGGCGCCAACTCGGAGACGAACTACGTGGCCGTGGG
CTCGCCGGGCTACGCCGCCGAGGGCCTGGCCCTGTTCTACACGGCCGAGCCGGG
CCTGACGCCGAACGACCCGGACGAGGGCCTGCTGACGCTGCTGGCCTACTCGAA
CTCGTCGGAGATTCCGGCCAACGGCGGCCTGGGCGAGTTCATGACGGCCTCGAA
CTGCCGCCAGTTCGTGTTCGGCGAGCCGTCGGTGGACTCGGTGGTGACGTTCCTG
GCCTCGATTGGCGCCTACTACGAGGACTACTGCACGTGCGAGCGCGAGAACATTT
TCGACCAGGGCATTATGTTCCCGGTGCCGAACTTCCCGGGCGAGTCGCCGACGAC
GTGCCGCTCGTCGATTTACGAGTTCCGCTTCAACTGCCTGATGGAGGGCGCCCCG
TCGATTTGCACGTACTCGGAGCGCCCGACGTACGAGTGGACGGAGGAGGTGGTG
GACCCGGACAACACGCCGTGCGAGCTGGTGTCGCGCATTCAGCGCCGCCTGTCG
CAGTCGAACTGCTTCCAGGACTACGTGACGCTGCAGGTGGTGGGCGCCGGCGCC
GGCATGGCCACGAAGGCCGTGTGCGTGCTGAAGGGCGACGGCCCGGTGCAGGGC
ATTATTAACTTCGAGCAGAAGGAGTCGAACGGCCCGGTGAAGGTGTGGGGCTCG
ATTAAGGGCCTGACGGAGGGCCTGCACGGCTTCCACGTGCACGAGTTCGGCGAC
AACACGGCCGGCTGCACGTCGGCCGGCCCGCACTTCAACCCGCTGTCGCGCAAG
CACGGCGGCCCGAAGGACGAGGAGCGCCACGTGGGCGACCTGGGCAACGTGAC
GGCCGACAAGGACGGCGTGGCCGACGTGTCGATTGAGGACTCGGTGATTTCGCT
GTCGGGCGACCACTGCATTATTGGCCGCACGCTGGTGGTGCACGAGAAGGCCGA
CGACCTGGGCAAGGGCGGCAACGAGGAGTCGACGAAGACGGGCAACGCCGGCT
CGCGCCTGGCCTGCGGCGTGATTGGCATTGCCCAGTAAtttctccataataatgtgtgagtagttccc
agataagggaattagggttcctatagggtttcgctcatgtgttgagcatataagaaacccttagtatgtatttgtatttgtaaaatacttctatc
aataaaatttctaattcctaaaaccaaaatccagtactaaaatccagatcccccgaatta
SEQ ID NO:37 ble cDNA with small flanking regions on both sides 
atacgacaaggtgaggaactaaaccatggccaagttgaccagtgccgttccggtgctcaccgcgcgcgacgtcgccggagcggtcgagttctgg
accgaccggctcgggttctcccgggacttcgtggaggacgacttcgccggtgtggtccgggacgacgtgaccctgttcatcagcgcggtccagg
accaggtggtgccggacaacaccctggcctgggtgtgggtgcgcggcctggacgagctgtacgccgagtggtcggaggtcgtgtccacgaactt
ccgggacgcctccgggccggccatgaccgagatcggcgagcagccgtgggggcgggagttcgccctgcgcgacccggccggcaactgcgtg
cacttcgtggccgaggagcaggactgacacggacc
SEQ ID NO 38: chlorella hexose transporter from Q39525 Parachlorella kessleri 
maggaivasggasrsseyqggltayvllvalvaacggmllgydngvtggvasmeqferkffpdvyekkqqivetspyctydnpklqlfvssl
flaglisciffsawitrnwgrkasmgiggiffiaagglvnafaqdiamlivgrvllgfgvglgsqvvpqylsevapfshrgmlnigyqlfvtigilia
glvnygvrnwdngwrlslglaavpglilllgaivlpespnflvekgrtdqgrrileklrgtshveaefadivaaveiarpitmrqswrslftrrymp
qlltsfviqffqqftginaiifyvpvlfsslgsassaallntvvvgavnvgstmiavllsdkfgrrfllieggitcclamlaagitlgvefgqygtedlp
hpvsagvlavicifiagfawswgpmgwlipseiftletrpagtavavmgnflfsfvigqafvsmlcamkfgvflffagwlvimvlcaifllpet
kgvpiervqalyarhwfwkkvmgpaaqeiiaedekrvaasqaimkeerisqtmk
SEQ ID NO 39: yeast hxt2 from P23585 Saccharomyces cerevisiae 
msefatsrvesgsqqtsihstpivqkletdespiqtkseytnaelpakpiaaywtviclclmiafggfvfgwdtgtisgfvnqtdfkrrfgqmks
dgtyylsdvrtglivgifnigcafggltlgrlgdmygrriglmcvvlvyivgiviqiassdkwyqyfigriisgmgvggiavlsptlisetapkhir
[?]
 
gtcvsfyqlmitlgiflgyctnygtkdysnsvqwrvplglnfafaifmiagmlmvpesprflvekgryedakrslaksnkvtiedpsivaemdt
imanveterlagnaswgelfsnkgailprvimgimiqslqqltgnnyffyygttifnavgmkdsfqtsivlgivnfastfvalytvdkfgrrkcll
ggsasmaicfvifstvgvtslypngkdqpsskaagnvmivftclfifffaiswapiayvivaesyplrvknramaiavganwiwgfligfftpfi
tsaigfsygyvfmgclvfsffyvfffvcetkgltleevnemyvegvkpwksgswiskekrvsee
SEQ ID NO 40: human GLUT1 from AAA52571 Homo sapiens

mepsskkltgrlmlavggavlgslqfgyntgvinapqkvieefynqtwvhrygesilpttlttlwslsvaifsvggmigsfsvglfvnrfgrrns
mlmmnllafvsavlmgfsklgksfemlilgrfiigvycglttgfvpmyvgevsptafrgalgtlhqlgivvgiliaqvfgldsimgnkdlwplll
siifipallqcivlpfcpesprfllinrneenraksvlkklrgtadvthdlqemkeesrqmmrekkvtilelfrspayrqpiliavvlqlsqqlsgina
vfyystsifekagvqqpvyatigsgivntaftvvslfvveragrrtlhliglagmagcailmtialalleqlpwmsylsivaifgfvaffevgpgpi
pwfivaelfsqgprpaaiavagfsnwtsnfivgmcfqyveqlcgpyvfiiftvllvlffiftyfkvpetkgrtfdeiasgfrqggasqsdktpeelf
hplgadsqv

## Claims

1. A composition comprising a microalgal polysaccharide, the composition produced by:
a) isolating a polysaccharide from microalgae;
b) drying the isolated polysaccharide to form a film that has been made completely or partially insoluble in water by heating; and
c) homogenizing the film to generate particles, wherein the particles increase in volume on contact with water compared to their anhydrous or partially hydrated volume,
wherein step b) is carried out by (i) heating in two parts wherein the second heating is at a temperature of 148°C to 160°C, or (ii) heating at a temperature of 135°C to 152°C.

2. The composition of claim 1, wherein step b) is carried out by heating in two parts and the first part comprises heating at a temperature of 60 °C to 100°C.

3. The composition of claim 1, wherein the polysaccharide particles are less than 70%, less than 60%, less than 50%, less than 40%, less than 30%, less than 20%, less than 10%, less than 5%, or less than 2% soluble in water.

4. The composition of any one of claims 1 to 3, wherein the particles have an average size of between 400 and 0.1 microns.

5. The composition of any preceding claim, wherein the particles are formulated with an oil suitable for topical administration.

6. The composition of claim 5, wherein the oil is hexadecanoic acid.

7. The composition of claim 5, wherein the oil is contained in an emulsion.

8. The composition of any preceding claim, wherein the polysaccharide contains an amount of sulfur by weight of at least 3%.

9. The composition of any preceding claim, wherein the polysaccharide is substantially free of protein.

10. The composition of any preceding claim, further comprising hyaluronic acid.

11. The composition of any preceding claim, wherein the polysaccharide is produced by a microalga selected from Table 1.

12. A method of manufacturing a composition, comprising:
a) isolating a polysaccharide from microalgae;
b) drying the isolated polysaccharide to a solid film that has been made completely or partially insoluble in water by heating;
c) homogenizing the film into particles, wherein the particles increase in volume on contact with water compared to their anhydrous or partially hydrated volume; and
d) formulating the particles into a non-aqueous material,
wherein step b) is carried out by (i) heating in two parts wherein the second heating is at a temperature of 148°C to 160°C, or (ii) heating at a temperature of 135°C to 152°C.

13. The method of claim 12, wherein step b) is carried out by heating in two parts and the first part comprises heating at a temperature of 60 °C to 100°C.

14. The method of claim 12 or 13, wherein the particles are formulated into the oil phase of an oil-in-water emulsion.

15. The method of any one of claims 12 to 14, wherein the polysaccharide is produced by a microalga selected from Table 1.

16. The method of any one of claims 12 to 15, wherein the particles are homogenized through milling.

## Patentansprüche

1. Zusammensetzung, umfassend ein Mikroalgen-Polysaccharid, wobei die Zusammensetzung produziert wird durch:
a) Isolieren eines Polysaccharids aus Mikroalgen;
b) Trocknen des isolierten Polysaccharids, so dass ein Film gebildet wird, der vollständig oder partiell in Wasser unlöslich gemacht wurde, durch Erwärmen; und
c) Homogenisieren des Films, so dass Partikel erzeugt werden, wobei die Partikel bei Kontakt mit Wasser im Vergleich zu ihrem wasserfreien oder partiell hydratisierten Volumen an Volumen zunehmen,
wobei Schritt b) durch (i) Erwärmen in zwei Teilen, wobei das zweite Erwärmen bei einer Temperatur von 148°C bis 160°C erfolgt, oder (ii) Erwärmen bei einer Temperatur von 135°C bis 152°C erfolgt.

2. Zusammensetzung nach Anspruch 1, wobei Schritt b) durch Erwärmen in zwei Teilen erfolgt, und der erste Teil das Erwärmen bei einer Temperatur von 60°C bis 100°C umfasst.

3. Zusammensetzung nach Anspruch 1, wobei die Polysaccharid-Partikel weniger als 70%, weniger als 60%, weniger als 50%, weniger als 40%, weniger als 30%, weniger als 20%, weniger als 10%, weniger als 5% oder weniger als 2% in Wasser löslich sind.

4. Zusammensetzung nach einem der Ansprüche 1 bis 3, wobei die Partikel eine Durchschnittsgröße zwischen 400 und 0,1 Mikron aufweisen.

5. Zusammensetzung nach einem vorhergehenden Anspruch, wobei die Partikel mit einem Öl formuliert sind, das zur topischen Verabreichung geeignet ist.

6. Zusammensetzung nach Anspruch 5, wobei das Öl Hexadecansäure ist.

7. Zusammensetzung nach Anspruch 5, wobei das Öl in einer Emulsion enthalten ist.

8. Zusammensetzung nach einem vorhergehenden Anspruch, wobei das Polysaccharid eine Schwefelmenge von mindestens 3%, bezogen auf das Gewicht, enthält.

9. Zusammensetzung nach einem vorhergehenden Anspruch, wobei das Polysaccharid im Wesentlichen frei von Protein ist.

10. Zusammensetzung nach einem vorhergehenden Anspruch, die zudem Hyaluronsäure umfasst.

11. Zusammensetzung nach einem vorhergehenden Anspruch, wobei das Polysaccharid durch eine aus Tabelle 1 ausgewählte Mikroalge produziert wird.

12. Verfahren zur Herstellung einer Zusammensetzung, umfassend:
a) Isolieren eines Polysaccharids aus Mikroalgen;
b) Trocknen des isolierten Polysaccharids zu einem festen Film, der vollständig oder partiell in Wasser unlöslich gemacht wurde, durch Erwärmen;
c) Homogenisieren des Films zu Partikeln, wobei die Partikel bei Kontakt mit Wasser im Vergleich zu ihrem wasserfreien oder partiell hydratisierten Volumen an Volumen zunehmen; und
d) Formulieren der Partikel zu einem nicht-wässrigen Material,
wobei Schritt b) durch (i) Erwärmen in zwei Teilen, wobei das zweite Erwärmen bei einer Temperatur von 148°C bis 160°C erfolgt, oder (ii) Erwärmen bei einer Temperatur von 135°C bis 152°C erfolgt.

13. Verfahren nach Anspruch 12, wobei der Schritt b) durch Erwärmen in zwei Teilen erfolgt, und der erste Teil das Erwärmen bei einer Temperatur von 60°C bis 100°C umfasst.

14. Verfahren nach Anspruch 12 oder 13, wobei die Partikel in die Ölphase einer Öl-in-Wasser-Emulsion formuliert werden.

15. Verfahren nach einem der Ansprüche 12 bis 14, wobei das Polysaccharid durch eine aus Tabelle 1 ausgewählte Mikroalge produziert wird.

16. Verfahren nach einem der Ansprüche 12 bis 15, wobei die Partikel durch Mahlen homogenisiert werden.

## Revendications

1. Composition comprenant un polysaccharide microalgal, la composition étant produite par
a) l'isolement d'un polysaccharide à partir de microalgues ;
b) le séchage du polysaccharide isolé afin de former un film ayant été rendu complètement ou partiellement insoluble dans l'eau par chauffage ; et
c) l'homogénéisation du film afin de générer des particules, où les particules augmentent de volume lors d'un contact avec de l'eau par rapport à leur volume anhydre ou partiellement hydraté ;
où l'étape b) est effectuée par (i) un chauffage en deux parties où le deuxième chauffage est effectué à une température allant de 148°C à 160°C, ou (ii) un chauffage à une température allant de 135°C à 152°C.

2. Composition selon la revendication 1, dans laquelle l'étape b) est effectuée par un chauffage en deux parties et la première partie comprend un chauffage à une température allant de 60°C à 100°C.

3. Composition selon la revendication 1, dans laquelle les particules de polysaccharide sont solubles à moins de 70%, moins de 60%, moins de 50%, moins de 40%, moins de 30%, moins de 20%, moins de 10%, moins de 5%, ou moins de 2%, dans l'eau.

4. Composition selon l'une quelconque des revendications 1 à 3, dans laquelle les particules possèdent une taille moyenne comprise entre 400 et 0,1 microns.

5. Composition selon l'une quelconque des revendications précédentes, dans laquelle les particules sont formulées avec une huile convenable pour une administration topique.

6. Composition selon la revendication 5, dans laquelle l'huile est l'acide hexadécanoïque.

7. Composition selon la revendication 5, dans laquelle l'huile est contenue dans une émulsion.

8. Composition selon l'une quelconque des revendications précédentes, dans laquelle le polysaccharide contient une quantité de soufre en poids d'au moins 3%.

9. Composition selon l'une quelconque des revendications précédentes, dans laquelle le polysaccharide est sensiblement dépourvu de protéines.

10. Composition selon l'une quelconque des revendications précédentes, comprenant en outre de l'acide hyaluronique.

11. Composition selon l'une quelconque des revendications précédentes, dans laquelle le polysaccharide est produit par une microalgue sélectionnée dans le Tableau 1.

12. Méthode de fabrication d'une composition, comprenant :
a) l'isolement d'un polysaccharide à partir de microalgues ;
b) le séchage du polysaccharide isolé en un film solide ayant été rendu complètement ou partiellement insoluble dans l'eau par chauffage ;
c) l'homogénéisation du film en particules, où les particules augmentent de volume lors d'un contact avec de l'eau par rapport à leur volume anhydre ou partiellement hydraté ; et
d) la formulation des particules en un matériau non aqueux ;
où l'étape b) est effectuée par (i) un chauffage en deux parties où le deuxième chauffage est effectué à une température allant de 148°C à 160°C, ou (ii) un chauffage à une température allant de 135°C à 152°C.

13. Méthode selon la revendication 12, dans laquelle l'étape b) est effectuée par un chauffage en deux parties et la première partie comprend un chauffage à une température allant de 60°C à 100°C.

14. Méthode selon la revendication 12 ou 13, dans laquelle les particules sont formulées dans la phase huileuse d'une émulsion huile-dans-eau.

15. Méthode selon l'une quelconque des revendications 12 à 14, dans laquelle le polysaccharide est produit par une microalgue sélectionnée dans le Tableau 1.

16. Méthode selon l'une quelconque des revendications 12 à 15, dans laquelle les particules sont homogénéisées par broyage.
